(19) **Europäisches Patentamt / European Patent Office / Office européen des brevets**

(11) **EP 3 773 758 B1**

(12) # EUROPEAN PATENT SPECIFICATION

(45) Date of publication and mention
of the grant of the patent:
**04.06.2025 Bulletin 2025/23**

(21) Application number: **19773044.3**

(22) Date of filing: **18.09.2019**

(51) International Patent Classification (IPC):
**A61L 9/012** (2006.01)    **A61L 9/04** (2006.01)
**A61L 9/12** (2006.01)

(52) Cooperative Patent Classification (CPC):
**A61L 9/012; A61L 9/048; A61L 9/125; A61L 9/127**

(86) International application number:
**PCT/EP2019/075084**

(87) International publication number:
**WO 2020/058373 (26.03.2020 Gazette 2020/13)**

(54) **FRAGRANCE DELIVERY DEVICE**

DUFTSTOFFSPENDER

DISPOSITIF DE DISTRIBUTION DE PARFUM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.09.2018   US 201862733138 P**

(43) Date of publication of application:
**17.02.2021   Bulletin 2021/07**

(73) Proprietor: **Firmenich SA**
**1242 Satigny (CH)**

(72) Inventors:
• **VAN SLEEUWEN, Rutger**
  **Plainsboro, New Jersey 08536 (US)**
• **O'LEARY, Nicholas**
  **Plainsboro, New Jersey 08536 (US)**
• **NORMAND, Valery**
  **Plainsboro, New Jersey 08530 (US)**
• **SHCHERBAKOV, Denis**
  **Plainsboro, New Jersey 08650 (US)**
• **GONZALEZ, Amy**
  **Plainsboro, New Jersey 08536 (US)**
• **DONNELLY, Jeffrey**
  **Plainsboro, NJ 08536 (US)**

(74) Representative: **Strych, Sebastian et al**
**Mitscherlich PartmbB**
**Patent- und Rechtsanwälte**
**Karlstraße 7**
**80333 München (DE)**

(56) References cited:
**EP-A1- 1 581 263        EP-A1- 3 222 297
WO-A1-2013/025585        WO-A1-2017/103863
WO-A1-2017/192508        WO-A1-2018/220059
US-A1- 2018 015 385        US-A1- 2018 117 872**

• **TRIPATHI YOGESH ET AL: "Triply periodic
minimal surface based geometry design of bio-
scaffolds", 2017 INTERNATIONAL
CONFERENCE ON ADVANCES IN MECHANICAL,
INDUSTRIAL, AUTOMATION AND
MANAGEMENT SYSTEMS (AMIAMS), IEEE, 3
February 2017 (2017-02-03), pages 348 - 350,
XP033227542, DOI: 10.1109/
AMIAMS.2017.8069237**
• **NURSHAUN SREEDHAR ET AL: "3D printed feed
spacers based on triply periodic minimal
surfaces for flux enhancement and biofouling
mitigation in RO and UF", DESALINATION., vol.
425, 1 January 2018 (2018-01-01), NL, pages 12 -
21, XP055731756, ISSN: 0011-9164, DOI: 10.1016/
j.desal.2017.10.010**

- JUNG Y ET AL: "Fluid Permeabilities of Triply Periodic Minimal Surfaces", ARXIV.ORG, CORNELL UNIVERSITY LIBRARY, 201 OLIN LIBRARY CORNELL UNIVERSITY ITHACA, NY 14853, 14 March 2006 (2006-03-14), XP080230019, DOI: 10.1103/ PHYSREVE.72.056319

## Description

### FIELD OF THE INVENTION

The present disclosure relates to the field of perfumery and more precisely it concerns a device and associated consumer articles, for dispensing an active composition into the surrounding space.

### BACKGROUND

Devices for dispensing an active composition into the surrounding space are known. Certain devices, such as, for example, devices utilizing active compositions formulated as gels may suffer from shrinkage as the gel evaporates over time. This shrinkage may adversely affect the dispensing of the at least one active composition into the surrounding space. US2018015385 A1, EP3222297 A1, WO2013025585 A1 present different fragrancing devices.

Consequently, a need exists for a simple and efficient delivery device for active compositions with fewer adverse effects on the dispensing of the at least one active composition into the surrounding space.

### SUMMARY

The invention is defined in the appended claims.

One aspect presented herein provides a device comprising:

a) a body portion,

wherein the body portion has a volume and at least one surface,
wherein the volume comprises at least one network of a plurality of fluidly connected passages,
wherein the at least one network of fluidly connected passages has at least one first end and at least one second end,
wherein the at least one first end and at least one second end are separated by a distance,
wherein at least one of the first or second ends are fluidly connected to the at least one surface,
wherein each individual passage within the plurality has a cross section,
wherein the distance, and the cross section of each passage within the plurality defines a surface,

b) at least one active composition,

wherein the at least one active composition is disposed within the at least one network of a plurality of fluidly connected passages, and
wherein the surface is configured to disperse the at least one active composition.

In one aspect, each individual passage within the plurality has one or more branches.

In one aspect, the body portion comprises a porous material.

In one aspect, the surface comprises a triply periodic minimal surface geometry. In one aspect, the triply periodic minimal surface geometry is selected from the group consisting of: a gyroid geometry, a lidinoid geometry, a Schwarz D "diamond" geometry, or a Schwarz P "primitive" geometry.

In one aspect, the surface is defined according to Equation 1:

$$F(x, y, z) = sin(x) \cdot cos(y) + sin(y) \cdot cos(z) + sin(z) \cdot cos(x) = T \qquad \text{Equation 1}$$

In one aspect, the value of T varies in at least one of the x, y, or z direction of the body portion. In one aspect, the value of T is greater in the center of the body portion than the value of T at the periphery of the body portion. In an alternate aspect, the value of T is greater at the periphery of the body portion than the value of T at the center of the body portion. In one aspect, the value of T is selected from a numerical value between 0 and 1.43. In an alternate aspect, the value of T is selected from a numerical value between 0 and -1.43.

In one aspect, the cross section of each individual passage within the plurality varies in at least one of the x, y, or z direction of the body portion. In one aspect, the cross section of each individual passage within the plurality is greater in the center of the body portion than the cross section of each individual passage within the plurality at the periphery of the body portion. In an alternate aspect, the cross section of each individual passage within the plurality is greater at the periphery of the body portion than the cross section of each individual passage within the plurality at the center of the body portion.

In one aspect, the body portion has a cross-sectional shape selected from the group consisting of: an irregular shape, a square, a rectangle, a circle, an ellipse, a rhombus, a semicircle, and a trapezium.

In one aspect, the body portion comprises two networks of a plurality of fluidly connected passages. In one aspect, a first active composition is disposed within the first network, and a second active composition is disposed within the second network.

In one aspect, the first and second networks do not interconnect.

In one aspect, at least one of either the first or second networks is not hollow.

In one aspect, the body portion comprises three networks of a plurality of fluidly connected passages. In one aspect, a first active composition is disposed within the first network, a second active composition is disposed within the second network, and a third active composition is disposed within the third network.

In one aspect, the first, second and third networks do not interconnect.

In one aspect, at least one of either the first, second or third networks is not hollow.

In one aspect, the at least one active composition is selected from the group consisting of an active composition comprising a wax, an active composition comprising a hydrogel, an active composition comprising an oleogel, an active composition comprising an organogel, or mixtures thereof.

In one aspect, the device further comprises an airflow shield.

One aspect presented herein provides a kit comprising the structure of device according to an aspect presented herein and an active composition.

## BRIEF DESCRIPTION OF THE DRAWINGS

Figure 1 shows the incorporation of an active composition into devices according to two aspects presented herein. The top row shows a body portion made via fused deposition modeling. The bottom row shows a body portion made via stereolithography.

Figure 2 shows an air freshener incorporating a device according to an aspect presented herein.

Figure 3 shows an image render of a device according to an aspect presented herein having a structure defined by at least one surface having a gyroid triply periodic minimal surface geometry.

Figure 4 shows image renders of devices having a structure defined by surfaces having triply periodic minimal surface geometries according to several aspects presented herein.

Figure 5 shows devices according to an aspect presented herein having a structure defined by at least one surface having a gyroid triply periodic minimal surface geometry according to two aspects presented herein.

Figure 6 shows an image render of a device according to an aspect presented herein having a structure defined by at least one surface having a gyroid triply periodic minimal surface geometry, wherein the cross section of each individual passage within the plurality varies in at least one of the x, y, or z direction of the body portion.

Figure 7 shows a device according to an aspect presented herein having a structure defined by at least one surface having a blend of two different triply periodic minimal surface geometries.

Figure 8 shows devices according to two aspects presented herein wherein the structure of the body portion is defined by at least one perforated surface formed using a wire-frame operation.

Figure 9 shows a device according to an aspect presented herein wherein the structure of the body portion is defined by at least one perforated surface formed from a porous filament.

Figure 10 shows an image render of a device according to an aspect presented herein wherein the structure of the body portion is defined by at least one perforated surface formed from a tessellated structure.

Figure 11 shows a device according to an aspect presented herein wherein a first active composition is disposed within the first network of a plurality of hollow passages, and a second active composition is disposed within the second network of a plurality of hollow passages.

Figure 12 shows the device according to the aspect shown in Figure 11, after the first and second active compositions have dried.

Figure 13 shows image renders of exemplary cross sections of an air freshener device incorporating a device according to several aspects presented herein and an airflow shield.

Figure 14 shows a representation of an air freshener device incorporating a device according to an aspect presented herein and an airflow shield.

Figure 15 shows a representation of a cross section of an air freshener device incorporating a device according to an aspect presented herein and an airflow shield.

Figure 16 shows the effect of airflow shields of differing $d_o/r_c$ on the evaporation of water from a device according to several aspects presented herein.

Figure 17 shows devices according to an aspect presented herein comprising an evaporated at least one active composition.

Figure 18 shows devices according to aspects presented herein comprising a single gyroid (left), a thickened gyroid with a gradient (center), and a filled gyroid with a gradient (right).

Figure 19 shows 3D printed accessories used for casting devices according to the aspects presented herein. From left to right: a device resting on a base, both inside a cup; a device and base inside a cup with a lid; a device on top of a base stand.

Figure 20 shows 3D printed accessories used for casting control hydrogels.

Figure 21 shows the devices and control samples described in Example 2.

Figure 22 shows the mass loss over time for the test and control devices described in Example 2.

Figure 23 shows the change in appearance of a control device described in Example 2 over time.

Figure 24 shows the change in appearance of a test device described in Example 2 over time.

Figure 25 shows image renders of four structures that were designed using MathMod, and subsequently 3D printed using an SLA printer.

Figure 26 shows four structures that were designed using MathMod, and 3D printed using an SLA printer, incorporating oleogel-based active compositions.

Figure 27 shows image renders that outline the mathematical estimation of the surface area of devices according to the aspects presented herein.

Figure 28 shows image renders of the structure and surface areas of the devices described in Examples 2 and 6, and their approximate calculated surface areas.

Figure 29 shows devices according to aspects presented herein.

Figure 30 shows a device according to an aspect presented herein.

Figure 31 shows the mass loss of the control and test devices described in Example 6 over time.

Figure 32 shows the mass loss of the control and test devices described in Example 6 over time.

Figure 33 shows the headspace analysis of the indicated functional ingredients from the control and test devices described in Example 6 over time. The points around or below a Peak Area Count value of 10,000 were below the mass spectrometer's threshold of detection and, as such, are considered undetectable.

Figure 34 shows the mean sensory intensity score of the control and test devices described in Example 6 over time. "Gyroid High" and "Gyroid Low" correspond to the two control devices.

Figure 35 shows the mean sensory intensity score of the control and test devices described in Example 6 over time (error bar represent confidence intervals, $\alpha$=0.05).

Figure 36 shows the changes in visual appearance of a device described in Example 6 over time.

Figure 37 shows the changes in visual appearance of a device described in Example 6 over time.

Figure 38 shows the changes in visual appearance of a device described in Example 6 over time.

Figure 39 shows the changes in visual appearance of a device described in Example 6 over time.

Figure 40 shows the changes in visual appearance of a control device described in Example 6 over time.

Figure 41 shows the changes in visual appearance of a control device described in Example 6 over time.

Figure 42 shows an image render of a device according to an aspect presented herein having a double-gyroid surface, separating three individual volumes.

Figure 43 shows an image render of a device according to an aspect presented herein.

Figure 44 shows an image render of a device according to an aspect presented herein.

Figure 45 shows an image render of a device according to an aspect presented herein.

Figure 46 shows an image render of a device according to an aspect presented herein.

Figure 47 shows an image render of a device according to an aspect presented herein.

Figure 48 shows an image render of a device according to an aspect presented herein.

Figure 49 shows an image render of a device according to an aspect presented herein.

Figure 50 shows an image render of a device according to an aspect presented herein.

Figure 51 shows an image render of a device according to an aspect presented herein.

Figure 52 shows an image render of a device according to an aspect presented herein.

Figure 53 shows an image render of a device according to an aspect presented herein.

Figure 54 shows an image render of a cross section of a device according to an aspect presented herein.

Figure 55 shows an image render of a cross section of a device according to an aspect presented herein.

Figure 56 shows an image render of a cross section of a device according to an aspect presented herein.

Figure 57 shows an image render of a device according to an aspect presented herein.

Figure 58 shows an image render of a device according to an aspect presented herein.

Figure 59 shows an image render of a device according to an aspect presented herein.

Figure 60 shows an image render of a device according to an aspect presented herein.

Figure 61 shows an image render of a device according to an aspect presented herein.

Figure 62 shows image renders of devices according to aspects presented herein.

Figure 63 shows (a) scaffold front views and (b) side views.

Figure 64 illustrates a cross section of "Control" and "Flip" scaffolds.

Figure 65 shows images of "Flip" and "Control" scaffolds.

Figure 66 is a graph showing mass loss of "Control" and "Flip" scaffolds over time.

Figure 67 shows images of "Control" scaffold over time.

Figure 68 shows images of "Flip" scaffold over time.

Figure 69 is a bar graph showing average sensory intensity of "Control" and "Flip" scaffolds over time.

Figure 70 shows a computer rendering and a 3D print of a higher order scaffold.

Figure 71 illustrates curved designs.

Figure 72 illustrates simple scaffold designs.

## DETAILED DESCRIPTION

In the following description, reference is made to specific embodiments which may be practiced, which is shown by way of illustration. These embodiments are described in detail to enable those skilled in the art to practice the invention described herein, and it is to be understood that other embodiments may be utilized and that logical changes may be made without departing from the scope of the aspects presented herein. The following description of example embodiments is, therefore, not to be taken in a limited sense, and the scope of the various aspects presented herein is defined by the appended claims.

The Abstract is provided to comply with 37 C.F.R. §1.72(b) to allow the reader to quickly ascertain the nature and gist of the technical disclosure. The Abstract is submitted with the understanding that it will not be used to interpret or limit the scope or meaning of the claims.

Devices for dispensing an active composition into the surrounding space are known. Certain devices, such as, for example, devices utilizing active compositions formulated as gels may suffer from shrinkage as the gel evaporates over time. This shrinkage may adversely affect the dispensing of the at least one active composition into the surrounding space. Without intending to be limited to any particular theory, the evaporation process may result in a reduction in the surface area of the gel, a reduction in mobility, and/or limited evaporation of the at least one active composition. Accordingly, the resulting dried hydrogel effectively 'locks in' the fragrance and has an undesirable visual effect.

Current market products allow the drying rate of the at least one active composition to be modified by the use of a cover around a typical gel air freshener. By raising or lowering the cover, the user can modify the evaporation rate and therefore have an influence over the release rate of functional ingredients within the at least one active composition. One could, for example, keep the opening small at the beginning of drying, gradually opening it every day. However, this manual tuning is cumbersome for the user and it would be desirable to have air fresheners with a 'built-in' release control mechanism.

### Devices

Devices according to the aspects presented herein contain a body portion having an internal structure that is filled with at least one active composition. As the at least one active composition evaporates, the at least one active composition shrinks, loses mass, and releases functional ingredients. Without intending to be limited to any particular theory, the internal structure of the body portion provides a surface upon which the at least one active substance may evaporate. Further, the surface may be configured to control the rate of evaporation of the at least one active composition, and/or control the amount and/or rate of release of functional ingredients from the at least one active composition. Additionally, or alternatively, the body portion may act as a support for the at least one active composition.

For example, the rate of evaporation of the at least one active composition may be controlled by varying the surface area of the internal structure of the body portion. By way of illustration, as the at least one active composition evaporates and shrinks, the at least one active composition may recede from the outer boundaries of the body portion.

Such shrinkage may result in a reduced surface area of the at least one active composition. Such a reduced surface area may be counteracted by the internal structure of the body portion. For example, the surface area of the internal structure may increase along the path that the at least one active composition recedes. Alternatively, the internal structure may provide "anchor points" which may reduce the rate at which the at least one active composition recedes. Alternatively, the internal structure may provide "anchor points" which may prevent or impede the at least one active composition from receding.

Without intending to be limited to any particular theory, a device according to an aspect presented herein may be generated with a particular geometry configured to alter the mass loss of an at least one active composition, compared to the mass loss of the at least one active composition alone. Alternatively, a device according to an aspect presented herein may be generated with a particular geometry configured to alter the amount of the perception of the at least one active composition, compared to the perception of the at least one active composition alone. Alternatively, a device according to an aspect presented herein may be generated with a particular geometry configured to alter the amount of the at least one active composition in the surrounding space, compared to the amount of the at least one active composition in the surrounding space of the at least one active composition alone.

A device according to an aspect presented herein may be suitable not only for hydrogel air fresheners, but can be expanded to any solid, semi-solid or even viscous liquid format of a fragrance. It may be desirable, for example, to maximize the fragrance level to formulations that are not self-supporting. In those cases these scaffolds would provide an additional benefit.

Accordingly, one aspect presented herein provides a device comprising:

a) a body portion,

wherein the body portion has a volume and at least one surface,

wherein the volume comprises at least one network of a plurality of fluidly connected passages,

wherein the at least one network of fluidly connected passages has at least one first end and at least one second end,

wherein the at least one first end and at least one second end are separated by a distance,

wherein at least one of the first or second ends are fluidly connected to the at least one surface,

wherein each individual passage within the plurality has a cross section,

wherein the distance, and the cross section of each passage within the plurality defines a surface,

b) at least one active composition,

wherein the at least one active composition is disposed within the at least one network of a plurality of fluidly connected passages, and
wherein the surface is configured to disperse the at least one active composition.

In some aspects, each individual passage within the plurality has one or more branches. In some aspects, at least one of first or second ends are open.

In some aspects, the device further comprises an airflow shield.

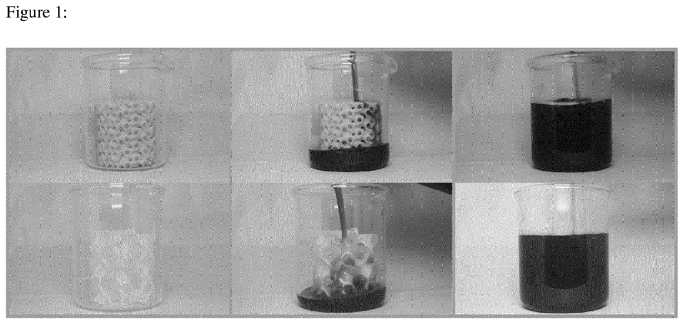

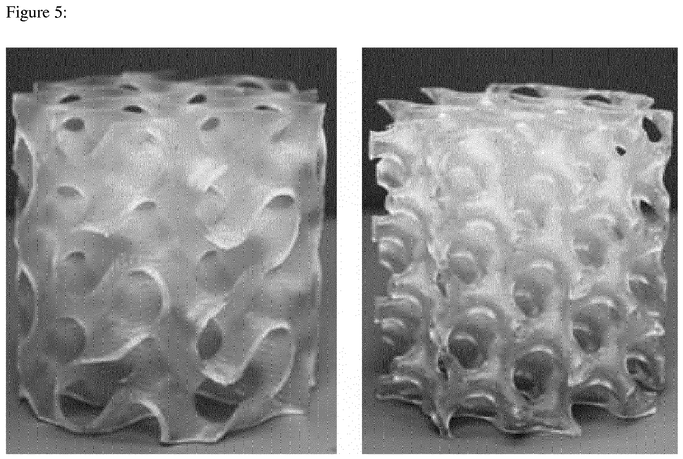

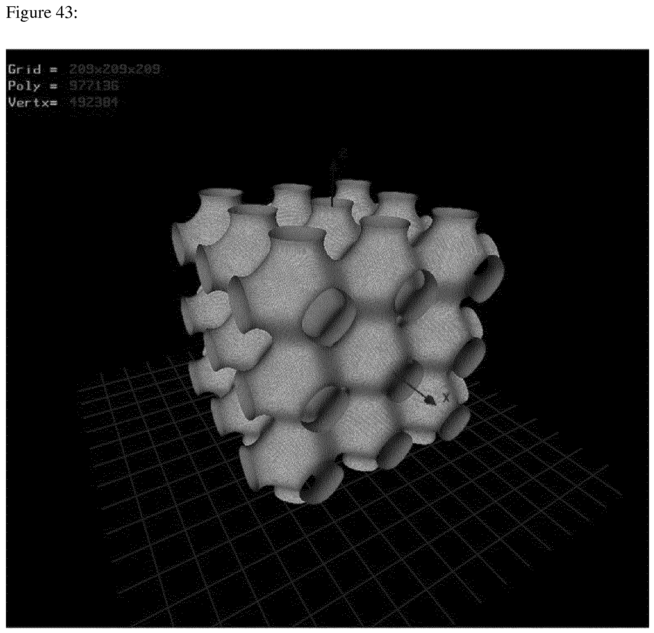

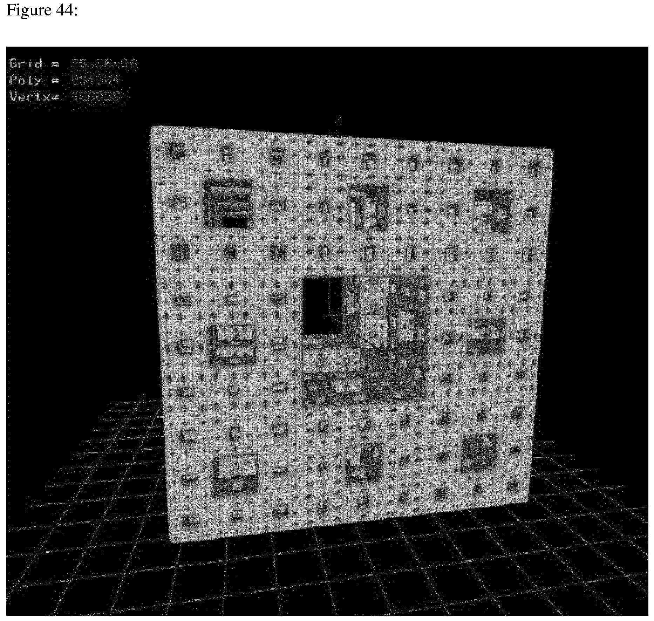

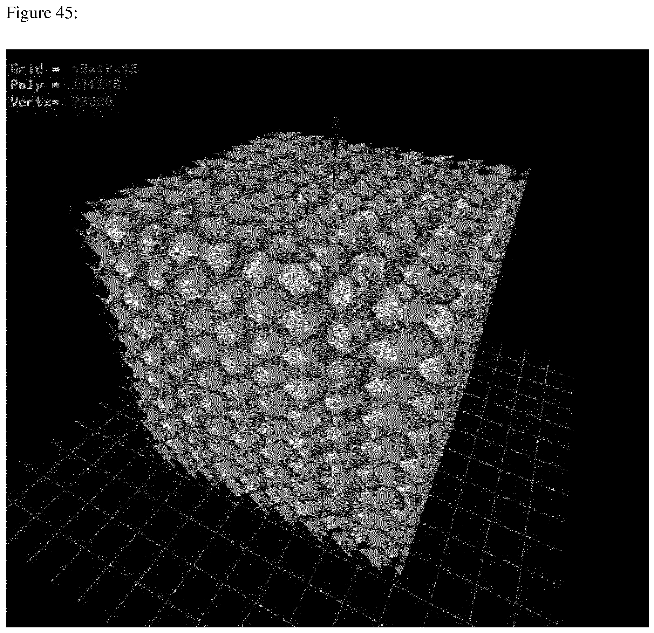

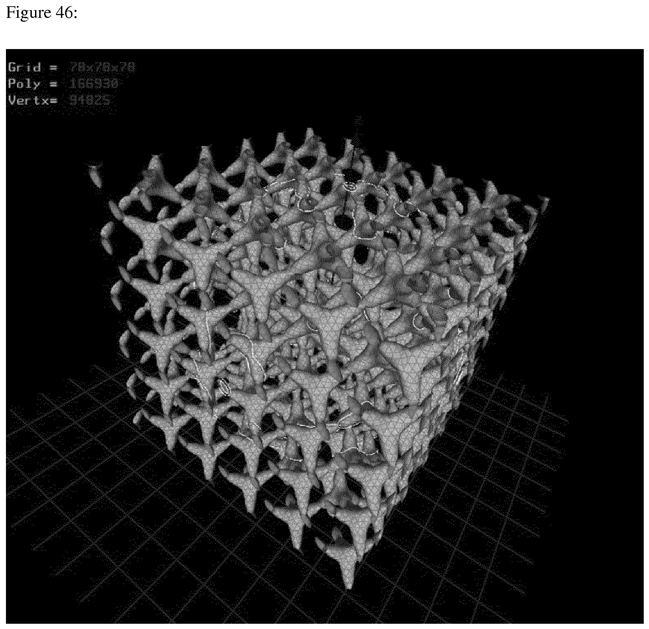

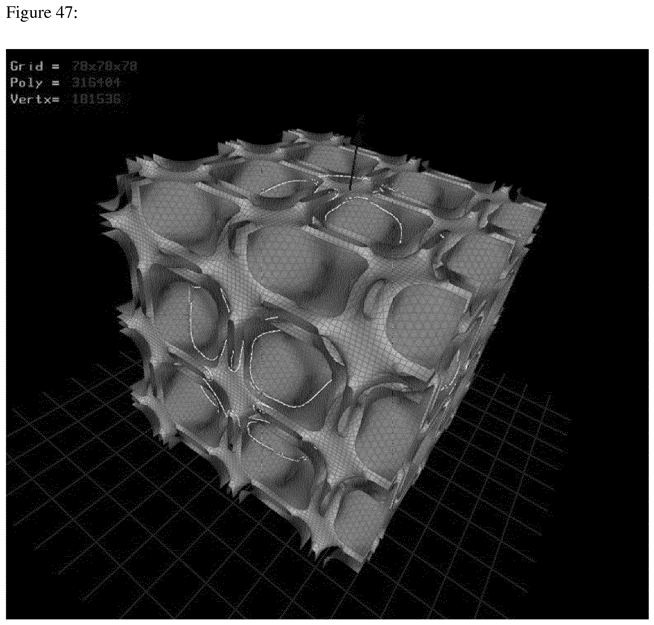

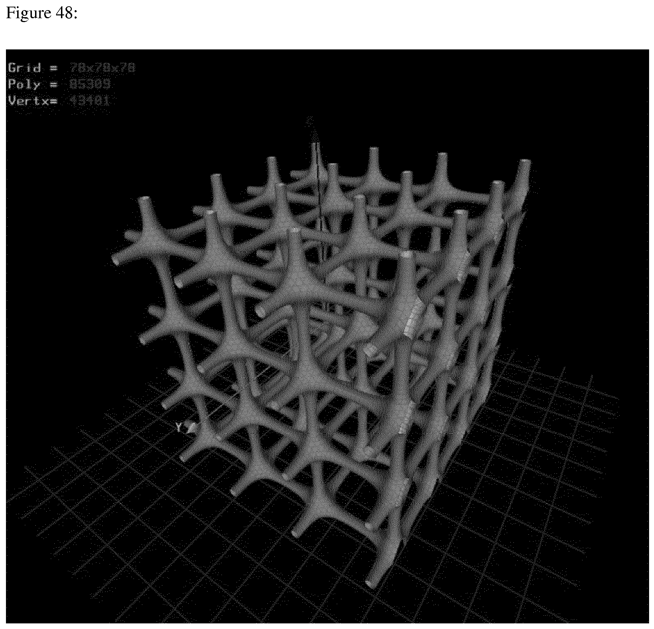

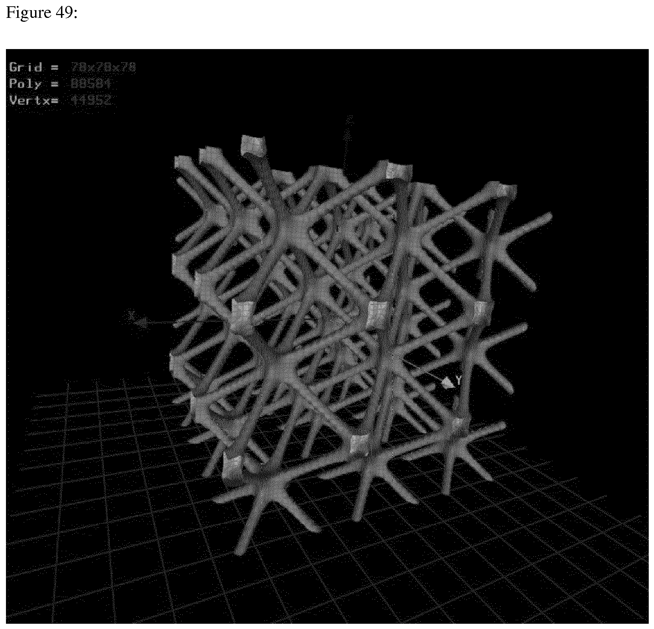

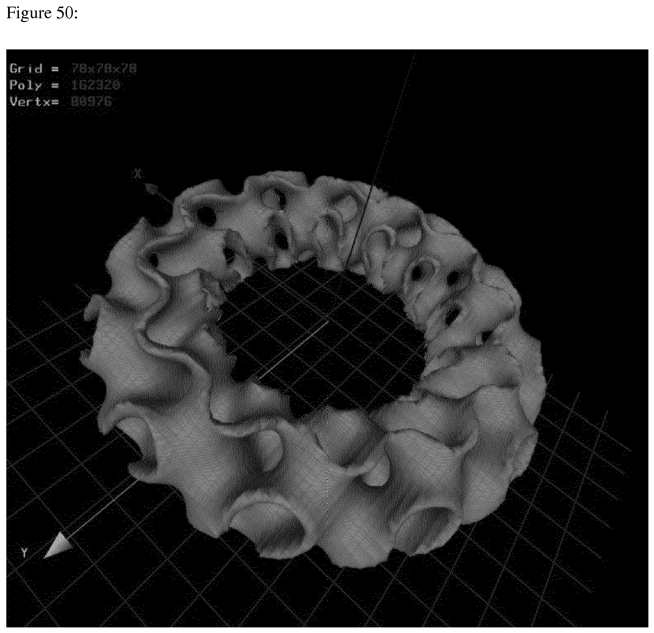

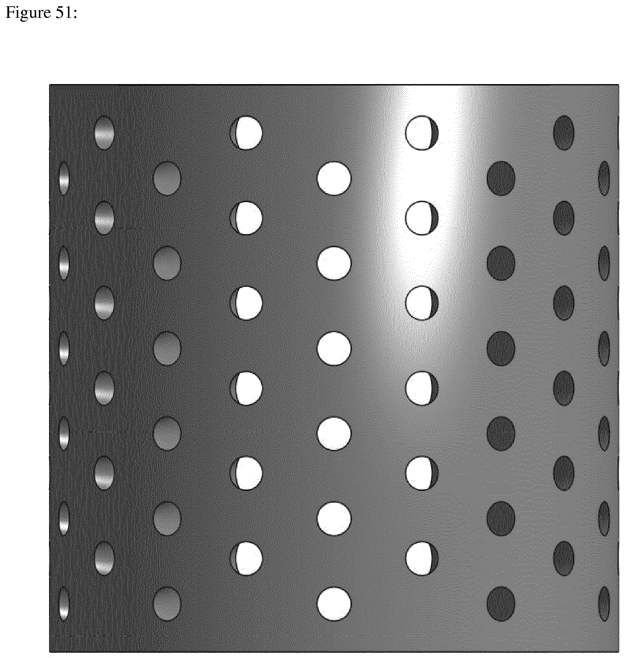

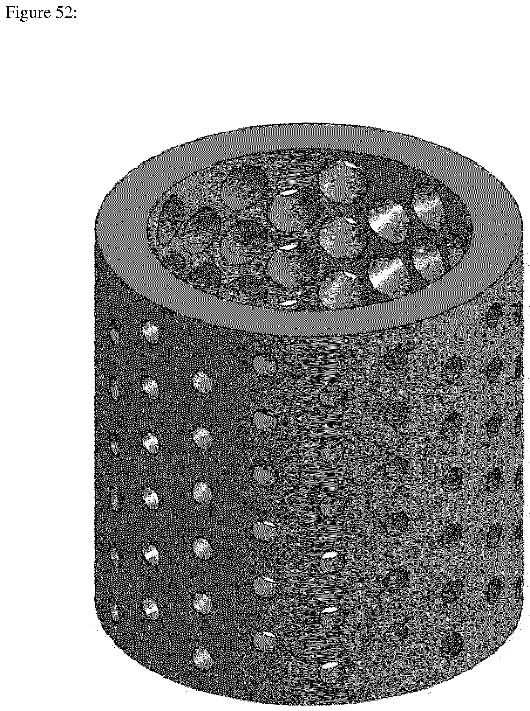

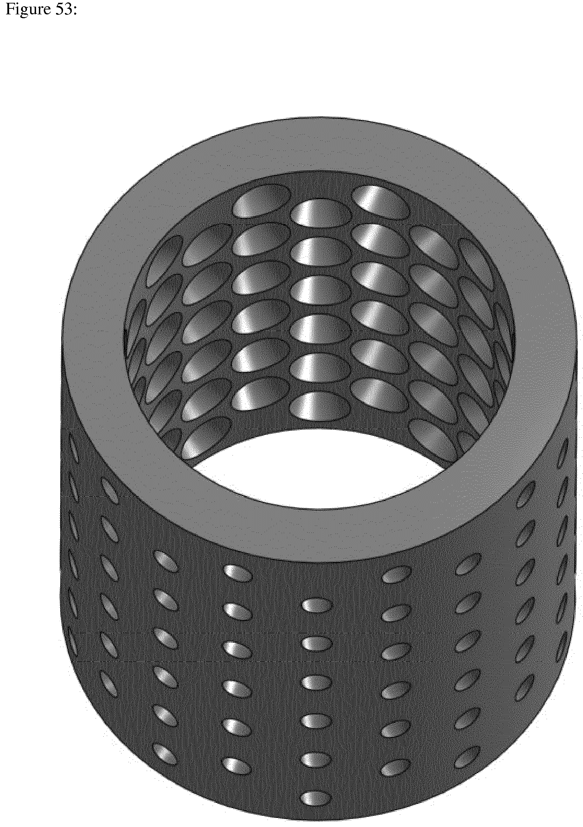

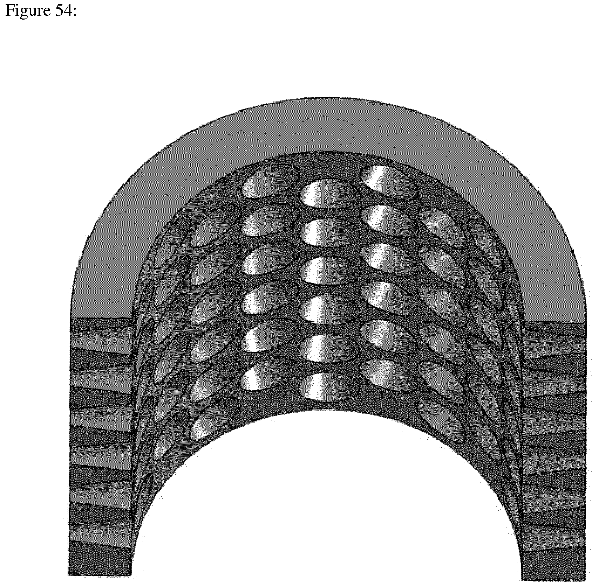

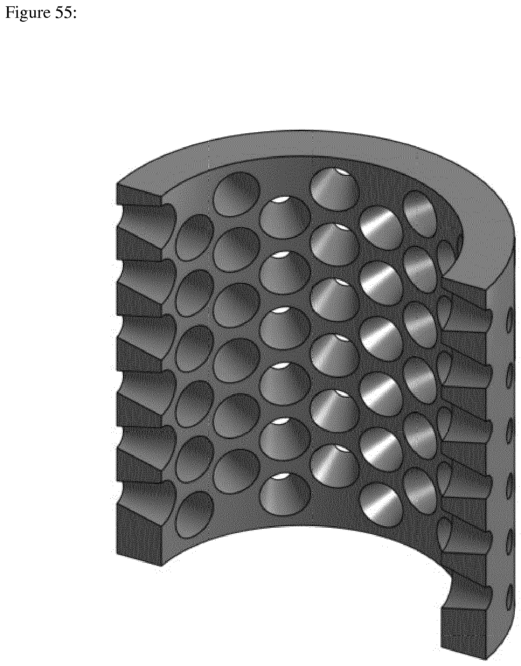

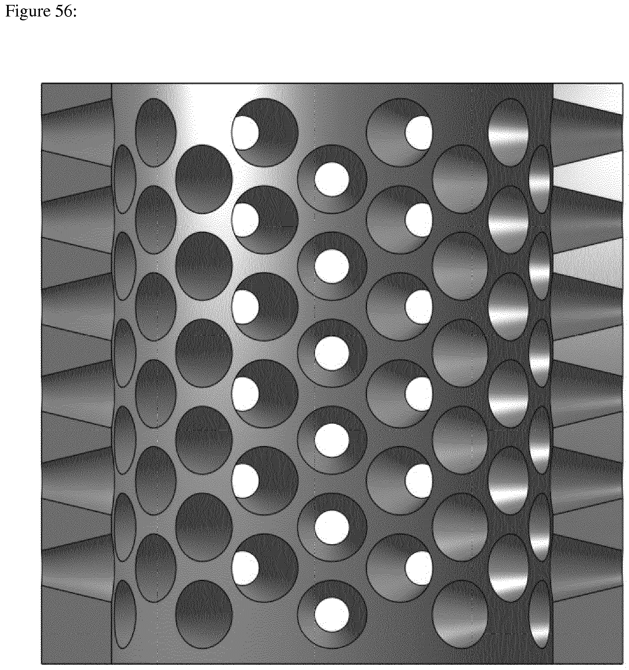

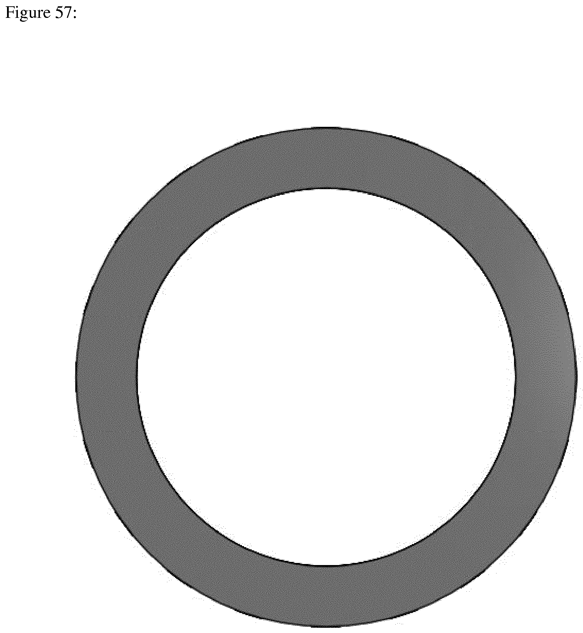

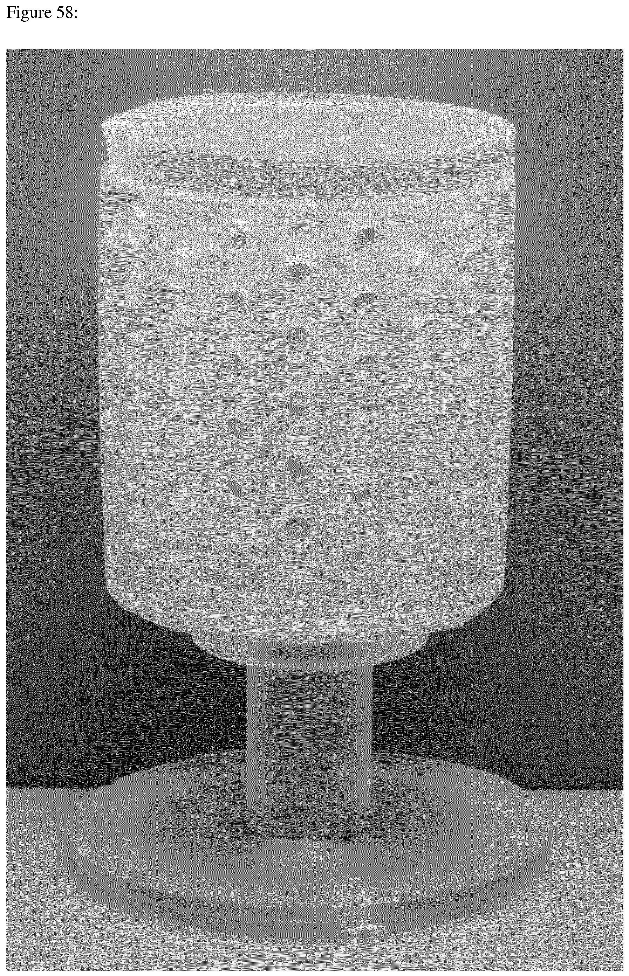

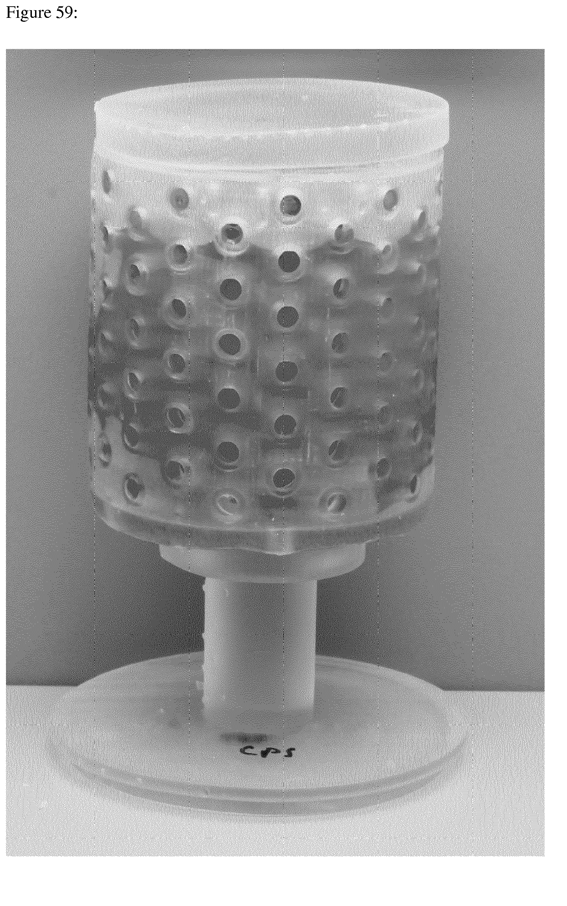

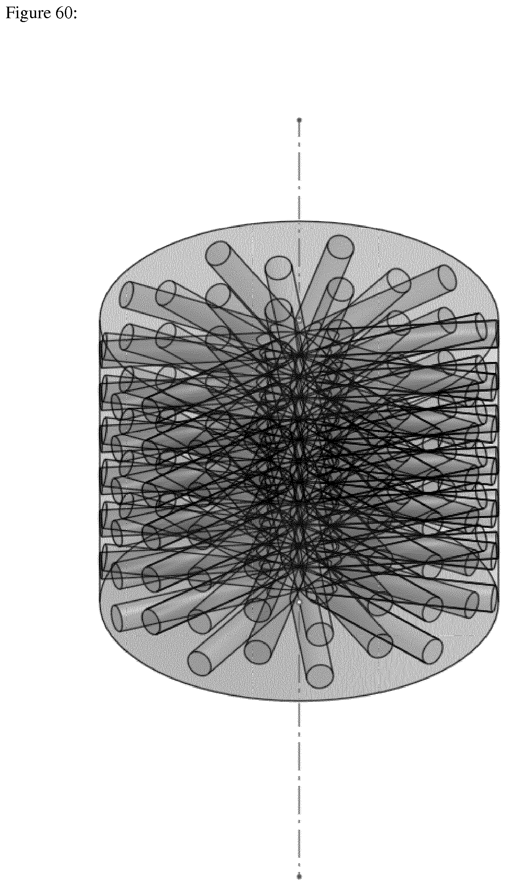

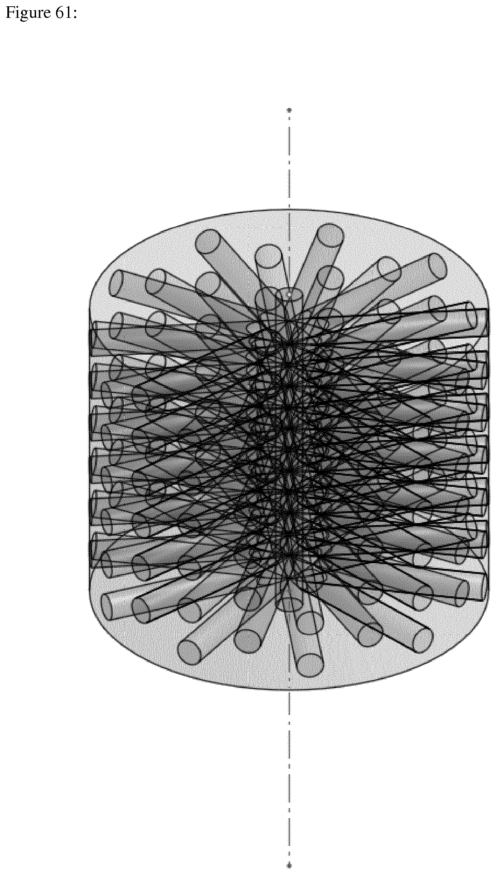

The body portion of the device may have any cross-sectional shape, such as, for example, an irregular shape, a square shape, a rectangular shape, a circular shape, an elliptical shape, a rhomboid shape, a semi-circular shape, a trapezoidal shape and the like. Examples of devices according some aspects provided herein are shown in Figures 1 to 5 and Figures 43 to 61.

In some aspects, the surface comprises a triply periodic minimal surface geometry. In one aspect, the triply periodic minimal surface geometry is selected from the group consisting of: a gyroid geometry, a lidinoid geometry, a Schwarz D "diamond" geometry, or a Schwarz P "primitive" structure geometry.

Examples of triply periodic minimal surface geometries suitable for use according to the aspects presented herein are disclosed in Gyroid and Gyroid-Like Surfaces: Rudolf, M., & Scherer, J. (2013), S. I. Publishing (Ed.), Double-Gyroid-Structured Functional Materials (pp. 7-19)). Additional examples of triply periodic minimal surface geometries suitable for use according to the aspects presented herein are disclosed in S. Andersson K. Larsson M. Larsson M. Jacob. (1999). Biomathematics, Mathematics of Biostructures and Biodynamics. Elsevier Science.

Without intending to be limited to any particular theory, the surface provides a body portion having a structure having a porosity, a surface area and volume that may be configured to disperse the at least one active composition.

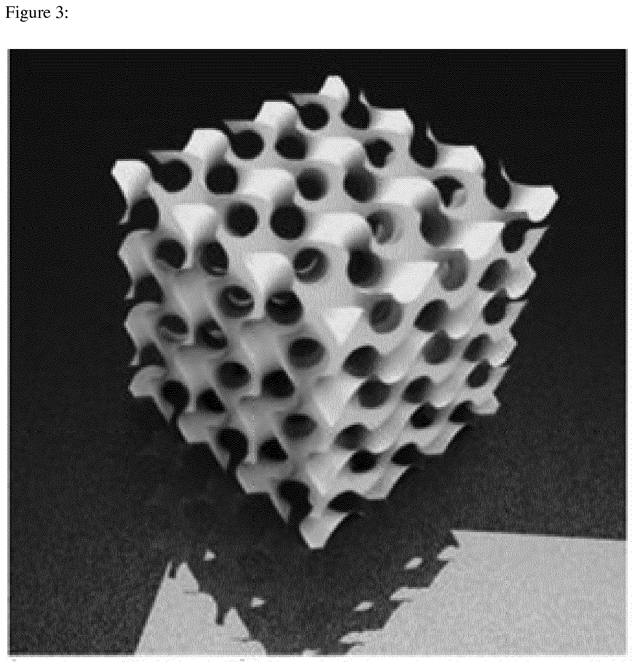

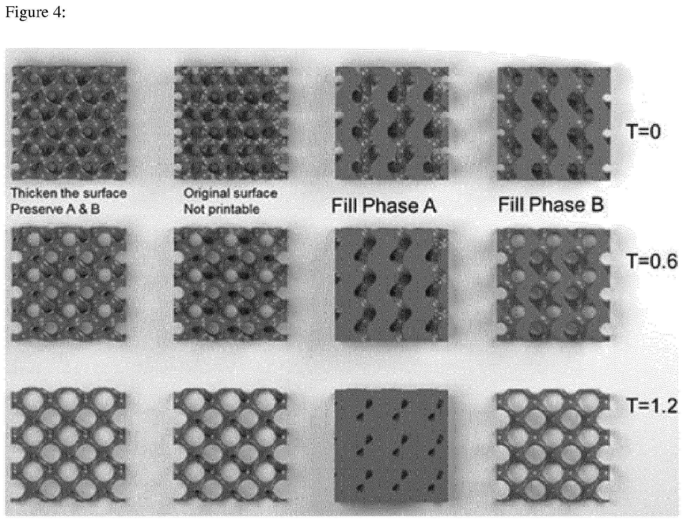

In some aspects, the surface is defined by a triply periodic minimal surface geometry. Referring to Figures 3 and 4, in some aspects, the surface defined by a triply periodic minimal surface geometry is defined according to Equation 1:

$$F(x,y,z) = sin(x) \cdot cos(y) + sin(y) \cdot cos(z) + sin(z) \cdot cos(x) = T \quad \text{Equation 1}$$

By way of illustration, varying the numerical value of T may vary the porosity, the surface area and/or volume of the body portion. For example, when T=0, the body portion is divided exactly into two separate enantiomeric interpenetrating single-gyroid volumes (both 50%) as depicted in Figure 3, and the upper left-hand body portion depicted in Figure 4. The two separate interpenetrating single-gyroid volumes each comprise a separate network of a plurality of hollow passages (referred to herein as "A" and "B").

Referring to Figure 4, when the value of T is between 0 and 1.413, the volume of volume A increases, while the volume of volume B decreases. Similarly, when the value of T is between 0 and -1.413 the opposite occurs, wherein the volume of volume B increases, while the volume of volume A decreases.

When values of the absolute value of T is between 1.413 and 1.5 the surfaces are no longer connected. For absolute values of T that exceed 1.5 no realistic solution for Equation 1 exists.

In alternative aspects, the porosity, the surface area and/or volume of the body portion may be altered by filling in a network of a plurality of hollow passages. Referring to Figure 4, in some aspects, either one of volume A or B may be solid. Examples of such aspects are shown in the right two columns of Figure 4.

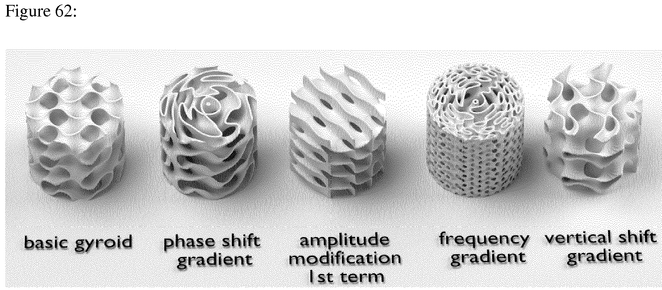

Referring to Figure 62, in some aspects, the at least one surface defined by a triply periodic minimal surface geometry is defined according to Equation 2:

$F(x,y,z)=(A_1sin(B_1x+C_1)+D_1)\cdot \quad (A_2cos(B_2y+C_2)+D_2)+ \quad (A_3sin(B_3y+C_3)+D_3)\cdot \quad (A_4cos(B_4z+C_4)+D_4)+ (A_5sin(B_5z+C_5)+D_5)\cdot (A_6cos(B_6x+C_6)+D_6)=T$ Equation 2. 
$\qquad$ Equation 2.

Wherein A = amplitude; B - frequency; C = phase shift; D = vertical shift, and wherein at least one of A, B, C, or D may vary in at least one of the x, y, or z direction of the body portion.

Using Equation 2 as an example, a basic sine wave of y=sin(x) can be modified as follows: y=A*sin(Bx +C)+D, where A-D represent parameters that alter the amplitude, the frequency, the phase shift and a vertical shift respectively. By altering these variables in one or all of the trigonometric functions of equation 2 (or similar equations), the different geometries shown in Figure 62 can be obtained.

In alternate aspects, the surface may be defined by combining more than one equation that defines a triply periodic minimal surface geometry. Such aspects are disclosed in Venkatesh, V., Reddy, K. A. K., & Sreekanth, E. (2014). Design of Mathematically Defined Heterogeneous Porous Scaffold Architecture for Tissue Engineering, 10(24), 1169-1174.

For example, the triply periodic minimal surface geometry may be defined according to Equations 3 and 4:

$$\varphi_G = \textbf{\textit{Gyroid}} = \textbf{sin}(x) \cdot \textbf{cos}(y) + \textbf{sin}(y) \cdot \textbf{cos}(z) + \textbf{sin}(z) \cdot \textbf{cos}(x) = 0 \text{ Equation 2}$$

$\varphi_D$ = ***Schwartz Diamond*** = **sin**(*x*) · **sin**(*y*) · **sin**(*z*) + **sin**(*x*) · **cos**(*y*) · **cos**(*z*) + **cos**(*x*) · **sin**(*y*) · **cos**(*z*) + **cos**(*x*) · **cos**(*y*) · **sin**(*z*) = 0 Equation 3 
$\qquad$ Equation 3

Then:

$$\varphi_{mix} = \mu \cdot \varphi_G + (1 - \mu) \cdot \varphi_D = 0 \text{ Equation 4}$$

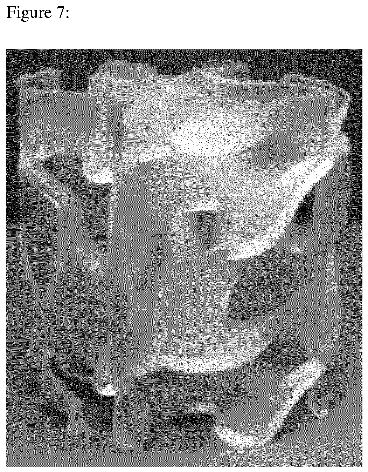

In some aspects, $\mu$ ranges from 0 to 1. Figure 7 depicts a body portion where $\mu= 0.5$.

In one aspect, the cross section of each individual passage within the plurality varies in at least one of the x, y, or z direction of the body portion. In one aspect, the cross section of each individual passage within the plurality is greater in the center of the body portion than the cross section of each individual passage within the plurality at the periphery of the body portion. In an alternate aspect, the cross section of each individual passage within the plurality is greater at the periphery of the body portion than the cross section of each individual passage within the plurality at the center of the body portion. Without intending to be limited to any particular theory, the variation of the cross section of each individual passage within the plurality may alter the evaporation rate of the at least one active composition, alter the rate at which the at least one active composition recedes, alter the amount of functional ingredients released from the at least one active composition, or any combination thereof.

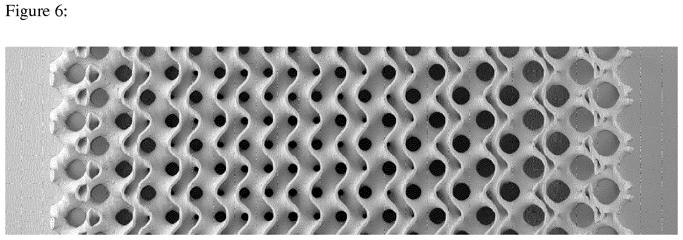

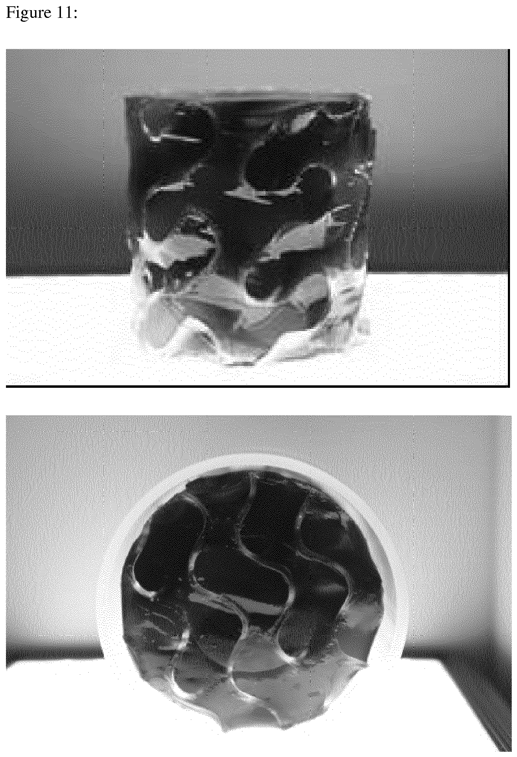

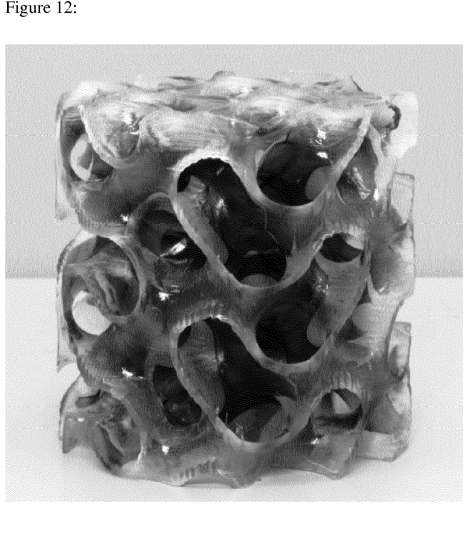

Without intending to be limited to any particular theory, the cross section of each individual passage within the plurality may be altered in at least one of the x, y, or z direction of the body portion, thereby generating a body portion having a radial porosity gradient. In this instance, the term "porosity gradient" refers to the variation in the cross section of each individual passage within the plurality in at least one of the x, y, or z direction of the body portion. An example of such a body portion is shown in Figure 6, wherein the body portion has a porosity gradient in the horizontal direction. Without intending to be limited to any particular theory, as the at least one active composition evaporates and shrinks, the at least one active composition may recede from the outer boundaries of the body portion and thus encounter a change in pore size allowing it to change the rate of evaporation in a tunable manner. The effect of a device according to an aspect presented herein on the evaporation of at least one active composition is shown in Figure 12 (depicting the device of Figure 11 after the two active compositions have dried). Referring to Example 6, and without intending to be limited to any particular theory, devices with the smallest holes on the outside appeared to be more efficient in reducing the evaporation rate of the at least one active composition. This may be caused by a 'boundary effect', where the moisture released from the at least one active composition is 'trapped' inside the device, reducing the relative humidity/ fragrance gradient, reducing the overall mass transfer rate.

Alternatively, the cross section of each individual passage within the plurality may be altered by varying the frequency parameter in any one of Equations 1 to 4 in at least one of the x, y, or z direction of the body portion.

Alternatively, the cross section of each individual passage within the plurality may be altered by varying the amplitude parameter in any one of Equations 1 to 4 in at least one of the x, y, or z direction of the body portion.

Alternatively, the cross section of each individual passage within the plurality may be altered by varying the phase shift parameter in any one of Equations 1 to 4 in at least one of the x, y, or z direction of the body portion.

Alternatively, the cross section of each individual passage within the plurality may be altered by varying the vertical shift parameter in any one of Equations 1 to 4 in at least one of the x, y, or z direction of the body portion.

Alternatively, the cross section of each individual passage within the plurality may be altered by varying $\mu$ in Equation 4 above as a function of distance in at least one of the x, y, or z direction of the body portion, wherein $\mu$ ranges from 0 to 1.

Alternatively, the cross section of each individual passage within the plurality may be altered by varying $\mu$ in Equation 4 and introducing porosity gradients in at least one of the x, y, or z direction of the body portion.

Referring to Figure 11, in some aspects the body portion comprises two networks of a plurality of fluidly connected passages. In one aspect, a first active composition is disposed within the first network, and a second active composition is disposed within the second network. In some aspects, the first and second networks do not interconnect.

In some aspects, at least one of either the first or second networks is not hollow.

In some aspects, the first and second active compositions are the same. Alternatively, in some aspects, the first and second active compositions are different. For example, by way of illustration, the first active composition may have a first olfactive note, and the second active composition may have a second olfactive note, different to the first olfactive note. In some aspects, the device may be configured to release the first active composition at a different rate than the second active composition. In some aspects, the device may be configured to release the first active composition and the second active composition at a rate that maintains the perception of a particular olfactive note at a consistent level over time.

Without intending to be limited to any particular theory, the device may be configured to release the first active composition at a different rate than the first active composition by providing a structure having an increased surface area in volume A, compared to volume B. Alternatively, the device may be configured to release the first active composition at a different rate than the first active composition by providing a structure having a decreased pore size in volume A, compared to volume B. Alternatively, the device may be configured to release the first active composition at a different rate than the first active composition by providing a structure having an increased surface area to volume ratio in volume A, compared to volume B.

In some aspects, random straight lines drawn through the center of a device intersect on average at least 2, or 3, or 4, or more times with the at least one surface.

In some aspects, the device has a surface area to volume ratio of at least 1 $cm^2$:$cm^3$, or at least 2 $cm^2$:$cm^3$, or at least 3 $cm^2$:$cm^3$, or at least 4 $cm^2$:$cm^3$, or at least 5 $cm^2$:$cm^3$.

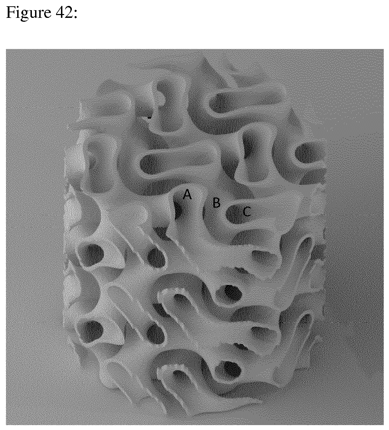

Referring to Figure 42, in some aspects, the body portion comprises three networks of a plurality of fluidly connected passages. In one aspect, a first active composition is disposed within the first network, a second active composition is disposed within the second network, and a third active composition is disposed within the third network. In some aspects, the first, second and third networks do not interconnect.

In some aspects, at least one of either the first, second, or third networks is not hollow.

In some aspects, the body portion has a cross-sectional shape selected from the group consisting of: an irregular shape, a square, a rectangle, a circle, an ellipse, a rhombus, a semicircle, and a trapezium.

In some aspects, the body portion comprises a porous material. Suitable porous materials for the body portion include, but are not limited to: porous porcelain materials, plastics, molded ceramics, glass fibers, clay, activated carbon, cellulose, and the like.

In some aspects, the porous material is selected from the group consisting of: charcoal, ceramic, plastic, clay, cellulose, and mixtures thereof. In some aspects, the porous material is charcoal.

In some aspects, the porous material is selected from the group consisting of: plastics, metals, uv-cured polymers, and mixtures thereof.

In some aspects, the material is selected from the materials disclosed in Wohler, T. (2016). Wohlers Report 2016 3D Printing and Additive Manufacturing State of the Industry. Annual Worldwide Progress Report. Wohlers Associates, Inc.).

In some aspects, the material comprises a wax.

In some aspects, the material is selected from the materials disclosed in International Patent Application Publication No. WO2016/172699.

The body portion may be formed by any suitable method, readily selected by one of ordinary skill in the art. Non-limiting examples of methods to form the body portion include casting, extrusion, 3-D printing, sintering, and the like.

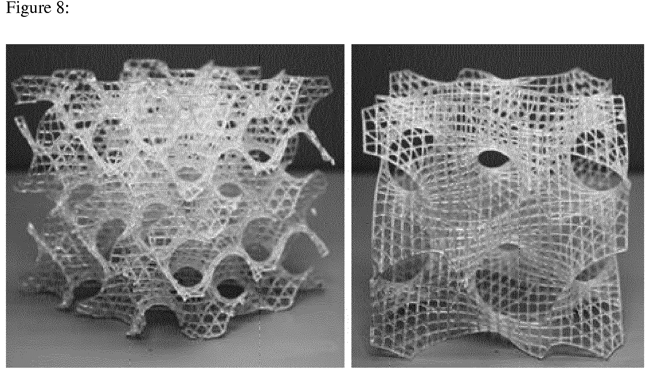

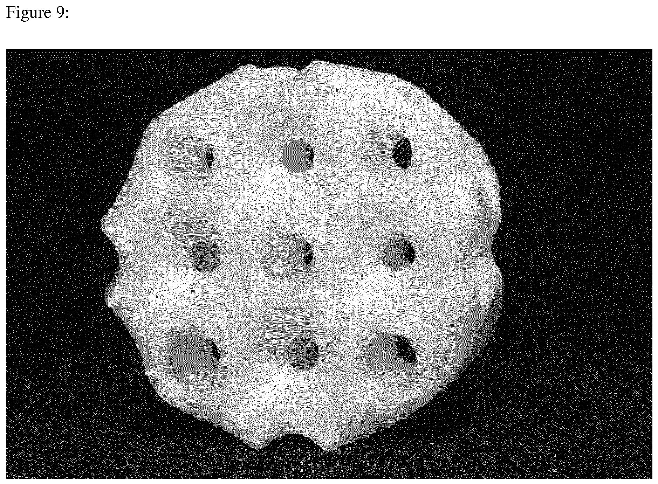

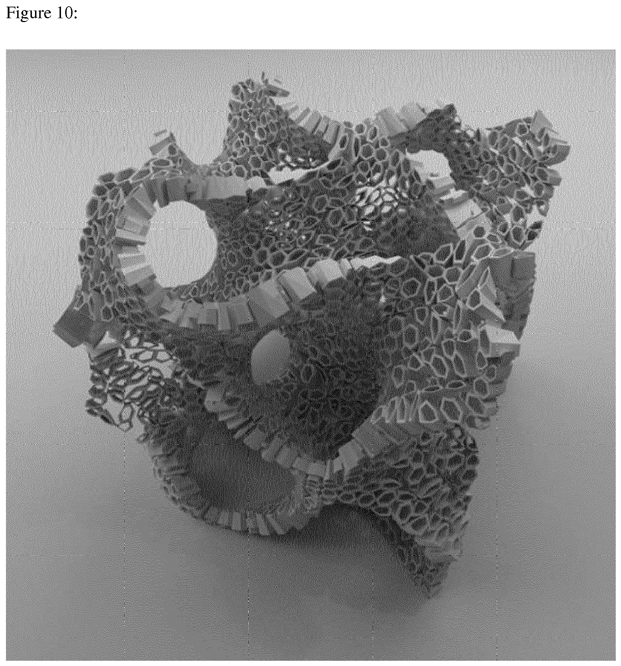

Referring to Figures 1 to 7, in some aspects, the structure of the body portion may be defined by at least one solid surface. Alternatively, referring to Figures 8 to 10, in some aspects, the structure of the body portion may be defined by at least one perforated surface. Examples of perforated surfaces include, but are not limited to wire-frames, tessellated shapes, fibers, trabecular structures, and the like.

## *The at Least One Active Composition*

As used herein, the term "active composition" refers to a gel, or viscous liquid which is at least partially volatile, i.e., can evaporate, and which is able to impart a fragrance or other benefit to the surrounding space.

In some aspects, the at least one active composition is selected from the group consisting of an active composition comprising a wax, an active composition comprising a hydrogel, an active composition comprising an oleogel, an active composition comprising an organogel, or mixtures thereof.

The at least one active composition may be disposed within the structure of the body portion by any suitable method, such as, for example, injecting or pouring, the at least one active composition into the body portion. Alternatively, the at least one active composition may be disposed within the structure of the body portion by immersing the body portion into the at least one active composition.

In some aspects, the at least one active composition may be incorporated directly in to the material comprising the body portion.

An example of an at least one active composition suitable for use in a device according to an aspect presented herein is the at least one active composition disclosed in International Patent Application Publication No. WO2017/017251 A1.

Another example of an at least one active composition suitable for use in a device according to an aspect presented herein is the at least one active composition disclosed in International Patent Application Publication No. WO2013/030153 A1.

Another example of an at least one active composition suitable for use in a device according to an aspect presented herein is the at least one active composition disclosed in International Patent Application Publication No. WO 02/055116 A1.

Another example of an at least one active composition suitable for use in a device according to an aspect presented herein is the at least one active composition disclosed in European Application Publication No. EP1177799 A1.

Another example of an at least one active composition suitable for use in a device according to an aspect presented herein is the at least one active composition disclosed in U.S. 6,039,266.

Another example of an at least one active composition suitable for use in a device according to an aspect presented herein is the at least one active composition disclosed in United States Patent Application Publication No. US2002/0041860 A1.

Another example of an at least one active composition suitable for use in a device according to an aspect presented herein is the at least one active composition disclosed in International Patent Application Publication No. WO2017/017251 A1.

The at least one active composition may contain between 0.1% by weight and 100% by weight fragrance-chemicals or essential oils; alternatively between 40% by weight and 100% by weight fragrance-chemicals or essential oils; alternatively between 60% by weight and 100% by weight fragrance-chemicals or essential oils. The balance of these formulations can include water, gellant, solvents, dyes, colorants, anti-oxidants, UV inhibitors, bittering agents, etc. as are generally known to skilled artisans.

In some aspects, the at least one active composition is a perfume. As perfume there can be used any ingredient or mixture of ingredients currently used in perfumery, i.e. capable of exercising a perfuming action. More often, however, a perfume will be a more or less complex mixture of ingredients of natural or synthetic origin. The nature and type of the ingredients do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitrites, terpene hydrocarbons, nitrogenous or sulphurous heterocyclic compounds and essential oils of natural or synthetic origin. Many of these ingredients are in any case listed in reference texts such as the book by S. Arctander, Perfume and Flavor Chemicals, 1969, Montclair, N.J., USA, or its more recent versions, or in other works of a similar nature, as well as in the abundant patent literature in the field of perfumery.

In some aspects, the perfuming action may further comprise providing a sensorial and/ or emotional benefit, or, alternatively, the perfuming action may be configured to prevent the habituation of the user to the perfume. In some aspects, the sensorial and/or emotional benefit may be provided by the addition of an additional agent to the at least one active composition. For example, by way of illustration, the at least one active composition may further comprise a cooling compound, that imparts a cooling sensation to a user.

Although special mention has been made hereinabove of the perfuming effect that can be exerted by the devices of the present disclosure, the same principles apply to analogous devices for the diffusion of malodor counteracting, or deodorizing or sanitizing vapors, the perfume being replaced by a malodor counteracting composition, a deodorizing composition, an antibacterial, an insecticide, an insect repellent or an insect attractant. As used herein, the term "sanitizing vapors", refers to the vapors of those substances which can enhance the degree of acceptance of the air surrounding the observer, but also to those substances which can exert an attractant or repellent effect towards certain species of insects, for instance towards houseflies or mosquitoes, or else, which can have bactericide or bacteriostatic activity. In some aspects, mixtures of such agents can also be used.

The term "malodor counteracting ingredient" is understood as being capable of reducing the perception of malodor, i.e. of an odor that is unpleasant or offensive to the human nose. The term "insect attractant or repellent" is understood as a compound having a positive or negative effect on insects. Examples of insect attractant or repellent ingredients can be found in reference texts or in other works of a similar nature as for example: A. M. El-Sayed, The Pherobase 2005, http://www.pherobase.net.

Accordingly, in some aspects, the body portion configured to ensure that the amount of active composition that evaporates from the body portion is in an amount sufficient to ensure the at least one active composition is at, or above an amount necessary to achieve its desired effect in the surrounding space.

In some aspects, the total surface area of the network(s) of a plurality of hollow passages is configured to ensure the amount of the at least one active composition that is disposed within is in an amount sufficient to ensure the amount of the at least one active composition that is released from the device into the surrounding space remains relatively constant throughout the lifetime of the device, and/ or is in an amount sufficient to be above an amount necessary to achieve its desired effect in the surrounding space.

In some aspects, the at least one active composition may also contain optional ingredients acting as, for example, solvents, thickeners, anti-oxidants, dyes, bittering agents and UV inhibitors.

In some aspects, the at least one active composition further comprises one or more solvents. In some aspects, the one or more solvents may be useful to have a single-phase liquid and/or to modulate the speed of evaporation of the at least one active composition into the surrounding air. The solvents may belong to the families of isoparaffins, paraffins, hydrocarbons, glycols, glycol ethers, glycol ether esters, esters or ketones.

Examples of commercially available solvents suitable for use in the present disclosure include the solvents known under the tradename Isopar® H, J, K, L, M, P or V (isoparaffins; origin: Exxon Chemical), Norpar® 12 or 15 (paraffins; origin: Exxon Chemical), Exxsol® D 155/170, D 40, D 180/200, D 220/230, D 60, D 70, D 80, D 100, D 110 or D 120 (dearomatised Hydrocarbons; origin: Exxon Chemical), Dowanol® PM, DPM, TPM, PnB, DPnB, TPnB, PnP or DPnP (glycol ethers; origin: Dow Chemical Company), Eastman® EP, EB, EEH, DM, DE, DP or DB (glycol ethers; origin: Eastman Chemical Company), Dowanol® PMA or PGDA (glycol ether esters; origin: Dow Chemical Company) or Eastman® EB acetate, Eastman® DE acetate, Eastman® DB acetate, Eastmanm EEP (all glycol ether esters; all origin: Eastman Chemical Company).

Other solvents suitable for use in the present disclosure include dipropylene glycol, propylene glycol, ethylene glycol ethyl ether acetate, ethylene glycol diacetate, isopropyl myristate, diethyl phthalate, 2-ethylhexyl acetate, methyl n-amyl ketone or di-isobutyl ketone.

In some aspects, the total amount of solvents present in the at least one active composition may vary between 0.0% and 99.9%, alternatively between 30% and 70%, the percentages being relative to the weight of the at least one active composition.

Non-limiting examples of useful thickener ingredients include ethyl cellulose (commercial examples of which are available from Hercules Inc.), fumed silica (commercial examples of which are available from Degussa) and styrene-butadiene-styrene block copolymers (commercial examples of which are available from Shell). Other non-limiting examples include gums and hydrocolloids, such carrageenan and other gellants (including those commonly used in air freshener devices).

In some aspects, the total amount of thickeners present in the at least one active composition may vary between 0.0% and 10%, alternatively between 1% and 4%, the percentages being relative to the weight of the at least one active composition.

Non-limiting examples of useful antioxidant ingredients include sterically hindered amines, i.e. the derivatives of the 2,2,6,6-tetramethyl-piperidine, such as those known under the tradename Uvinul® (origin BASF AG) or Tinuvin® (origin: Ciba Speciality Chemicals), as well as the alkylated hydroxyarene derivatives, such as butylated hydroxytoluene (BHT).

In some aspects, the total amount of antioxidants present in the at least one active composition may vary between 0.0% and 10%, alternatively between 1% and 4%, the percentages being relative to the weight of the at least one active composition.

Other optional ingredients of the at least one active composition comprise dyes. Suitable dyes may be oil-soluble and can be found in the Colour Index International, published by The Society of Dyers and Colourist. Non-limiting examples of suitable dyes include derivatives of the anthraquinone, methine, azo, triarylmethane, triphenylmethane, azine, aminoketone, spirooxazine, thioxanthene, phthalocyanine, perylene, benzopyran or perinone families. Examples of such dyes which are commercially available are known under the tradename Sandoplast® Violet RSB, Violet FBL, Green GSB, Blue 2B or Savinyl® Blue RS (all anthraquinone derivatives; origin: Clariant Huningue S.A.), Oilsol® Blue DB (anthraquinone; origin: Morton International Ltd.), Sandoplast® Yellow 3G (methine; origin: Clariant Huningue S.A.), Savinyl® Scarlet RLS (azo metal complex; origin: Clariant Huningue S.A.), Oilsol® Yellow SEG (monoazo; origin: Morton International Ltd.), Fat Orange® R (monoazo; origin: Hoechst AG), Fat Red® SB (diazo; origin: Hoechst AG), Neozapon® Blue 807 (phtalocyanine; origin: BASF AG), Fluorol® Green Golden (perylene; origin: BASF AG).

In some aspects, the total amount of dyes present in the at least one active composition may vary between 0.0% and 0.5%, alternatively between 0.005% and 0.05%, the percentages being relative to the weight of the at least one active composition.

In some aspects, a bittering agent may be desirable in order to render the product unpalatable, making less likely that the at least one active composition is ingested, especially by young children. Non-limiting examples of bittering agents include isopropyl alcohol, methyl ethyl ketone, methyl n-butyl ketone or yet a denatonium salt such as the denatonium benzoate known also under the trademark Bitrex™ (origin: Mac Farlan Smith Ltd.).

The bittering agent may be incorporated in the at least one active composition in a total amount comprised between 0.0% and 5%, the percentages being relative to the total weight of the at least one active composition. In the case of Bitrex™ the amount can be comprised between 0.0% and 0.1%, alternatively between 0.001% to 0.05% of the total weight of the at least one active composition.

Non-limiting examples of useful UV-inhibitor ingredients, include benzophenones, diphenylacrylates or cinnamates such as those available under the trade name Uvinul® (origin: BASF AG).

In some aspects, the total amount of UV-inhibitors present in the at least one active composition may vary between 0.0% and 0.5%, alternatively between 0.01% and 0.4%, the percentages being relative to the total weight of the at least one active composition.

*Methods Utilizing a Device According to Some Aspects Presented Herein*

Some aspects provide a method of dispensing an at least one active composition into a surrounding space, comprising providing a device according to some aspects presented herein, placing the device into a space, and allowing the at least one active composition to evaporate from the device.

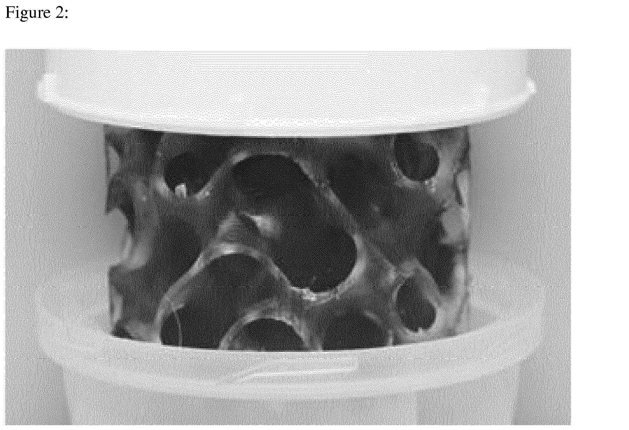

Referring to Figure 2, in some aspects, a device presented herein is incorporated into an air freshener. In some aspects, the air freshener further comprises an airflow shield around the device. Such airflow shield may alter the dispersion of the at least one active composition.

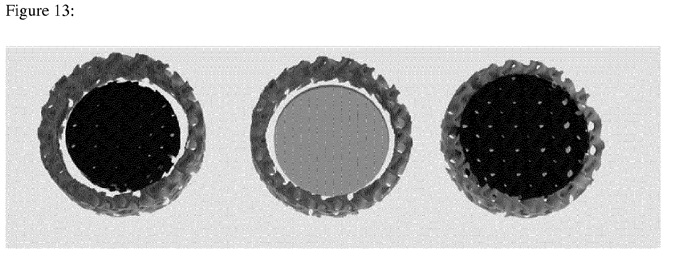

Referring to Figure 13, in one embodiment, the airflow shield comprises:
a second body portion having a structure,

wherein the second body portion is configured to enclose the body portion of a device according to an aspect presented herein,

wherein the structure of the body portion is defined by at least one surface,

wherein the structure has a thickness,

wherein the at least one surface defines a network of a plurality of hollow tubes,

wherein each individual hollow tube within the plurality has a cross section, at least one first end, and at least one second end,

wherein the at least one first and second ends are separated by a distance,

wherein the distance, and the cross section of each individual tube within the plurality defines surface within each individual tube within the plurality,

wherein the surface within each individual tube within the plurality, the distance, the cross section, the first end, and the second end of each individual tube within the plurality defines a volume, and

wherein at least one of the at least one first or second ends intersect an outer surface of the structure.

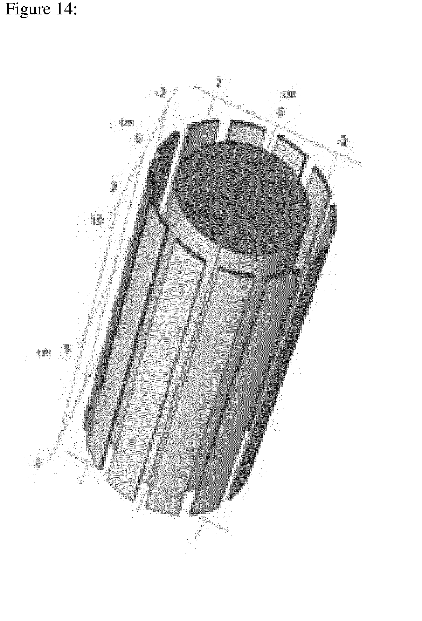

Referring to Figure 14, in one embodiment, the airflow shield comprises a structure comprising vertical slits configured to enclose the body portion of a device according to an aspect presented herein.

Referring to Figures 13 to 15, in some embodiments, the airflow shield is separated from the body portion of a device according to an aspect presented herein by a buffer zone.

Without intending to be limited to any particular theory, the airflow shield allows some air to pass over the body portion of a device according to an aspect presented herein, and allows for control over how much air is allowed to flow through the air freshener, thereby controlling the evaporation rate of the at least one active composition.

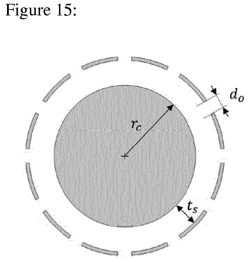

Figure 15 shows a cross-section of the air freshener depicted in Figure 14, wherein the middle circle contains a body portion of a device according to an aspect presented herein. The radius of the body portion of a device according to an aspect presented herein is $r_c$, the thickness of the air flow buffer zone is $t_s$, and the size of the slit openings is $d_o$.

The effect of the airflow shield and its design parameters may be demonstrated using computational fluid dynamics (CFD). The evaporation rate of water (per unit area) from the body portion of a device according to an aspect presented herein may be calculated from Navier Stokes flow equations coupled with mass transport equations, both solved via the Finite Element method on a grid.

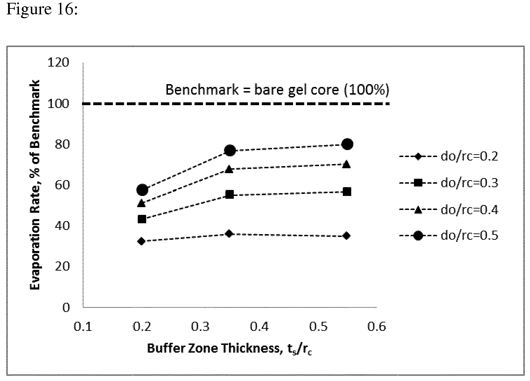

For example, the air freshener devices depicted in Figures 13 and 14 may subjected to a simulated laminar flow of dry air. The evaporation rate of water from the body portion of a device according to an aspect presented herein calculated from CFD for different values of buffer zone thickness ($t_s$) and sizes of the slit openings in the airflow shield ($d_o$). For generality, the latter are scaled by the size of the body portion of a device according to an aspect presented herein ($r_c$), such that the buffer zone thicknesses are given as ($t_s/r_c$) and sizes of slit openings are given as ($t_s/d_o$). Furthermore, evaporation rate from a body portion of a device according to an aspect presented herein lacking an airflow shield, without the airflow shield, is considered 100%. Theoretical results are shown in Figure 16.

### Kits

Some aspects provide a kit, comprising a device according to some aspects presented herein and at least one active composition, wherein a user may select a particular at least one active composition from the at least one active composition, and utilize the selected at least one active composition according to the aspects presented herein.

The present invention is best illustrated but is not limited to the following examples.

### EXAMPLES

**Example 1: A Device According to an Aspect Presented Herein Containing an at Least One Active Composition Formulated as a Kappa-Carrageenan-Based Hydrogel**

*3D-Printing of the Structure:* Structures were designed using MathMod (General Public License version 3.0) comprising a triply periodic minimal surface geometry. The surface of the structures were thickened programmatically by modifying the script developed by Abderrahman Taha (TicknessGenerator-1.0 by Abderrahman Taha 24/08/2015 https://www.facebook.com/permalink.php?story_fbid=772823949501730&id=52951025383 3102). The file was exported and reformatted into an STL file for printing onto a Formlabs SLA printer. The obtained structures were rinsed with Isopropyl alcohol followed by air drying at room temperature.

Preparation of the Hydrogel: Around 5.8 gram of kappa-carrageenan powder was slowly added to 180 gram of 18MΩ water at 85°C while stirring, and the mixture was continuously stirred until the powder was fully dissolved (around 5 min). Next, 0.2 g of Neolone™ (microbicide) was added and a small amount of color (Blue 1 Dualcert). Then a fragrance (2.1 g - see below for the composition) was added and stirring was continued. The obtained hot hydrogel solution was poured into the structures. The structures were cylindrical and had a 50 mm diameter, 50 mm height and internal bore diameter of 16mm. A slight radial gradient was applied in the pore size and the MathMod script for this geometry is provided below. The central bore allowed the structures to be positioned onto a commercially available cone air freshener stand with covers.

| Example | Gelling agent | Fragrance | Water (g) | Gelling agent (g) | Fragrance (g) | Microbicide (g) | Color (g) | Scaffold |
|---|---|---|---|---|---|---|---|---|
| 1a | Ticaloid 710H | Perfumery Composition 1 | 180.3 | 5.8 | 2.1 | 0.2 | 0.02 | SLA Thickene d Gyroid with gradient |
| 1b | Commercial Gelling formulation | Perfumery Composition 1 | 180.0 | 5.8 | 2.1 | 0.2 | 0.02 | SLA Thickene d Gyroid with gradient |

| Raw material | wt% |
|---|---|
| Benzyl acetate | 10.0 |
| Citronellol | 2.0 |
| Coranol[1] | 5.0 |
| Decalactone gamma | 1.0 |
| Delta damascone | 0.1 |
| Dihydromyrcenol | 2.5 |
| Dipropylene glycol | 21.8 |
| Eugenol | 1.0 |
| Florol® [2] | 6.0 |
| Geranyl acetate | 1.5 |
| Hedione® [3] | 3.0 |
| Iso e super[4] | 3.0 |
| Lilial® [5] | 2.0 |
| Linalol | 10.0 |
| Methyl anthranilate | 1.5 |
| Phenylacetaldehyde dimethyl acetal | 0.5 |
| Phenylethyl alcohol | 7.0 |
| (Z)-3-hexenyl 2-hydroxybenzoate | 1.0 |
| Styrallyl acetate | 2.0 |
| Undecalactone gamma | 2.5 |
| Verdox ™ [6] | 7.0 |
| Verdyl acetate[7] | 5.0 |
| Verdyl propionate[8] | 4.0 |

(continued)

| Raw material | wt% |
|---|---|
| 2,4-Dimethyl-3-cyclohexene-1-carbaldehyde[9] | 0.6 |

| |
|---|
| 1) 4-cyclohexyl-2-methyl-2-butanol; origin: Firmenich SA, Geneva, Switzerland<br>2) tetrahydro-2-isobutyl-4-methyl-4(2H)-pyranol; origin: Firmenich SA, Geneva, Switzerland<br>3) Methyl dihydrojasmonate; origin: Firmenich SA, Geneva, Switzerland<br>4) 1-(octahydro-2,3,8,8-tetramethyl-2-naphtalenyl)-1-ethanone; origin: International Flavors & Fragrances, USA<br>5) 3-(4-tert-butylphenyl)-2-methylpropanal; origin: Givaudan-Roure SA, Vernier, Suisse<br>6) 2-tert-butyl-1-cyclohexyl acetate; origin: International Flavors & Fragrances, USA<br>7) mixture of tricyclo[5.2.1.0(2,6)]dec-3-en-8-yl acetate and tricyclo[5.2.1.0(2,6)]dec-4-en-8-yl acetate<br>8) mixture of tricyclo[5.2.1.0(2,6)]dec-3-en-8-yl propanoate and tricyclo[5.2.1.0(2,6)]dec-4-en-8-yl propanoate (b)<br>9) origin: Firmenich SA, Geneva, Switzerland |

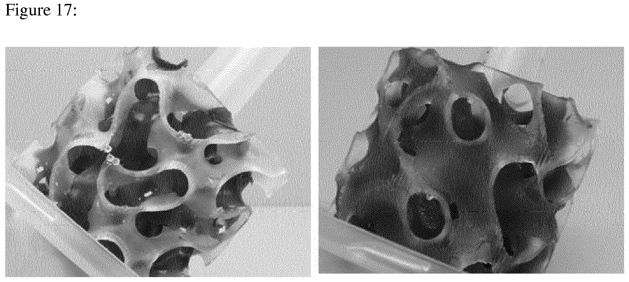

The structures were periodically evaluated and had good olfactory performance. The drying surface receded into the structure, clearly leading to an increase in surface area (see Figure 17 - Example 1a (left), and Example 1b (Right)).

These data suggest that devices according to aspects presented herein can serve as supports for hydrogel air freshener devices, without interfering with the normal function of these systems.

APPENDIX I: Example scripts in Mathmod

```
{
  "MathModels": [
    {
      "Iso3D": {
        "Component": [
          "Gyroid"
        ],
        "Const": [
          "thickness=0.2",
          "heightc=5",
          "Diameter=10"
        ],
        "Funct": [
          "GyroidGrad=mu/100*(cos(x)*sin(y)+cos(y)*sin(z)+cos(z)*sin(x))+(1-
mu/100)*(sin(x)*sin(y)*sin(z)+sin(x)*cos(y)*cos(z)+cos(x)*sin(y)*cos(z)+cos(x)*cos
(y)*sin(z))",
          "DFx=(GyroidGrad(x+0.05,y,z,t)-GyroidGrad(x,y,z,t))/0.05",
          "DFy=(GyroidGrad(x,y+0.05,z,t)-GyroidGrad(x,y,z,t))/0.05",
          "DFz=(GyroidGrad(x,y,z+0.05,t)-GyroidGrad(x,y,z,t))/0.05",

          "Rapport2=(sqrt(DFx(x,y,z,t)*DFx(x,y,z,t)+DFy(x,y,z,t)*DFy(x,y,z,t)+DFz(x,y,z,t)*D
Fz(x,y,z,t)))",
          "Iso3=(GyroidGrad(x-DFx(x,y,z,t)*thickness/Rapport2(x,y,z,t),y-
DFy(x,y,z,t)*thickness/Rapport2(x,y,z,t),z-
DFz(x,y,z,t)*thickness/Rapport2(x,y,z,t),t))",

          "Iso2=(GyroidGrad(x+DFx(x,y,z,t)*thickness/Rapport2(x,y,z,t),y+DFy(x,y,z,t)*thickn
ess/Rapport2(x,y,z,t),z+DFz(x,y,z,t)*thickness/Rapport2(x,y,z,t),t))",
          "TickGyroidGrad=(Iso2(x,y,z,t)*Iso3(x,y,z,t))"
        ],
        "Fxyz": [
          "if(abs(z)>heightc,1,if(x^2+y^2<((Diameter/2)^2-
(z/heightc)),TickGyroidGrad(x,z,y,t),1))"
        ],
        "Name": [
          "Gyroid"
        ],
        "Xmax": [
          "Diameter/2+1"
        ],
        "Xmin": [
          "-Diameter/2-1"
        ],
        "Ymax": [
          "Diameter/2+1"
        ],
        "Ymin": [
```

```
                "-Diameter/2-1"
              ],
              "Zmax": [
                "heightc+1"
              ],
              "Zmin": [
                "-heightc-1"
              ]
            },
            "Sliders": {
              "Max": [
                "100"
              ],
              "Min": [
                "0"
              ],
              "Name": [
                "mu"
              ],
              "Position": [
                "0"
              ],
              "Step": [
                "1"
              ]
            }
          }
        ]
      }
```

Appendix II: Example Script Mathmod with slight gradient.

```
"MathModels": [
   {
     "Iso3D": {
       "Cnd": [
         ""
       ],
       "Component": [
         "Gyroid"
       ],
       "Const": [
         "th=0.5",
         "freq=0.3",
         "gradient=0.05",
         "porosity=0.8",
         "bored=16",
         "heightc=25",
         "Diameter=50",
         "a=1",
         "b=1",
         "c=0"
       ],
       "Funct": [

"GyroidGrad=cos(freq*x)*sin(freq*y)+cos(freq*y)*sin(freq*z)+cos(freq*z)*sin(freq*
x)-porosity+gradient*(a*(x)^2+b*(y)^2+c*z^2)^0.5",
         "DFx=(GyroidGrad(x+0.05,y,z,t)-GyroidGrad(x,y,z,t))/0.05",
         "DFy=(GyroidGrad(x,y+0.05,z,t)-GyroidGrad(x,y,z,t))/0.05",
         "DFz=(GyroidGrad(x,y,z+0.05,t)-GyroidGrad(x,y,z,t))/0.05",

"Rapport2=(sqrt(DFx(x,y,z,t)*DFx(x,y,z,t)+DFy(x,y,z,t)*DFy(x,y,z,t)+DFz(x,y,z,t)*D
Fz(x,y,z,t)))",
         "Iso3=(GyroidGrad(x-DFx(x,y,z,t)*th/Rapport2(x,y,z,t),y-
DFy(x,y,z,t)*th/Rapport2(x,y,z,t),z-DFz(x,y,z,t)*th/Rapport2(x,y,z,t),t))",

"Iso2=(GyroidGrad(x+DFx(x,y,z,t)*th/Rapport2(x,y,z,t),y+DFy(x,y,z,t)*th/Rapport2(x
,y,z,t),z+DFz(x,y,z,t)*th/Rapport2(x,y,z,t),t))",
         "TickGyroidGrad=(Iso2(x,y,z,t)*Iso3(x,y,z,t))"
       ],
       "Fxyz": [

"if(x^2+y^2<(bored/2)^2,1,if(abs(z)>heightc,1,if(x^2+y^2<(Diameter/2)^2,TickGyroi
dGrad(x,z,y,t),1)))"
       ],
       "Name": [
         "Gyroid"
```

```
                        ],
                        "Xmax": [
                            "((Diameter/2)^2-y^2)^(0.5)"
                        ],
                        "Xmin": [
                            "-((Diameter/2)^2-y^2)^(0.5)"
                        ],
                        "Ymax": [
                            "((Diameter/2)^2-x^2)^(0.5)"
                        ],
                        "Ymin": [
                            "-((Diameter/2)^2-x^2)^(0.5)"
                        ],
                        "Zmax": [
                            "50"
                        ],
                        "Zmin": [
                            "-50"
                        ]
                    }
                }
            ]
        }
```

In the next example, tuning of the release rates of an active composition using the device is shown.

**Example 2: A Comparison of Devices According to Aspects Presented Herein Containing an at Least One Active Composition Formulated as a Kappa-Carrageenan-Based Hydrogel**

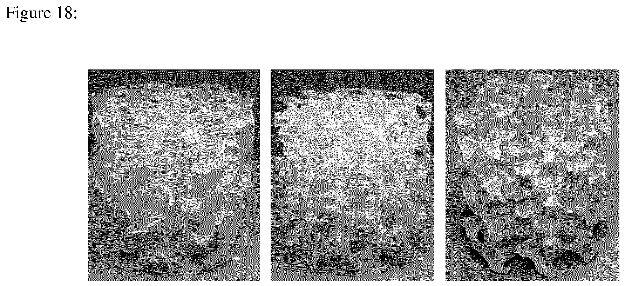

*3D-Printing of the Structures:* Referring to Figure 18, structures were designed using MathMod (General Public License version 3.0) comprising a triply periodic minimal surface gyroid geometry. The structures were 3D printed using Formlabs Clear Resin (GPCL04) in a Formlabs Form 2 printer. The structures were cylindrical and had a 50 mm diameter and 50 mm height without a central bore.

Eight gels were tested: two replicates of three different structures as well as two controls. The three different structures were a single gyroid (referred to as gyroid), a thickened gyroid with a gradient, and gyroid with a gradient for which one of the two volumes was filled in, as shown in Figure 18. All eight samples were tested concurrently to minimize the effect of small variations in temperature/humidity.

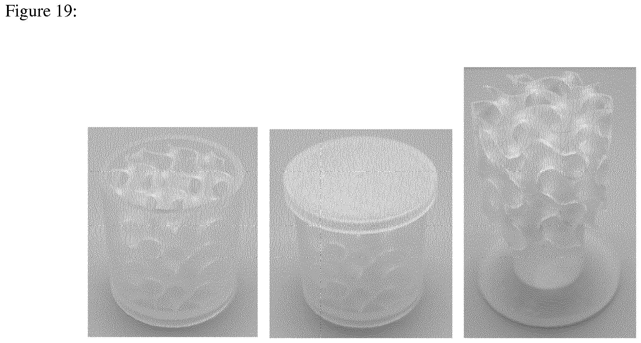

*Preparation of Gel Casting Accessories and Holders:* Various accessories and holders were 3D printed including cups, lids, bases, and posts. Each cup was designed to hold a disk-shaped base and a device and had a hole in the bottom used to push a device out. The disk-shaped base was designed to be placed in a cup under a device in order to facilitate the device's removal from the cup. Cup lids were designed to go onto the cup after pouring the gel in order to keep the top surfaces of each gel flat and uniform. Base stands were designed for finished gel-device prototypes to be placed upon during testing. The base stand enbled much of the bottom surface of the device to be open to the air. These 3D accessories and holders are shown in Figure 19.

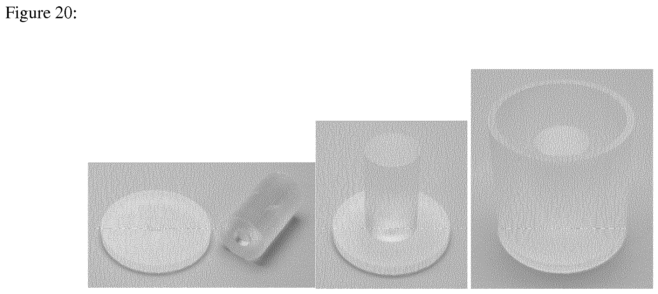

Posts were designed as controls. They had the same volume as the gyroid structure but, instead of the gyroid shape, it was condensed into a cylinder shape. This allowed for the same amount of gel to be used with the controls and the gyroid devices. On the bottom surface, the post had a fitting in order to fit into a disk shaped base with a notch. These would then be placed in a cup the same way as a device and base were. The notch allowed for the post to be centered inside the cup. These 3D prints are shown in Figure 20. All accessories were designed using Onshape software and printed using Formlabs Clear Resin (GPCL04) in a Formlabs Form 2 SLA Printer.

*Hydrogel Preparation:* One gram of Neolone™ (microbicide) was added to a 960.5 g DI water bath at around 79°C while the bath was being stirred at around 300 rpm. The bath was then heated above 80°C (80.5-87°C) and 28.50 grams of a commercial gelling formulation primarily based on kappa-carrageenan were slowly added while stirring at around 435 rpm. The mixture was continuously stirred for about 30 minutes at around 520 rpm and the carrageenan powder was fully dissolved. The heat was then shut off and the mixing turned down to around 430 rpm to add fragrance. 10 grams of Fragrance Composition 2 (see below) was added to the mixture at temperatures 72-76°C. The heat was then turned back on and the mixer was turned up to around 575 rpm for about 2 minutes. The heat was then shut off and, in order to account for evaporation of water during the mixing process, the whole mixture was weighed and water was added back until there were about 960.5 grams of water. The mixture was then mixed for 2 minutes at around 515 rpm. The obtained hot hydrogel solution was then poured into a 3D printed structure.

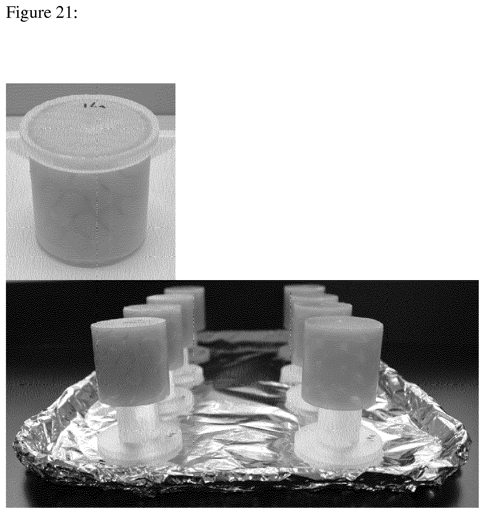

Gel Casting and Controls: A disk-shaped base was placed into each cup and some hot gel was poured on top of the base. A device was then placed into the cup with some gel and more hot gel was poured over the device until the cup was filled to the top. A lid was then placed onto the cup, shown in Figure 21.

For the controls, the disk-shape base with a notch and the post were placed into an empty cup. Hot gel was then poured into the cup and filled to the top and a lid was placed on the cup. The controls and devices with gel were then sealed with paraffin and aluminum foil and placed in a refrigerator overnight to complete the gel casting. After being cast, the gels were removed from cups so that all that remained were devices with gel or posts with gel. They were then placed on base stands for testing, as in Figure 21.

Fragrance Composition 2:

| Raw material | % |
|---|---|
| ACETYLMETHYLCARBINOL | 8 |
| ACETYLPROPIONYLE | 2 |
| AMYL SALICYLATE FIRINC | 13 |
| CARBINOL PV | 0.1 |
| COUMARIN | 2 |
| DIHYDROMYRCENOL PURE | 18 |
| DYNASCONE | 0.5 |
| HERBALDEHYDE | 0.5 |
| HEXYL ACETATE | 0.9 |
| ISONONYL ACETATE | 5 |
| LAVANDIN GROSSO ARR | 28 |
| LAVANDIN SUMIAN | 9 |
| METHYL OCTINECARBONATE | 0.5 |
| TERPINYL ACETATE | 12 |
| TRIFERNAL | 0.5 |

1) 2-Butanone, 3-hydroxy-
2) 2,3-Pentanedione
3) Benzoic acid, 2-hydroxy-, pentyl ester
4) 1-Octen-3-ol
5) 2H-1-Benzopyran-2-one
6) 7-Octen-2-ol, 2,6-dimethyl-
7) 4-Penten-1-one, 1-(5,5-dimethyl-1-cyclohexen-1-yl)-
8) Hexanal, 3,5,5-trimethyl-
10) 1-Hexanol, 3,5,5-trimethyl-, acetate
13) 2-Nonynoic acid, methyl ester
15) Benzenepropanal, .beta.-methyl-

Referring to Figure 21, the test and control castings were placed on 3D printed stands for evaporation. The gels were periodically removed from the controlled room to be weighed, photographed, and evaluated.

*Results:* At the beginning of the test, the character and intensity of the fragrance from the gels incorporated into devices was the same or close to that of the control gels. However, the control gels gave off a marine note from the carrageenan base that was absent from the gels incorporated into devices. Towards the end of the test, the gels incorporated into devices continued to smell fragrant while the controls hardly gave off any odor. Specifically the single and thickened gyroid devices were noted to give off the strongest fragrance intensity towards the end of the test.

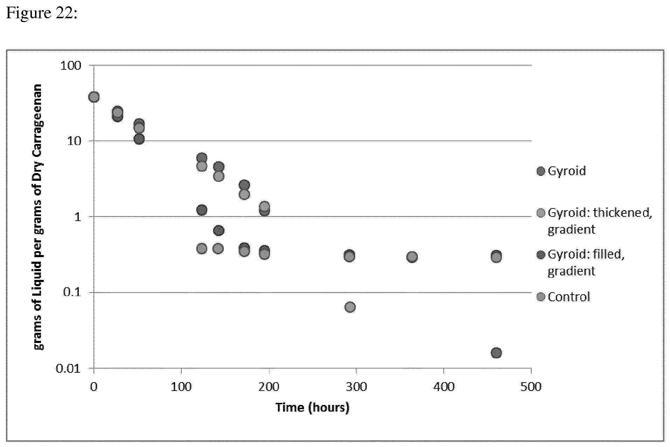

*Weight Loss:* Weight loss due to water and fragrance evaporation is shown in Figure 22, where the mass of liquid per mass of dry carrageenan is plotted over time. Figure 22 shows that, at the beginning of the test, the gels incorporated into devices and control gels all performed similarly. However, over time, the gels incorporated into devices lost moisture and fragrance at a slower rate than that of the control gels. At 100 and 150 hours, the filled in gyroid device continued to lose mass while the liquid content of the control had leveled off. Between the time points of 100 and 200 hours, gels in the single and thickened gyroid devices also continued to lose liquid when the control had stopped losing any weight.

After 200 hours, the gels from the control and the gel incorporated into the filled in device stopped losing mass whereas the gels incorporated into either the single or thickened gyroid devices continued to lose mass. The gels incorporated into either the single or thickened gyroid devices were also able to lose a higher percentage of their liquid weight than the control and the gels incorporated into either the filled-in devices.

The values on the y-axis were calculated knowing the moisture contents of the carrageenan powder as well as the water and fragrance content of the final gel mixture. Mass loss due to water and fragrance evaporation was measured. A log scale was used because the performance of the scaffolds and the controls mostly differed at later time points and it allows these differences to be seen more easily. Slight negative values were removed (none below - 0.09 g of liquid per g of dry carrageenan) and any data points below 0.01 were deemed insignificant. Data from replicate experiments are not shown, but are similar to the results shown in this plot.

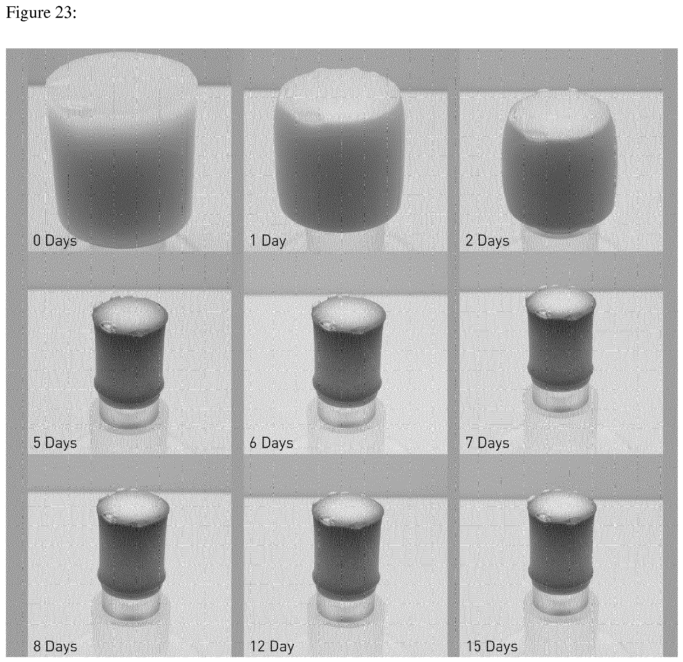

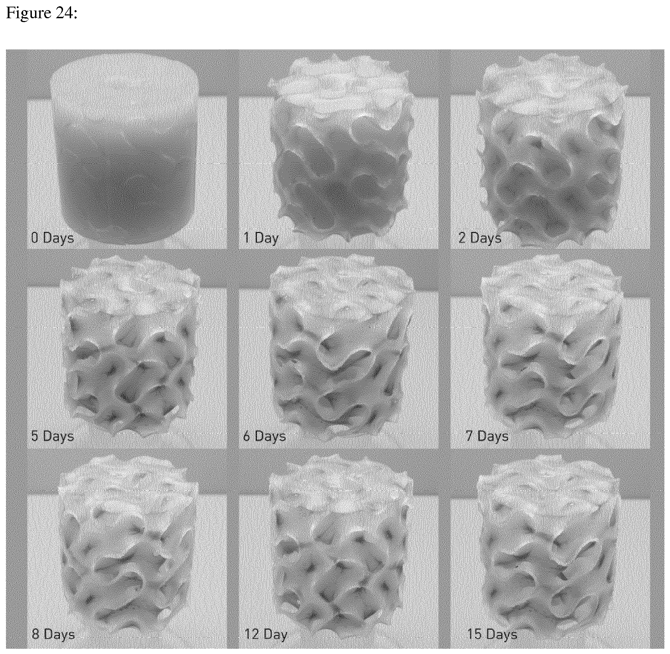

*Visual Results:* The gels incorporated into devices and control gels had very different appearances as they dried. The control gels shrank uniformly (maintaining their cylindrical shape) over time while the gels incorporated into devices dried to the shape of the structure surface and, eventually, holes developed leaving the inside of the gel open to the air. The time sequence of a control and a gel incorporated into a single gyroid device are shown in Figures 23 and 24.

*Conclusion:* The geometries used in the test devices changed the release mechanics of liquids from gels when compared to a more typical prior art gel air freshener. The test devices were able to maintain liquid delivery into the air for longer than the gels without a 3D printed structure. This middle to end time of the air freshener's life, shown at around 100-200 hours in this example, is around when prior art air fresheners tend to slow down in delivering fragrance. The devices, however, extend water and fragrance evaporation well into this time period.

The control air freshener also appeared to have trapped some liquid inside the gel, while some of the test device geometries lessened the amount of liquid trapped inside the gel. Overall, using 3D printed mathematical surfaces as devices for gel favorably impacts how water and fragrance are released from gels.

**Example 3: A Dual-Release Device According to Aspects Presented Herein Containing Two Active Compositions Formulated as a Kappa-Carrageenan-Based Hydrogels**

The following two hydrogels were formulated according to the methods described above:

| Ingredient | Gel A | Gel B |
|---|---|---|
| **Water 80°C** | 180.1 | 180.15 |
| **Carrageenan Ticaloid 710H** | 5.74 | 5.81 |
| **Color** | 0.01005 | 0.03020 |
| **Fragrance** | 1.61 Fragrance Composition 3 | 1.62 Fragrance Composition 1 |
| **Neolone** | 0.24 | 0.22 |

Perfumery Composition 3:

| Raw material | % |
|---|---|
| Benzyl acetate | 2.50 |

(continued)

| Raw material | % |
|---|---|
| Hexyl acetate | 5.40 |
| Prenyl acetate | 0.40 |
| Aldehyde C10 | 1.60 |
| Allyl amyl glycolate | 0.50 |
| Undecalactone gamma | 1.50 |
| Calone® [1] | 0.15 |
| Ethyl caproate | 0.30 |
| Cetalox® [2] | 0.15 |
| Citronellol | 2.20 |
| Verdyl acetate[3] | 1.00 |
| Delta damascone | 0.10 |
| Dihydromyrcenol | 7.00 |
| Dipropylene glycol | 35.00 |
| 4-(1,1-diméthyléthyl)-1-cyclohexyl acetate[5] | 3.30 |
| Eugenol | 0.50 |
| Farenal[5] | 0.30 |
| Floralozone[6] | 1.00 |
| Iso e super[7] | 1.60 |
| Isoraldeine[8] | 0.50 |
| Lavandin grosso | 1.30 |
| Lilial® [9] | 4.00 |
| 2,6-diméthyl-5-heptanal[10] | 1.00 |
| Menthone | 0.50 |
| Crystal moss | 0.30 |
| Ethyl 2 methylbutyrate | 2.20 |
| Neobutenone® [11] alpha | 0.10 |
| Ethyl oenanthate | 1.10 |
| Phenylethyl alcohol | 2.20 |
| Precyclemone b[12] | 0.60 |
| Rosemary oil | 0.60 |
| Hexyl salicylate | 4.40 |
| (Z)-3-hexenyl 2-hydroxybenzoate | 1.10 |
| Scentenal® [13] | 0.60 |
| Mixture of terpenes from orange | 5.50 |
| Verdox™ [14] | 6.60 |
| Beta ionone | 2.00 |

(continued)

| Raw material | % |
|---|---|
| 2,4-Dimethyl-3-cyclohexene-1-carbaldehyde[165] | 0.90 |

| |
|---|
| 1) 7-Methyl-2H,4H-1,5-benzodioxepin-3-one; origin: Firmenich SA, Geneva, Switzerland<br>2) dodecahydro-3a,6,6,9a-tetramethyl-naphtho[2,1-b]furan; origin: Firmenich SA, Geneva, Switzerland<br>3) mixture of tricyclo[5.2.1.0(2,6)]dec-3-en-8-yl acetate and tricyclo[5.2.1.0(2,6)]dec-4-en-8-yl acetate<br>4) origin: Firmenich SA, Geneva, Switzerland<br>5) 2,6,10-trimethyl-9-undecenal; origin: Symrise AG, Germany<br>6) 3-(4/2-Ethylphenyl)-2,2-dimethylpropanal; origin: International Flavors & Fragrances, USA<br>7) 1-(octahydro-2,3,8,8-tetramethyl-2-naphtalenyl)-1-ethanone; origin: International Flavors & Fragrances, USA<br>8) N-methyl ionone; origin: International Flavors & Fragrances, USA<br>9) 3-(4-tert-butylphenyl)-2-methylpropanal; origin: Givaudan-Roure SA, Vernier, Suisse<br>10) Origin: Givaudan-Roure SA, Vernier, Suisse<br>11) 1-(5,5-dimethyl-1-cyclohexen-1-yl)-4-penten-1-one ; origin: Firmenich SA, Geneva, Switzerland<br>12) (+-)-1-methyl-4-(4-methyl-3-pentenyl)-3-cyclohexene-1-carbaldehyde; origin: International Flavors & Fragrances, USA<br>13) 8(9)-méthoxy-tricyclo[5.2.1.0.(2,6)]décane-3(4)-carbaldehyde; origin: Firmenich SA, Geneva, Switzerland<br>14) 2-tert-butyl-1-cyclohexyl acetate; origin: International Flavors & Fragrances, USA |

An exemplary device is shown in Figure 11. The performance of this hydrogel air freshener was clearly different from prior art gel air fresheners in that it clearly delivered two unique fragrances, two different colors, and providing a unique aesthetically pleasing air freshener device. The flexibility that 3D printed devices provide is clearly demonstrated. We could envision that it may be possible to use other equations (e.g. double gyroid) to possibly deliver 3 or even more fragrances at a time.

**Example 4: Devices According to Aspects Presented Herein Containing an at Least One Active Compositions Formulated as Oleogels**

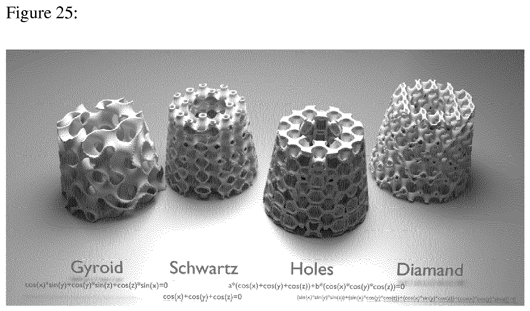

Figure 25 shows four structures that were designed using MathMod, and 3D printed using an SLA printer (Formlabs Form 2 with Clear Resin GPCL04). Four different oleogels were prepared by mixing and heating fragrance oil (~65-80°C), adding the oleogelators, dissolving it while stirring, according to the formulations provided in the table below. Small amounts of colorant were added (e.g. yellow lake 5 and/or yellow lake 6) to examples 4-1 and 4-4 below.

Test Formulations:

| Example | Scaffold | Gelator Wax | Weight Wax (g) | Fragrance Composition | Weight Fragrance (g) |
|---|---|---|---|---|---|
| 4-1 | Gyroid | Candelilla | 80.04 | 4 | 122.02 |
| 4-2 | Schwarz Primitive | Candelilla | 40.08 | 2 | 163.05 |
| 4-3 | Holes | Beeswax | 40.01 | 4 | 162.85 |
| 4-4 | Schwarz Diamond | Carnauba | 40.08 | 2 | 162.90 |

Perfumery Composition 4:

| Raw material | % |
|---|---|
| ALDEHYDE C 10 | 6 |
| ALDEHYDE C 8 | 2 |

(continued)

| Raw material | % |
|---|---|
| CITRAL [1] | 20 |
| CITRONELLA OIL JAVA | 10 |
| CITRONELLYL NITRILE | 10 |
| DIHYDROMYRCENOL PURE [2] | 12 |
| GERANIOL P [3] | 15 |
| GERANYL ACETATE EXTRA [4] | 5 |
| ORANGE TERP BM CITRONOVA FAB | 10 |
| TERPINOLENE [5] | 10 |
| 1. 2,6-Octadienal, 3,7-dimethyl-<br>2. 7-Octen-2-ol, 2,6-dimethyl-<br>3. 2,6-Octadien-1-ol, 3,7-dimethyl-, (E)-<br>4. 2,6-Octadien-1-ol, 3,7-dimethyl-, acetate, (E)-<br>5. Cyclohexene, 1-methyl-4-(1-methylethylidene)- | |

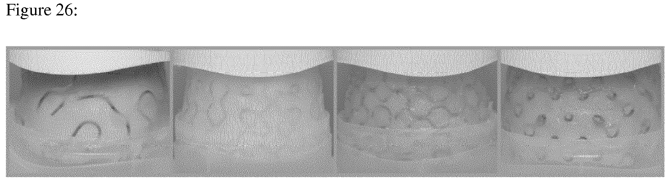

The printed devices were placed in a glass beaker and the warm oleogel liquid was poured over the device. This was then covered with Parafilm and placed in refrigerator. Samples were removed from the jar and excess oleogel on the surfaces was removed. The prototypes were then placed into commercially available cone air freshener holders (see Figure 26).

*Results:* These oleogel based air freshener devices were very powerful, since they are much higher in fragrance content (60-80% by mass) than a hydrogel air freshener. At these high oil levels some of these oleogels created a gel or viscous liquid with a buttery, petroleum-jelly like consistency. These formulations would be unsuitable for a traditional cone air freshener device. Surprisingly, with these 3D printed structures, these oleogel formulations could now be used, allowing the oleogel to conform itself to the shape of the object without showing signs of sagging or other deformation.

*Conclusion:* 3D printed devices are suitable not only for hydrogel air fresheners, but can be expanded to any solid, semi-solid or even viscous liquid format of a fragrance. It may be desirable, for example, to maximize the fragrance level to formulations that are not self-supporting. In those cases these scaffolds would provide an additional benefit.

**Example 5: Estimation of the Surface Area of Devices According to Aspects Presented Herein**

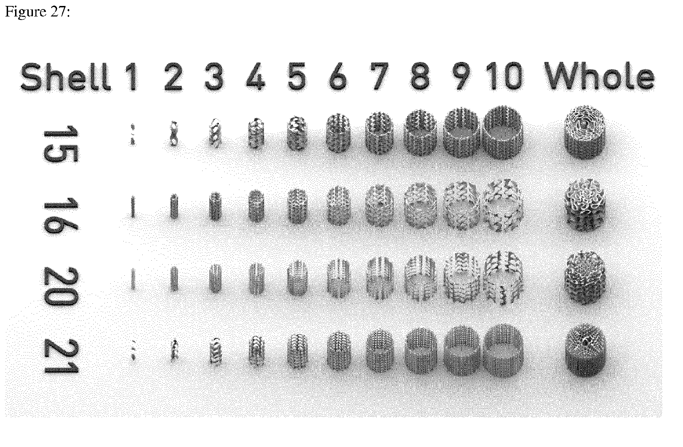

Figure 27 shows the process by which the surface area of a device according to an aspect presented herein may be estimated. In short, a computer script sections a device into several concentric "cores", for which the approximate surface area (in cm$^2$) of the scaffolds can then be calculated.

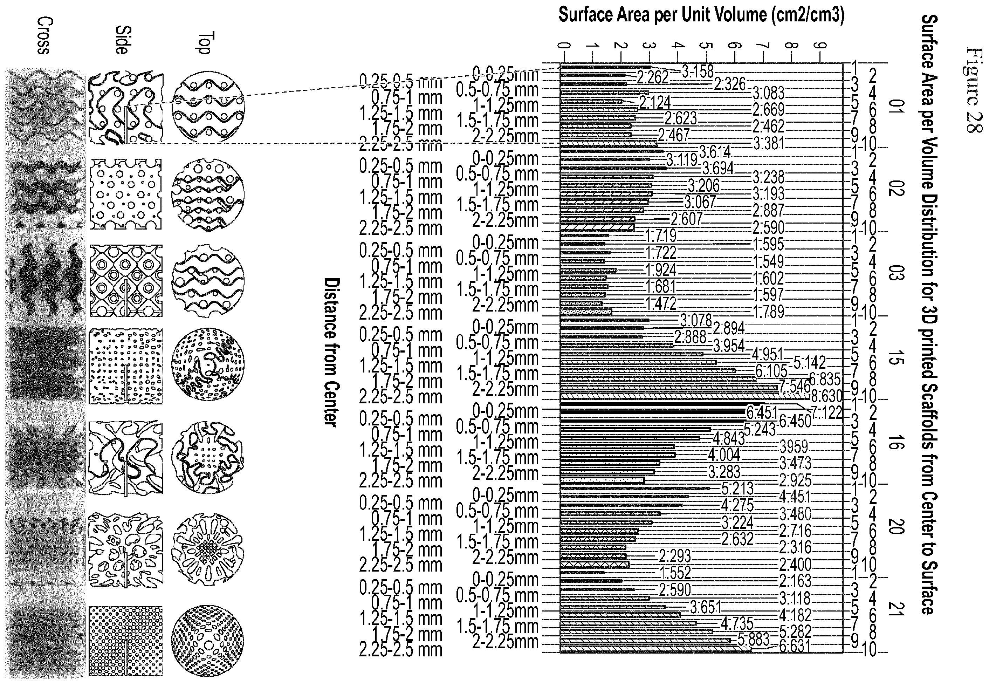

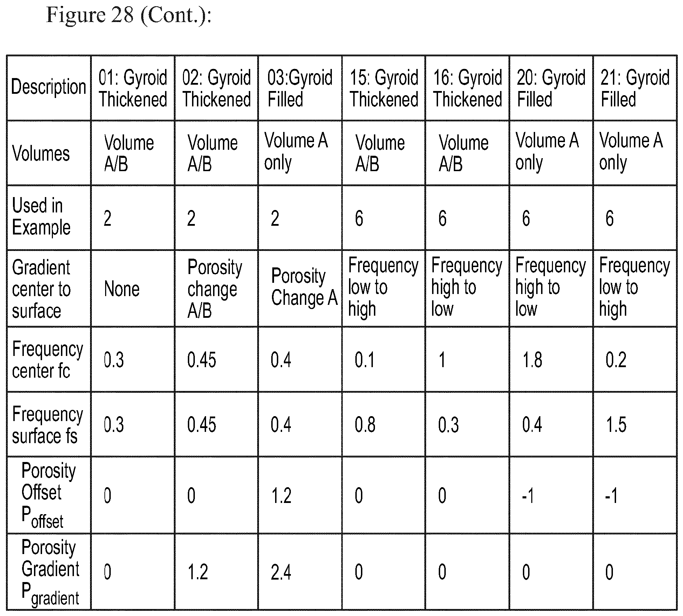

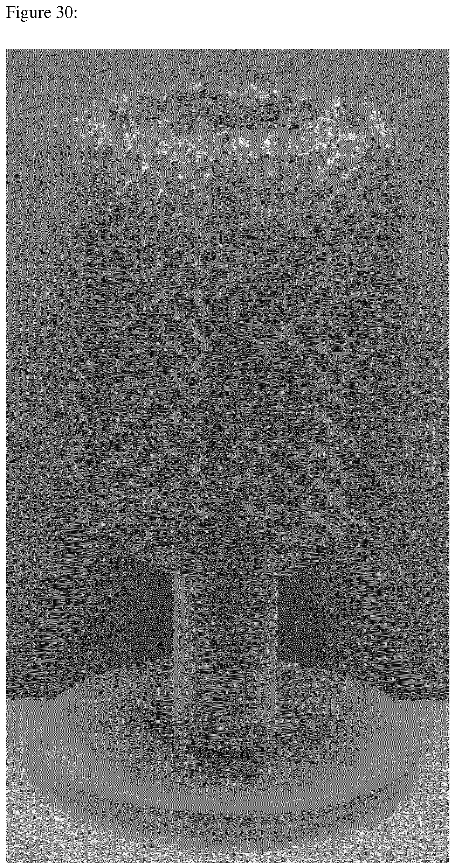

Referring to Figures 28 and 30, structures generally conform to the following gyroid equation:

$$\cos(x) \cdot \sin(y) + \cos(y) \cdot \sin(z) + \cos(z) \cdot \sin(x) = 0 \quad \textbf{Equation 5}$$

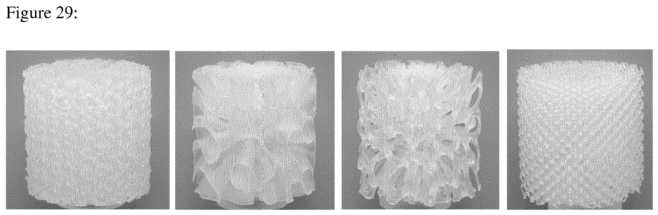

Equation 5 was modified to allow for porosity and frequency gradients for objects with radius *r*. All the devices shown in Figure 29 can essentially be described by the following generalized equation:

$$\cos\left(\left(f_c + (f_s - f_c)\right) \cdot \left(\tfrac{\sqrt{x^2+y^2}}{r}\right)x\right) \cdot \sin\left(\left(f_c + (f_s - f_c)\right) \cdot \left(\tfrac{\sqrt{x^2+y^2}}{r}\right)y\right) + \cos\left(\left(f_c + (f_s - f_{sc})\right) \cdot\right.$$

$$\left(\tfrac{\sqrt{x^2+y^2}}{r}\right)y\right) \cdot \sin\left(\left(f_c + (f_s - f_c)\right) \cdot \left(\tfrac{\sqrt{x^2+y^2}}{r}\right)z\right) + \cos\left(\left(f_c + (f_s - f_c)\right) \cdot \left(\tfrac{\sqrt{x^2+y^2}}{r}\right)z\right) \cdot$$

$$\sin\left(\left(f_c + (f_s - f_c)\right) \cdot \left(\tfrac{\sqrt{x^2+y^2}}{r}\right)x\right) - P_{offset} + P_{gradient}\left(\tfrac{\sqrt{x^2+y^2}}{r}\right) = 0 \quad \text{Equation 6}$$

Various values of frequency at the center *(f_c)* and the surface *(f_s)*, the Porosity offset *(P_{offset})* and its gradient *(P_{gradient})* are provided in the table in Figure 28.

As can be seen from the top chart in the same figure, the three prototypes that were used in Example 2 above contained relatively large holes, with surface areas around 1.5-3 $cm^2/cm^3$ with only mild gradients. The prototypes used in Example 6 below have considerably higher surface areas (~ 3-5 $cm^2/cm^3$) and the gradients are much more pronounced, resulting in regions with large surface area (e.g. 8 $cm^2/cm^3$).

Another metric that we have evaluated is a *count* of the number of surfaces encountered when traveling in a straight line from one surface point through the center to another surface along randomly angled lines at random heights (z axis). This may be considered to be a semi-quantitative measure of the *complexity, intricacy or tortuosity* of the object. Following the line in the cross-section depicted in Figure 28, depending on the z-value 2 or 3 surfaces are encountered before exiting the geometry. The blue line of Gyroid 15 shows that one would easily encounter 6 or more surfaces before exiting (depending on the height chosen). Since surfaces occur where equation 6 equals zero (aka the *roots of the function*) a root finding algorithm was employed in *R* to find the min, max, mean and median values of the number of surfaces or roots (see table below). Please note that since Gyroid 3, 20 and 21 have only one volume that is occupied with gel, the volumes occupied with plastic were excluded and therefore the number of surfaces were divided by two.

| Scaffold | 1 | 2 | 3* | 15 | 16 | 20* | 21* |
|---|---|---|---|---|---|---|---|
| Max. number of surfaces | 7 | 8 | 8/2=4 | 20 | 13 | 22/2=11 | 29/2=14.5 |
| Mean number of surfaces | 3.8 | 4.9 | 4.6/2=2.3 | 11.4 | 7.3 | 10.6/2=5.3 | 15.2=7.6 |
| Median number of surfaces | 4 | 5 | 4/2=2 | 11 | 7 | 10/2=5 | 15/2=8 |

It is clear that scaffold 15 and 21 are the most intricate, whereas the scaffolds of example 2 (Gyroid 1, 2 and 3) were less elaborate.

## Example 6: Mass Loss and Fragrance Release from Devices According to Aspects Presented Herein

Devices were designed using MathMod (https://sourceforge.net/projects/mathmod/) and are described before and shown in Figure 27 (Gyroid 15, 16, 20 and 21). Devices were 3D printed using Formlabs Clear Resin (GPCL04) in a Formlabs Form 2 printer (see Figure 30). The devices were cylindrical and had a 50 mm diameter and 50 mm height. Six fragrance gels were tested: four different devices as well as two controls. They were tested concurrently in separate sink booths that are designed for sensory evaluation and have active controls for maintaining reasonable constant levels in air flow, temperature and relative humidity.

Similar to Example 2, various accessories and holders were 3D printed including cups, lids, bases, and posts. While all four devices fit into the same size cup during gel casting, the amount of material, and therefore plastic volume, differed slightly for each one. Cylindrical posts were designed as controls to cover the range of plastic volumes of the devices. The post of *ControlHi* was designed to displace the same gel volume and mass, as the biggest scaffold being tested and the other *ControlLo* post as the smallest scaffold being tested. However, upon inspection of the data it was observed that Gyroid 15 actually was slightly outside this envelope (it contained less gel). This is not considered to be a major problem and makes the performance of this scaffold even more surprising since it clearly outperformed the controls while containing less actives. Other than size, the post designs were the same as those described in Example 2.

One gram of Neolone™ (microbicide) was added to 960.5 g of blue dyed DI water in a beaker being stirred at around 200 rpm and held in a temperature controlled bath. The bath was then heated above 80°C (80.6-87°C) and 28.6 gram of gelling formulation primarily based on kappa-carrageenan (Ticaloid 710H) was slowly added while stirring at around 290 rpm. The mixture was continuously stirred for about 23 minutes at around 600 rpm and the carrageenan powder was fully dissolved. The heat was then shut off and the mixing turned down to around 60 rpm to add fragrance. 10 gram of fragrance (See table below) was added to the mixture at around 79°C. The mixer was then turned up to around 600 rpm for about a minute. In order to account for evaporation of water during the mixing process, the whole mixture was then weighed and water was added back until there was about 960.5 gram of water. The mixture was then mixed for about a minute at around 600 rpm. The obtained hot hydrogel solution was then poured into a 3D printed device.

Perfume Composition:

| Raw material | wt% |
|---|---|
| Benzyl acetate | 10.0 |

(continued)

| Raw material | wt% |
| --- | --- |
| Citronellol | 2.0 |
| Coranol[1] | 5.0 |
| Decalactone gamma | 1.0 |
| Delta damascone | 0.1 |
| Dihydromyrcenol | 2.5 |
| Dipropylene glycol | 21.8 |
| Eugenol | 1.0 |
| Florol® [2] | 6.0 |
| Geranyl acetate | 1.5 |
| Hedione® [3] | 3.0 |
| Iso e super[4] | 3.0 |
| Lilial® [5] | 2.0 |
| Linalol | 10.0 |
| Methyl anthranilate | 1.5 |
| Phenylacetaldehyde dimethyl acetal | 0.5 |
| Phenylethyl alcohol | 7.0 |
| (Z)-3-hexenyl 2-hydroxybenzoate | 1.0 |
| Styrallyl acetate | 2.0 |
| Undecalactone gamma | 2.5 |
| Verdox™ [6] | 7.0 |
| Verdyl acetate[7] | 5.0 |
| Verdyl propionate[8] | 4.0 |
| 2,4-Dimethyl-3-cyclohexene-1-carbaldehyde[9] | 0.6 |

1) 4-cyclohexyl-2-methyl-2-butanol; origin: Firmenich SA, Geneva, Switzerland
2) tetrahydro-2-isobutyl-4-methyl-4(2H)-pyranol; origin: Firmenich SA, Geneva, Switzerland
3) Methyl dihydrojasmonate; origin: Firmenich SA, Geneva, Switzerland
4) 1-(octahydro-2,3,8,8-tetramethyl-2-naphtalenyl)-1-ethanone; origin: International Flavors & Fragrances, USA
5) 3-(4-tert-butylphenyl)-2-methylpropanal; origin: Givaudan-Roure SA, Vernier, Suisse
6) 2-tert-butyl-1-cyclohexyl acetate; origin: International Flavors & Fragrances, USA
7) mixture of tricyclo[5.2.1.0(2,6)]dec-3-en-8-yl acetate and tricyclo[5.2.1.0(2,6)]dec-4-en-8-yl acetate
8) mixture of tricyclo[5.2.1.0(2,6)]dec-3-en-8-yl propanoate and tricyclo[5.2.1.0(2,6)]dec-4-en-8-yl propanoate (b)
9) origin: Firmenich SA, Geneva, Switzerland

Gel Casting and Controls: A disk-shaped base was placed into each cup and some hot gel was poured on top of the base. The devices were then placed into the cup with some gel and more hot gel was poured over the device until the cup was filled to the top. A lid was then placed onto the cup, as described in Example 2.

For the controls, the disk-shape base with a notch and the post were placed into an empty cup. Hot gel was then poured into the cup and filled to the top and a lid was placed on the cup. The controls and devices with gel were then sealed with paraffin and aluminum foil and placed in a refrigerator overnight to complete the gel casting. After being cast, the gels were removed from cups so all that remained were devices with gel or posts with gel. A thin layer of excess gel was removed from controls and scaffold to ensure that the devices were exactly positioned 'flush' with the gel surface. They were then placed on base stands for testing, as is shown by one sample in Figure 30.

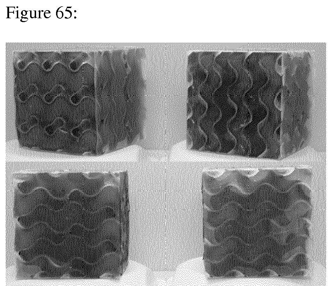

After the gels were cast, they were placed on 3D printed stands on the floor of sensory booths to allow for sensory evaluation during a typical air freshener use scenario. The gels were periodically removed from the booths to be weighed and photographed. See Figure 65.

In addition, samples were periodically switched between individual booths to eliminate any bias based on small environmental differences. The temperature in two of the five chambers was independently recorded every 10 minutes with temperature humidity loggers (iButton, DS1923 Hygrochron). The two chambers showed average temperatures of 19.1°C $\pm$ 0.9 and 19.4°C $\pm$ 0.9 with a relative humidity of 55.1% $\pm$ 3.4 and 55.1$\pm$ 3.4 respectively.

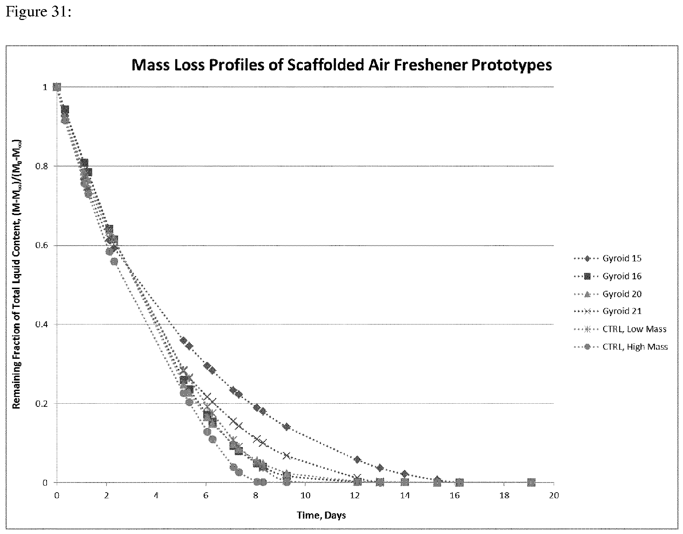

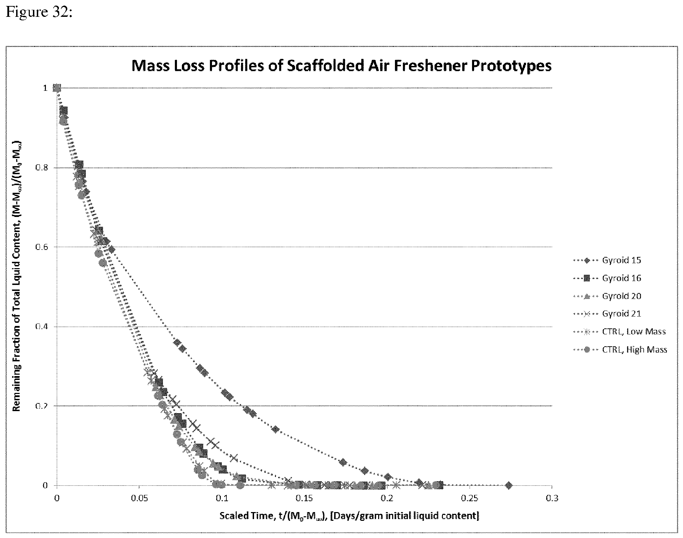

*Weight Loss Results:* Weight loss due to water and fragrance evaporation is shown in Figures 31 and 32. Figure 31 provides a view where the dimensionless mass loss is plotted versus time. In Figure 32, the x-axis is normalized and scaled such that small individual differences in weight of the hydrogel are essentially eliminated to truly compare the rate of evaporation on an equal footing.

It is evident from this data that the two controls lost their mass very quickly. The device containing air fresheners initially had a very similar release rate. After about 3-7 days this rate started to diminish leading to a more sustained release, drying more gradually than the controls. It is clear as well that after about 3 weeks all air fresheners were essentially completely dried out. It appears that the two devices with the smallest holes on the outside were most efficient in reducing the drying rate. This is most likely caused by a 'boundary effect', where the humidity released from the gel is 'trapped' inside the device, reducing the humidity/fragrance gradient, reducing the overall mass transfer rate. This is useful feature, since this allows for tuning the longevity of an air freshener, without necessarily adding more hydrogel.

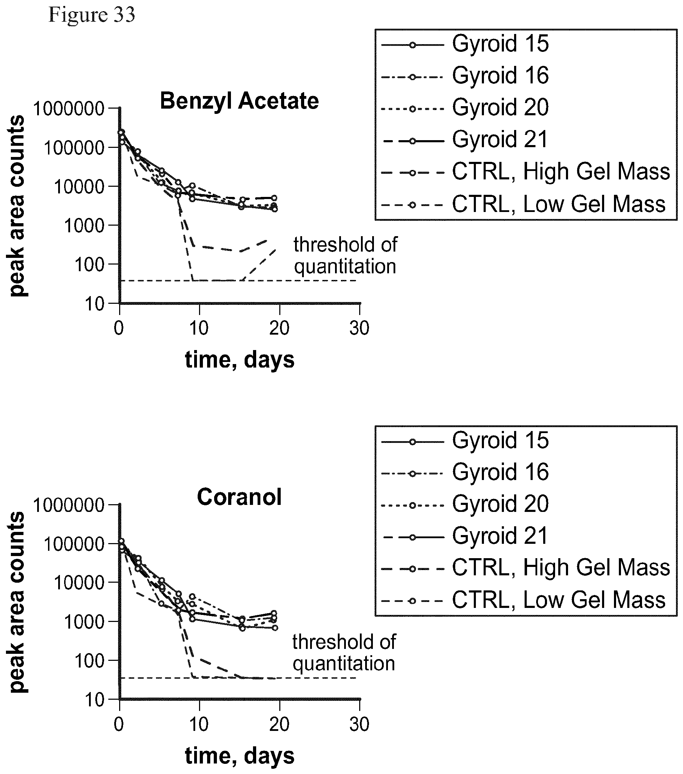

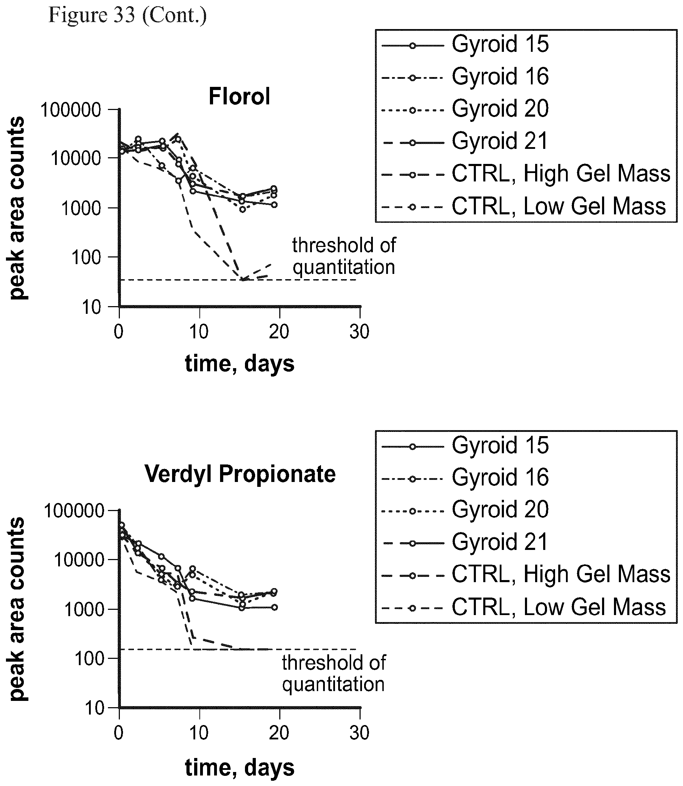

*Fragrance Release measurements using SPME headspace:* The fragrance concentration in the sink booths was measured by sampling the headspace (SPME) on the same days as the sensory panels. A SPME fiber was positioned into each booth for a duration of 35 minutes, while the door was closed. The compounds collected by the fiber were immediately injected on an Agilent 7890A gas chromatograph (GC) equipped with an Agilent 5975C mass spectrometer (MSD). The GC column was an Agilent HP5MS 5% Phenyl Methyl Silox; 30m X 250$\mu$m X 025um. A direct injection method was used meaning that no incubation was used for the samples. The GC oven method used was 50°C to 280°C at 10°C per minute with a hold at 280°C for 2 minutes for a total method time of 25 minutes. Four specific compounds were monitored over time: benzyl acetate, coranol, verdyl proprionate and florol and the results are summarized in Figure 33.

The fragrance release data follows the mass loss data very closely; it shows for prior art hydrogel air fresheners moisture loss and fragrance loss are inherently linked, which has been demonstrated before. When the moisture content of the gel is reduced, mobility in the gel diminishes and fragrance loss is reduced, leading to a reduced performance of the air freshener with time. The test devices show a surprising diversion from this typical performance trend. The test devices demonstrated a high level of fragrance compounds in the headspace even when the sample is completely dried. For the 4 compounds tested, a significantly higher headspace concentration was seen from between 10-20 days, and the levels appear to be dropping only gradually at this late stage. The lowest threshold of quantification is shown for each compound in Figure 33.

*Sensory Evaluation:* The fragrance intensity of devices according to several aspects presented herein was evaluated by panelists over a period of three weeks. Evaluation sessions began when samples were freshly prepared. The six samples were evaluated at t = 0, 2, 5, 7, 9, 15 and 19 (t=days). The samples included four test devices (Gyroid15, Gyroid16, Gyroid20 and Gyroid21) as well as two controls (ControlHi, ControlLo).

Each sample was placed on the floor of a separate 1.78 m$^3$ glass cabin and was hidden behind a shield to prevent bias due to visual sample differences. Each booth was labeled with a different 3-digit blind code for each session. Panelists were not trained, but had experience evaluating fragrance intensity. Between 29 - 35 panelists participated in each session. Panelists were instructed to evaluate the headspace of each sample by opening the hatch of the cabin, taking one sniff, closing the cabin and rating the overall fragrance intensity. Samples were presented in a random order for each panelist. Ratings were made on continuous line scales from 0-10, where "0" indicated "None" and "10" indicated "Very strong" for overall fragrance intensity.

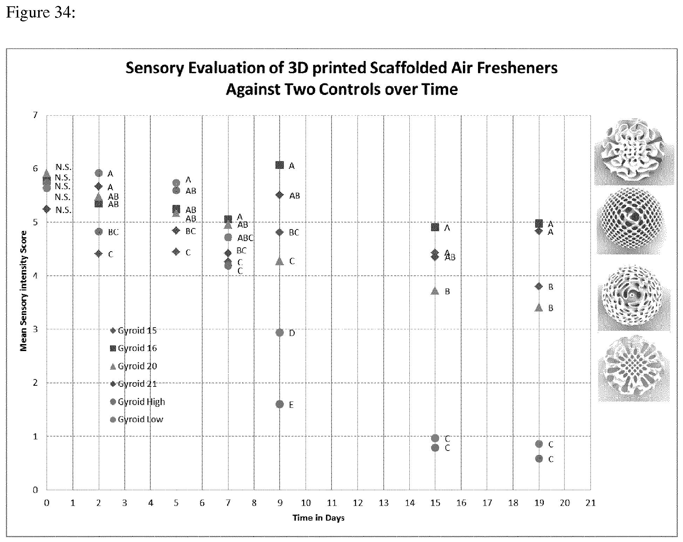

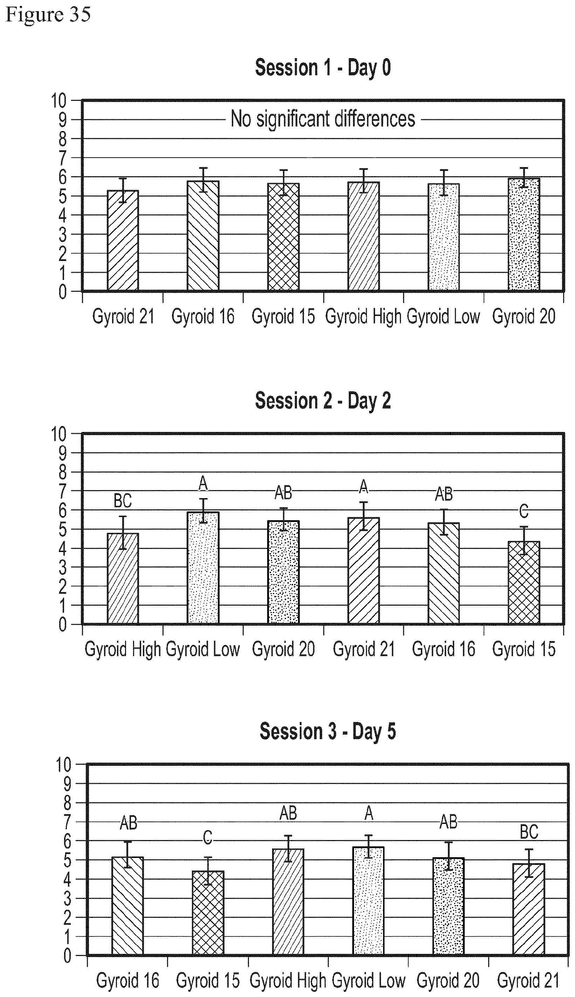

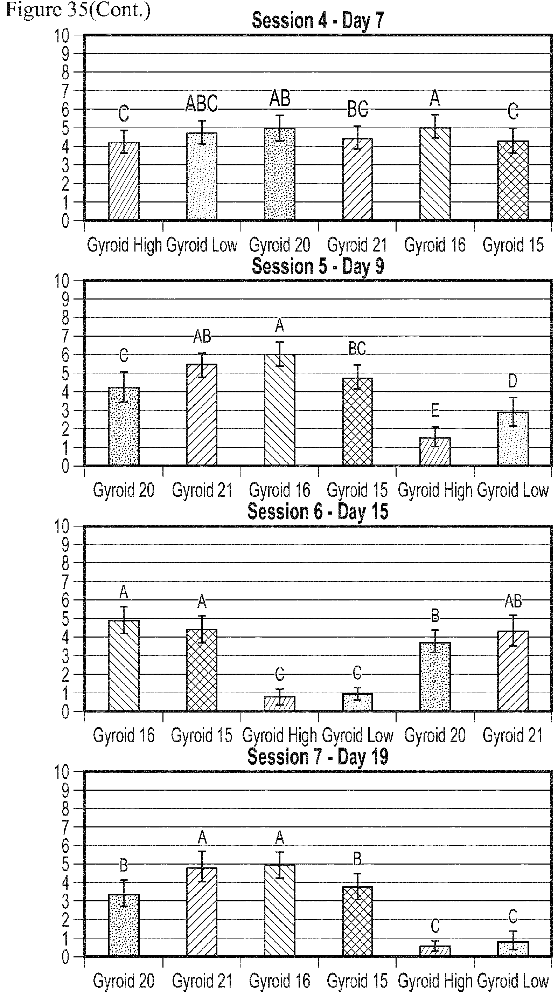

Panelists were asked to take a short break between each sample evaluation. All answers were recorded on a Fizz Forms paper ballot. The forms were scanned and data analysis was conducted using Fizz Calculations 2.50 (Biosystemes). Overall intensity ratings for each sample were averaged and a one-way Analysis of Variance (ANOVA) using $\alpha$=0.05 and the Duncan test was done for each session to compare the effect of the product on fragrance intensity. The results are shown in Figures 34 and 35.

These data clearly show that initially all air fresheners perform the same. This is a good confirmation of the rigor of the experimental setup. Initially, the device is not contributing yet, since the gel surface and volume are approximately the same as the control. During the first week there is a slight diminishing of intensity for all prototypes, with only minor differences between samples. After one week, there is a clear difference between the 3D printed devices and the controls. The controls have severely lowered intensity scores, whereas the test devices continued to receive high intensity rankings. After almost 3 weeks, device 16 in particular, has only slightly dropped from the initial intensity level (~5), whereas the controls are scoring below 1 on average.

This data is complimentary to the data presented before on headspace analysis and confirms that the test air freshener devices provide a surprising fragrance performance effect. Even when these air fresheners have almost released all their water, they continue to perform with high fragrance concentrations in the air (headspace data) and with great fragrance performance (sensory data).

There also appears to be an approximate ranking in the device performance based on sensory data (see inset images in Figure 34). Based on this data it is quite clear that different versions of the same basic gyroid equation can create different devices with slightly different release properties. We note devices 15,16,20 and 21 all have at least one region with a large surface area (e.g. > 3-4 cm$^2$/cm$^3$) per unit volume.

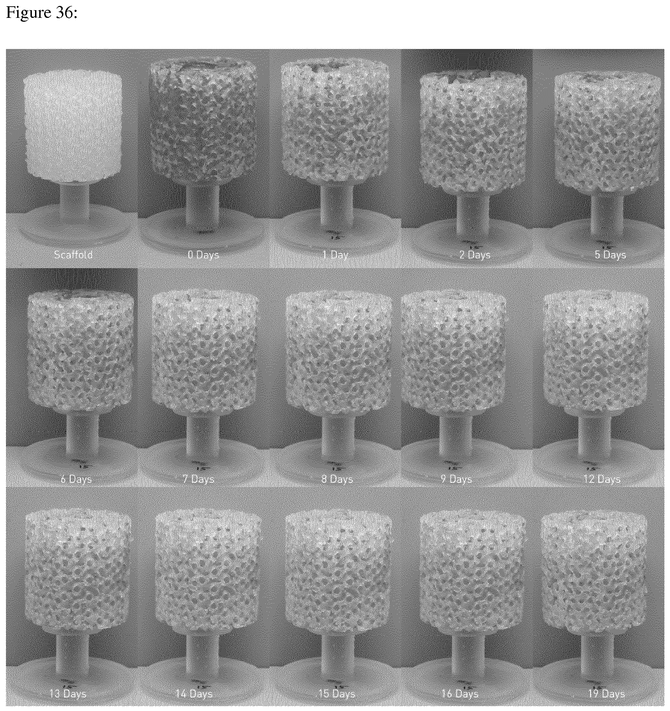

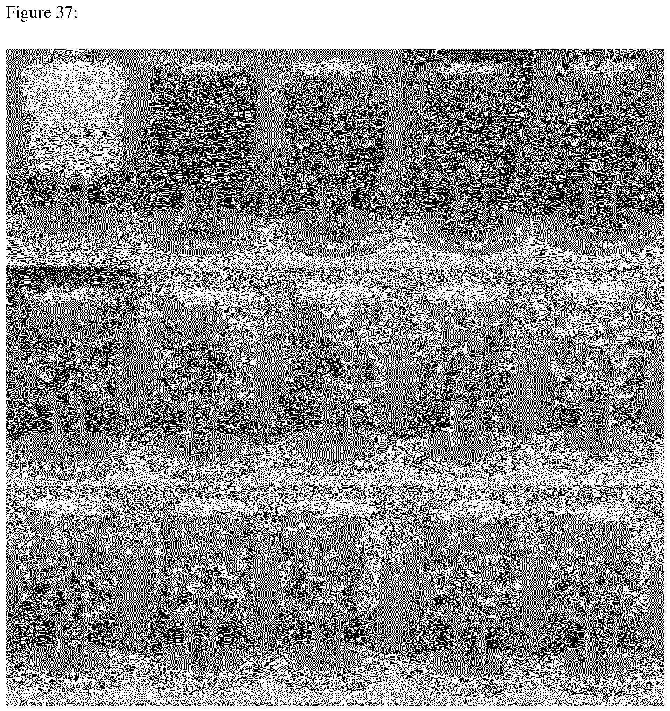

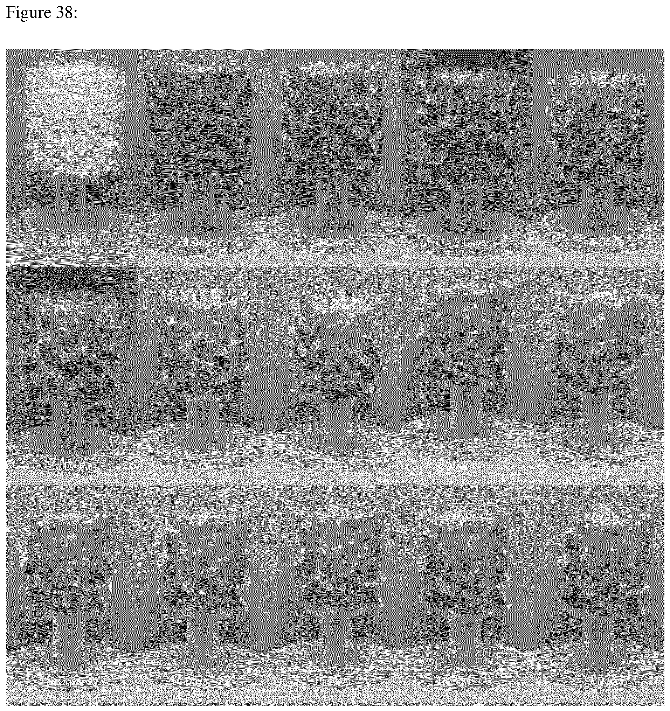

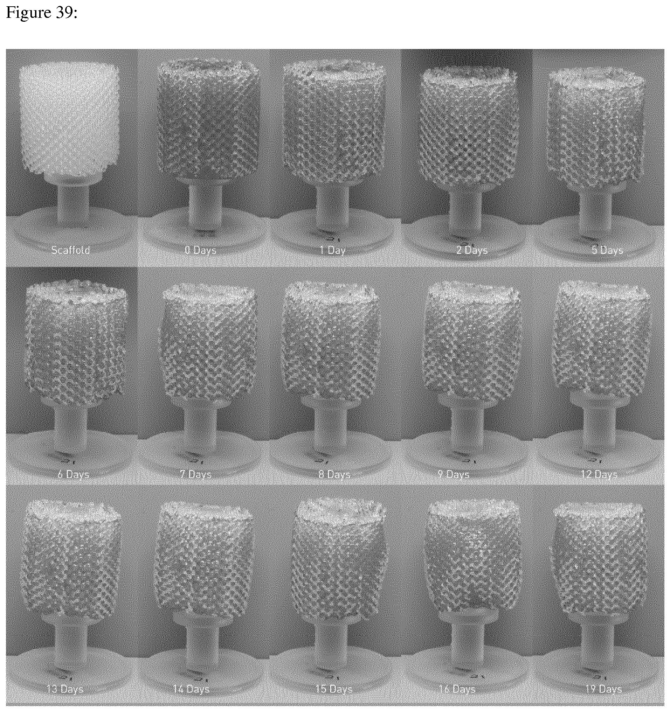

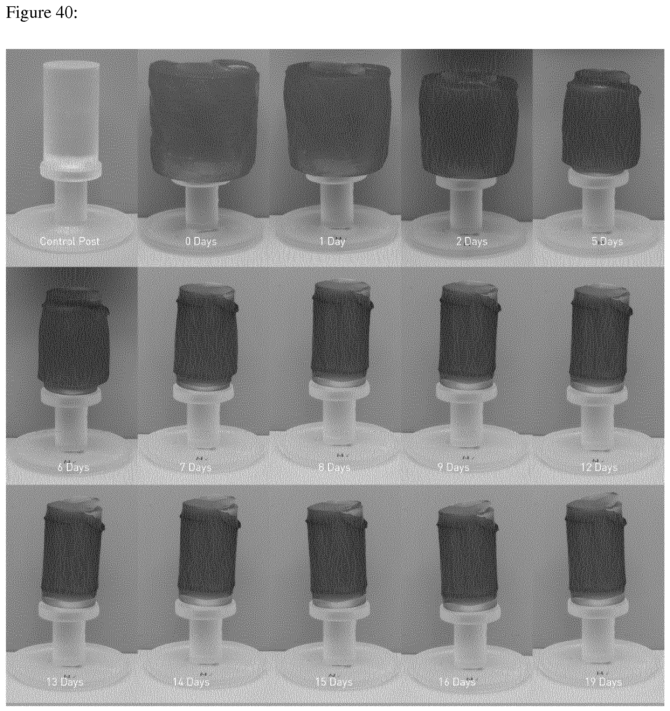

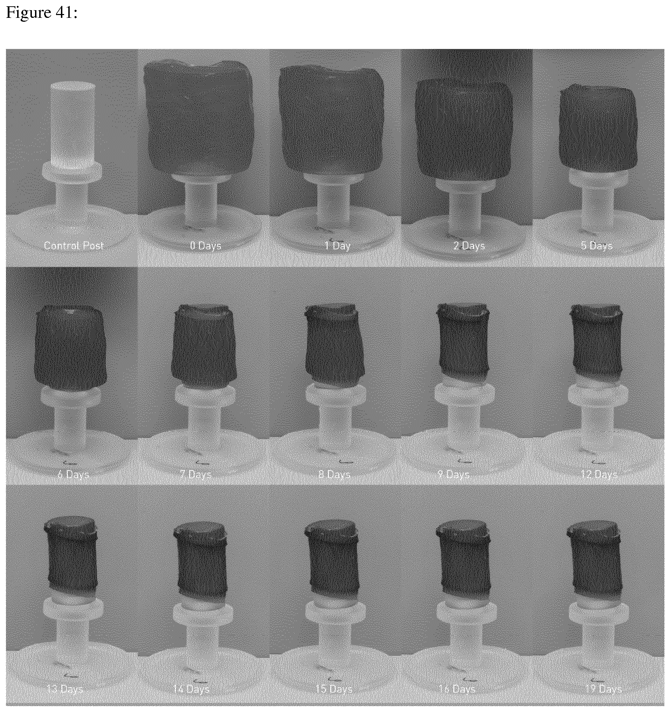

Photographs of all air fresheners at different points in time are shown in Figures 36-41. The devices show that the receding gel surface creates new surfaces and the structure geometry forces the surface to conform to its shape, not allowing the surface to freely move as it wants. Also, notice how the test devices air fresheners tend to get lighter in appearance, whereas the controls appear to get darker. The control air fresheners clearly show uniform shrinking with diminishing surface area over time. The test device air fresheners have an advantage in that they have a more consistent/appealing appearance later in life that matches their more consistent performance.

At the end of drying, all air fresheners were inspected closely and were sawed in half to inspect the core of the sample as well. All devices showed very dry films, which suggested that most of the moisture (and mobility) was lost. However, the test devices prototypes were surprisingly intense in fragrance when sniffed, whereas the controls were barely fragrant at all. It was observed that the device containing air fresheners had generated thin, semi-brittle, hard films that appear to release fragrance very effectively (~0.1 mm thick). Also, these films separated from the structure in many spots exposing both sides of the film to air. The control films were considerably thicker (about 0.5-0.9mm), and they were firmly stuck to the central post, further limiting the surface area. It is noteworthy that the control films became increasingly fragrant once they were removed from the post, indicating that there was still remaining fragrance in the control, however it was significantly locked in probably due to a combination of low mobility and relatively large permeation distance.

Without intending to be limited to any particular theory, devices according to certain aspects presented herein provide a structure having an increased surface area, compared to conventional gel-based air fresheners. The data presented in this example indicate that structures having smaller pores perform better than structures having larger pores. This enhanced performance may be, in part, due to an increased surface area, an increased or uniform airflow through the device, an increased number of routes for airflow through the device, alterations in airflow, creating a boundary layer that slows down the evaporation of the at least one active composition, adhesion points for the at least one active composition, resulting in a decreased rate in recession, or an increased surface area of the at least one active composition during evaporation, formation of a thin film of the at least one active composition (resulting in a reduced distance over which fragrance molecules have to diffuse to reach the surface and release), or any combination thereof.

Further, the enhanced performance observed with structures having smaller pores appeared to be similar in devices having a uniformly small pore throughout the structure, compared to structures having smaller pores at the center of the structure, and a porosity gradient that increases in size to the periphery of the structure, or structures having larger pores at the center of the structure, and a porosity gradient that decreases in size to the periphery of the structure.

**Example** 7: **Dual Fragrance Release Scaffolds**

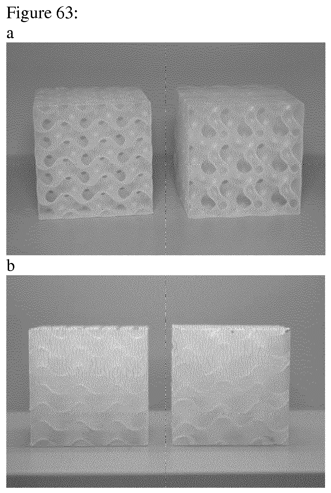

Two enantiomorphic scaffolds "Control" and "Flip" separating a cube (7.5 cm side) into two equal volumes A and B were designed. See Figure 63.Thurman15!

Thurman1515!

Both only open in the x-direction and have walls in the y and z direction. The designs are governed by the following implicit equations:

(cos(x)*sin(y)+cos(y)*sin(z)+cos(z)*sin(x))*(abs(y)- 9.8)*(abs(z)-9.8)=0 Control:

(cos(x)*sin(y)+cos(y)*sin(z)+cos(z)*sin(x)+(90/100)*(- 0.2*abs(x)+1))*(abs(y)-9.8)*(abs(z)-9.8)=0 Flip:

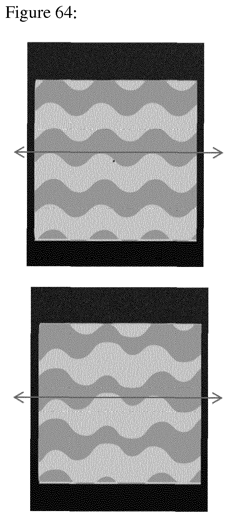

Scaffold "Control" is based on a Gyroid equation where volume A and B are equally distributed from surface to center. Scaffold "Flip" uses a gradient, where volume A is larger on the surface and smaller near the center, whereas volume B is the opposite. Representative cross sections of the scaffolds are shown below in Figure 64. The balance between volume A and B was 50/50 for the whole object. All surfaces on the perimeter were insulated except for the horizontal direction. These two volumes were filled with two different fragranced hydrogels. Citral (Citrus) and Isopropyl Quinoline (Earthy moss/Woody) were chosen as the compounds to evaluate for the air freshener sensory tests.

Preparation of Hydrogels for Filling 3D Printed Scaffolded Air Fresheners Control and Flip:

Colored water was used to prepare the fragranced hydrogels, so the hydrogel could be visualized in the 3D printed scaffold and easily photographed. A blue color was used for the Citral hydrogel and a red color was used for the Isopropyl Quinoline hydrogel. 5.27 milligrams of Blue 1 Dual Cert Dye (Lot# 1000477153) was added to 500 milliliters of water, and 80.5 milligrams of Color Red Lake 40 (Lot# 1000200607) was added to 500 milliliters of water and shaken well.

Citral Hydrogel - 480.97 grams of water was added to a beaker and heated to 75-80°C while stirring with an overhead stirrer. After reaching 75-80°C, 14.28 grams of carrageenan powder was added to the water and stirred until dissolved (approximately 5 minutes). After this time, 5.015 grams of Citral and 5.084 milligrams of Neolone were added and the mixture was stirred for approximately 2-3 minutes. After this time, the material was prepared for addition to the 3D printed scaffolds.

Isopropyl Quinoline Hydrogel - 480.98 grams of water was added to a beaker and heated to 75-80°C while stirring with an overhead stirrer. After reaching 75-80°C, 14.26 grams of carrageenan powder was added to the water and stirred until dissolved (approximately 5 minutes). After this time, 5.018 grams of Isopropyl Quinoline and 5.005 milligrams of Neolone were added and the mixture was stirred for approximately 2-3 minutes. After this time, the material was prepared for addition to the 3D printed scaffolds.

Procedure for filling and maintaining the 3D Printed Scaffolds for Sensory Tests:

The empty 3D printed scaffolds were weighed. The scaffolds were then covered in parafilm, wrapped with duct tape, and shrink wrapped on 5 sides to prevent leakage of the hot hydrogel when it was added. A 60 milliliter syringe was filled with the freshly prepared hot fragranced hydrogel and the two different volumes of the scaffolds were filled simultaneously. Scaffold 1 (control) was filled with 187.92 grams of Citral blue hydrogel and 193.42 grams of Isopropyl Quinoline red hydrogel. Scaffold 2 (flip) was filled with 191.24 grams of Citral blue hydrogel and 200.59 grams of Isopropyl Quinoline red hydrogel.

The small weight differences between volumes A and B and between Control and Flip scaffold were deemed insignificant for the purpose of this study. After filling, the scaffolds were left undisturbed in the ventilation hood to cool for approximately 1 hour so the hydrogel would solidify. After this time, the scaffolds were wrapped in aluminum foil and placed in a plastic bag to reduce the evaporation of water and left to cure overnight in the ventilation hood. In the morning, the 3D printed scaffolds were totally unwrapped. Any excess gel was removed from the outsides of the scaffolds. The weight was noted. The four closed sides (they had small minor holes) were covered with parafilm again and the scaffold weights were noted. On predetermined days when sensory test were scheduled, the scaffolds were removed from the hoods, weighed, and photographed.

Weight loss results and visual results:

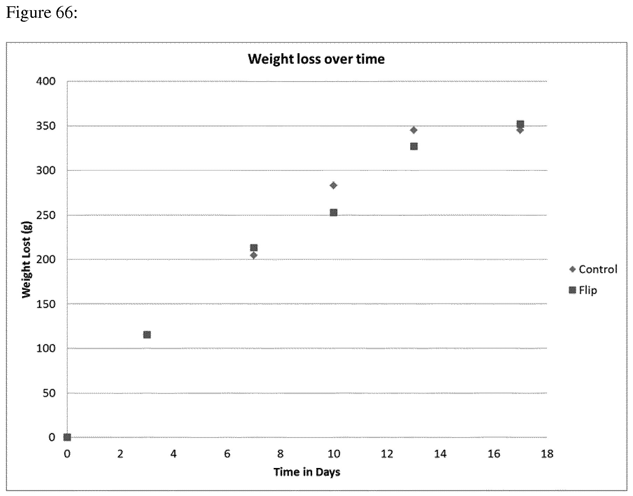

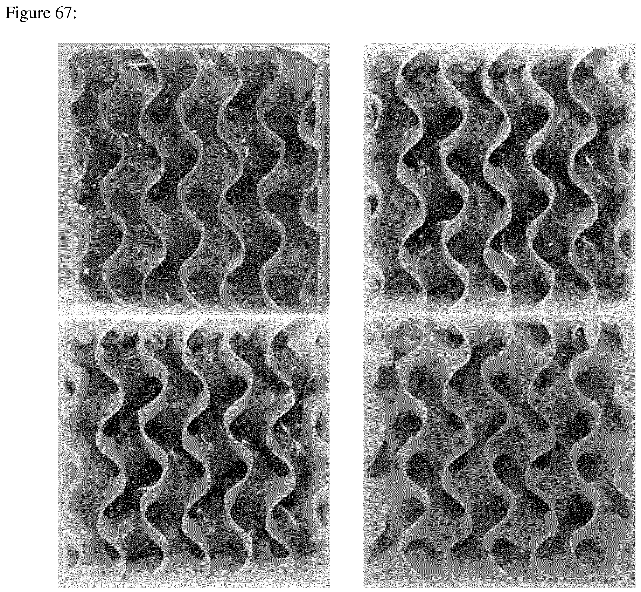

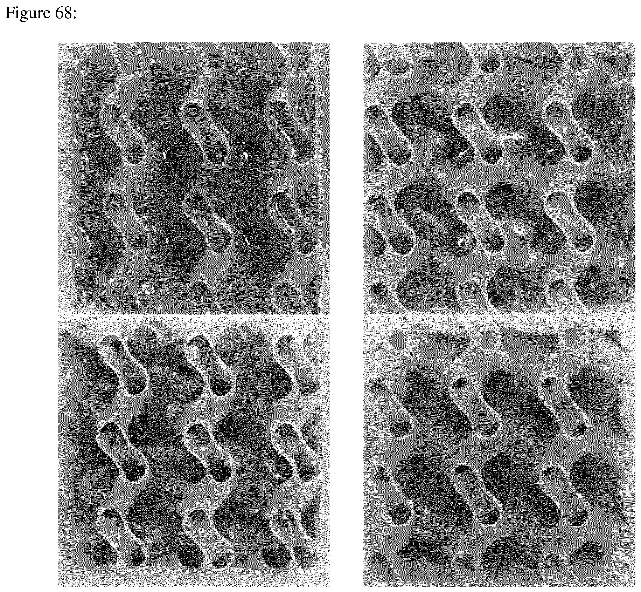

Figures 66, 67 and 68 show that the mass loss from both air fresheners was nearly identical. Therefore, we did not significantly alter the mass transfer from these devices and both performed equally.

Sensory Evaluation Procedures:

The fragrance intensity of two 3D printed air fresheners was evaluated by panelists over a period of three weeks. Evaluation sessions began when samples were freshly prepared. The two samples were evaluated at t = 0, 3, 7, and 17 (t=days). The samples included the control scaffold and the test scaffold. Each sample was placed at the bottom of a separate glass aquarium tank (Volume = 5.5 gallons) approximately 30-45 minutes prior to testing to allow the headspace to equilibrate. The glass lid of the aquarium tank had a circular sniff opening covered by a sliding lid to be opened during each evaluation. The aquarium tank was covered in opaque contact paper to prevent bias due to visual sample differences. In addition, an opaque weigh dish was placed on top of each air freshener to further obscure the sample. Each tank was labeled with a different 3-digit blind code for each session. Panelists were not trained, but had experience evaluating fragrance intensity. Between 23 - 24 panelists participated in each session. Before testing, panelists were provided with three reference jars containing the same gels used to fill the control and test scaffolds. The references were labeled "Citrus" (contained citral), "Earthy/moss - Woody" (contained Isopropyl quinoline) and "Mixture" (contained 50:50 Citral : Isopropyl quinoline). Panelists were instructed to evaluate each jar and familiarize themselves with the quality of references. Afterwards, they were asked to evaluate the headspace of each sample by sliding open the lid of the tank, taking a sniff above the circular opening, closing the lid and rating the citrus intensity and earthy/moss - woody intensity on separate continuous line scales from 0-10, where "0" indicated "None" and "10" indicated "Very strong". Samples were presented in a random order for each panelist. Panelists were asked to take a short break between each sample evaluation. All answers were recorded using Fizz Acquisition 2.51 (Biosystemes).

Sensory results:

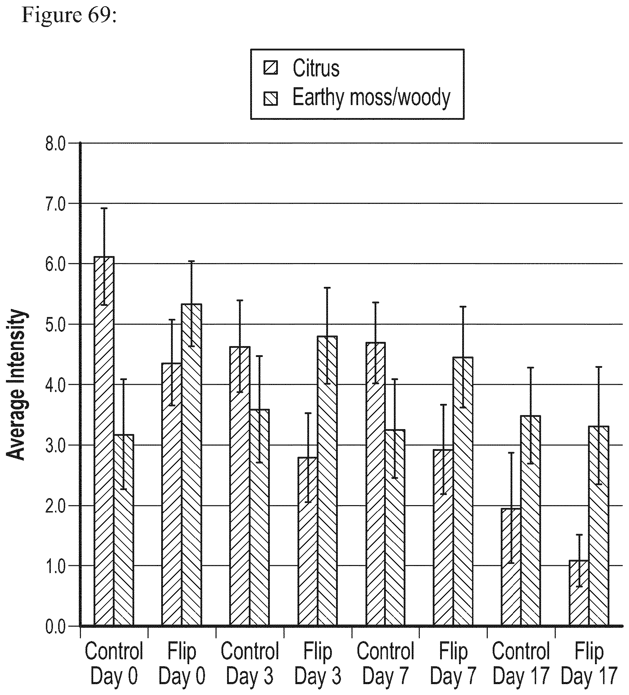

The results (See Figure 69) indicate that initially the control scaffold was dominated by Citrus even when the exposed surface was initially around 50/50. Later in the test (Day 17) the sensory intensity in the control was now dominated by Earthy moss/woody notes; the control had flipped. Surprisingly, the flip effect was observed in the control, whereas the flip effect was negated in the other scaffold due to its design.

This demonstrates that for two dual release air fresheners that are equal in composition (e.g. they contain the same amount of fragrance A and B) and dry at equal rates, the character of the fragrance over time can be adjusted by manipulating the shape of the scaffold.

## Example 8: Higher Order Scaffolds

Higher or n-order scaffolds can be created by multiplication of various triply periodic minimal surfaces with various offsets. For example, by multiplying the four following equations, 4 surfaces that are non-intersecting can be obtained, separating 5 volumes from one another:

$$G1 = \sin x * \cos y + \sin y * \cos z + \sin z * \cos x + 1 = 0$$

$$G2 = \sin x * \cos y + \sin y * \cos z + \sin z * \cos x + 0.2 = 0$$

$$G3 = \sin x * \cos y + \sin y * \cos z + \sin z * \cos x - 0.5 = 0$$

$$G4 = \sin x * \cos y + \sin y * \cos z + \sin z * \cos x - 1.1 = 0$$

$$G5 = G1 * G2 * G3 * G4 = 0$$

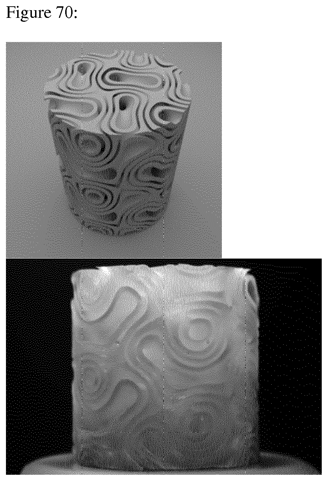

See Figure 70.

## Example 9: Fragranced PEBAX with SLA Scaffold

A scaffolded air freshener with a PEBAX based gel (see U.S. Patent No. 7.,708,982) instead of a hydrogel was created.

PEBAX SA01 (20.17g) was combined with a commercial fragrance (80.17g) in a 400ml beaker equipped with an overhead stirrer. A small amount of Cobalt Blue Liquid Dye D1960 (approximately 5mg) was added to achieve the desired blue color. The material was then heated to approximately 140°C to melt the PEBAX. An SLA 3D printed scaffold was placed into a paper coffee cup and the melted fragranced PEBAX solution was poured over the scaffold completely covering it. It was allowed to cool overnight undisturbed in the ventilation hood. The next day, the paper cup was removed from the scaffold and the excess fragranced PEBAX was trimmed away with a scalpel to reveal the shape of the 3D printed scaffold. The scaffold with the PEBAX containing fragrance functioned as an air freshener, and was monitored over time as it was located in a fume hood.

PEBAX SA01 (20.17g) was combined with a commercial fragrance [Note this not a formulation we can disclose, is this an issue?] (80.17g) in a 400ml beaker equipped with an overhead stirrer. A small amount of Cobalt Blue Liquid Dye D1960 (approximately 5mg) was added to achieve the desired blue color. The material was then heated to approximately 140°C to melt the PEBAX. An SLA 3D printed scaffold was placed into a paper coffee cup and the melted fragranced PEBAX solution was poured over the scaffold completely covering it. It was allowed to cool overnight undisturbed in the ventilation hood. The next day, the paper cup was removed from the scaffold and the excess fragranced PEBAX was trimmed away with a scalpel to reveal the shape of the 3D printed scaffold. The scaffold with the PEBAX containing fragrance functioned as an air freshener, and was monitored over time as it was located in a fume hood. It showed some of the desirable traits of our invention, although it was less pronounced since the PEBAX shows less signs of shrinking. It merely demonstrates that PEBAX containing fragrance is compatible with the current invention. It may be wise to ensure that it is listed as a gelator in the filing and other compounds analogous to it.

**Example 10: Air Fresheners - Simple Scaffolds**

The purpose of this Example was to in extend water (and ultimately fragrance) release time and maximize gel surface area simultaneously (membrane formation). This would prevent case-hardening, trapping moisture and fragrance ultimately, and maximizing payload volume/minimizing scaffold volume. The challenge was to find a way to produce more surface area within a shrinking overall volume, while retarding overall release rate to prolong functional lifespan of an air freshener.

To obtain directional design guidance, designs were loaded with the standard air freshener gel (no fragrance) and suspended in a hood (~200 ft3/min). Images and weights were taken of gel-filled designs at zero time and subsequently twice a day (once in morning, once in afternoon). The test endpoint was identified as the point when scaffold weight dropped less than 0.1g for two consecutive sample periods.

Gel was prepared and then poured into/over a scaffold in a beaker and left to cool overnight and sealed to prevent moisture loss (Parafilm). The following morning the gel/scaffold was removed from the beaker. Excess gel was trimmed to the surface plane of all sides with a straight bladed knife. A zero time weight and image was taken, and the scaffold was hung in the hood airflow (sash closed); if a side of a given design was not entirely flat, it was trimmed to the surface that was upper-most to that plane.

Generally speaking, all cube-based designs performed in roughly the same fashion, with the same progress-of-drying or gel retraction pattern exhibited.

The primary pattern was that the primary (external-most) gel surface would retract back through the first layer of cubic "scaffold cells", and stop at the face of the second layer of "scaffold cells". This is where the primary exterior-most surface of the gel would locate and remain.

As the gel moisture loss advanced into the depth of the structure, multiple secondary "gel fronts" developed into discrete internal "gel cells" defined by membranes in the internal "scaffold cells".

Initially, the "scaffold cells" are completely gel-filled, but that state subsequently transitions to two discrete volumes. The gel volume in the "scaffold cell" retracts slightly from the interior edges of the "scaffold cell" framework as moisture is lost and then appears to stabilize in that location with the "gel cell" initially defining the remaining gel volume in a given "scaffold cell". These were generally arranged so that one "gel cell" was apportioned to one "scaffold cell", but not always or exclusively; in some cases a single "gel cell" could encompass multiple "scaffold cells". This "gel cell" therefore defines the location of moisture (and fragrance). At this point in the drying process, at least one discrete gap or "port" developed in "gel cell" membranes in at least one of the corners of the "gel cell". These "communication ports" occurred at variable depths from the external-most primary gel surface.

These "communication ports" allowed internally located "gel cells" to communicate with the next shallower layer of "gel cells". Thus, in a serialized transport fashion, moisture could travel from port to port and layer to layer from the interior to the exterior of the structure where ultimately the moisture was slowly released to the ambient atmosphere by the system.

Ultimately, membranes were produced in the form of "gel cell" wall surfaces, yielding an increase in surface area. However, because of a "port" restricted moisture transport from "cell" to "cell" through multiple layers radially, it is conceivable that moisture loss (and potentially fragrance release) would be retarded and but not precluded as would occur with a case-hardened condition. This feature appeared to be independent of "scaffold cell" size, geometry, or design volumes tested.

All of the cube-based scaffold designs performed in a similar fashion, although minor performance differences were noted. Some cube designs had "inhibitor tabs" designed onto the cross bars. One variant had four tabs arranged radially about the crossbars at 90° increments, and a second variant the same, but with all 4 tabs rotated 45° so as to be radially offset from the crossbars. The idea in both variants was to "inhibit" a gel front from retraction in an effort to further promote membrane formation. This approach was marginally more performant than the simpler cube-only-based designs.

Because the gel was in a dynamic condition as moisture was lost, it was assumed that a number of factors would come into play. Viscosity should increase at the surface of the gel because that was where drying occurred first, slowing the rate of moisture loss of the remaining gel in a given "gel cell". As gel viscosity increased, the surface tension of the gel should change as well, in turn affecting how the gel interacted with the structural crossbars of a given design. There is more surface area presented by the structure at the corners of a "scaffold cell" and most "gel-cell" corners remained attached to the crossbars of the "scaffold cell" for at least some time. Regions between the corners did not always stay attached, and while conjecture would assume that this would primarily be caused by more surface area contact at the corners than mid-crossbar, it could also be influenced by different local moisture content as well (where gel meets crossbar, corner-attached vs. center-detached).

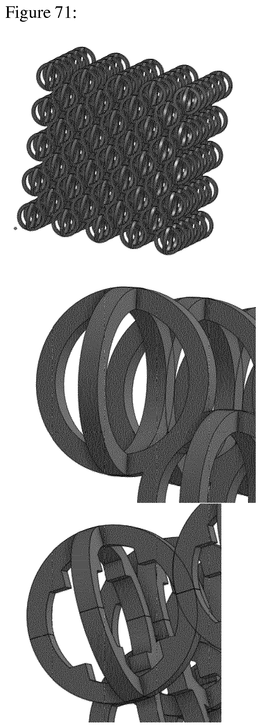

A different geometry was developed based on interlaced hoops. This approach was derived from the observation that curves always developed in the "gel cell" membrane as "gel cell" moisture content dropped during drying. Since the curves seemed to be a natural preference as the gel dried, it seemed worth trying. It was these curves that apparently drew the gel membranes away from the "scaffold cell" crossbars by artificially lengthening the corner to corner distance during drying. This distance change would put tension on the corner attachments via the gel surface facing the crossbar, potentially promoting the generation of "communicating ports" at the corners by pulling gel away from the corners. Two designs were tried, both with and without teeth. The "teeth" were a spin-off of the earlier idea of "inhibitors" to better hold the gel membrane from retracting as much; this was implemented as "Toothed-Hoops". See Figure 71.

At 72 hours, how the outermost (primary) membrane formation showed mostly at the extreme outer surface of the scaffold, not at the outermost edge of the interior second "scaffold cell" layer as previously found.

In the final analysis, moisture trapped in the interior "gel cells" was slowly released to the surrounding environment, with a resultant retardation in moisture loss rate. Case hardening was not observed to occur in a similar fashion to a traditional air freshener gel cone, which leaves moisture (and fragrance) trapped and inaccessible in the interior of the gel cone. The production of large surface areas via internal "gel cells" composed of membranes that allowed communication of moisture (and fragrance) to the gel and scaffold surface appear to be successful. These concepts are also a "worst case" scenario, as cubes intrinsically have a higher surface to volume ratio as compared to a cylinder or conical shape, and therefore would dry faster than a cylinder or cone under similar conditions. Hence, it is believed that fragrance release should be not only more complete, but fragrance release time ultimately extended, if the basic retraction patterns discerned here remains consistent upon the addition of fragrance. The other assumption is that a similar retraction pattern would be observed when utilizing a similar, but different gel formulation (other than the changes introduced with fragrance content alone).

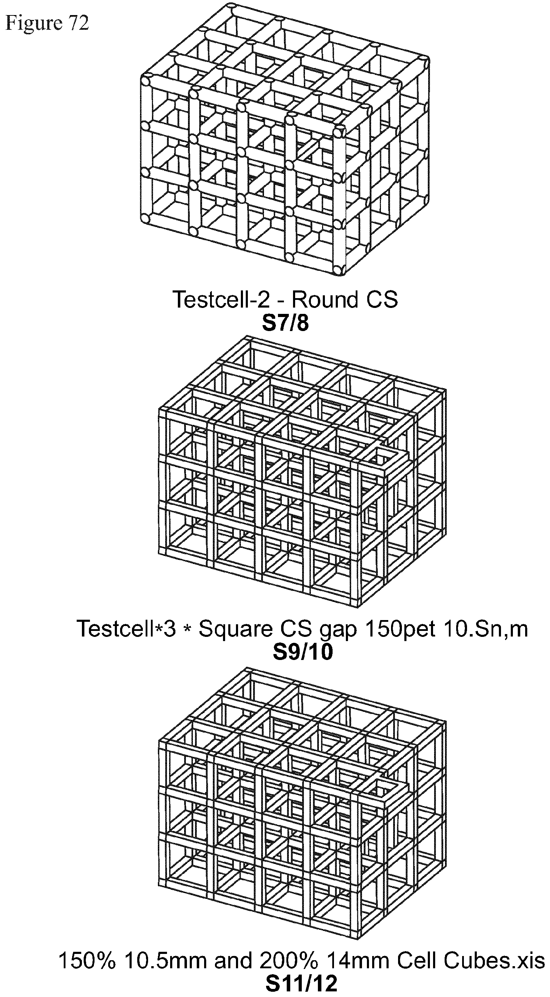

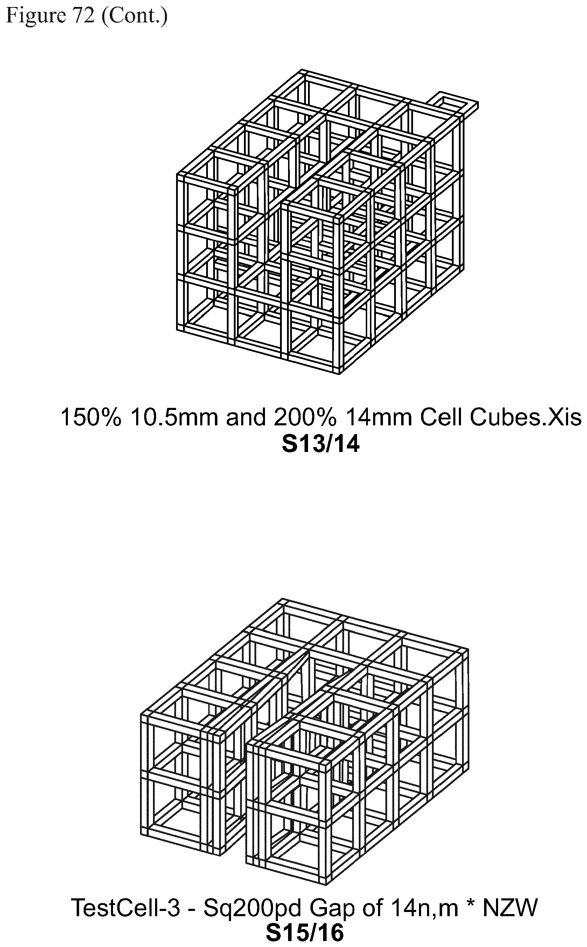

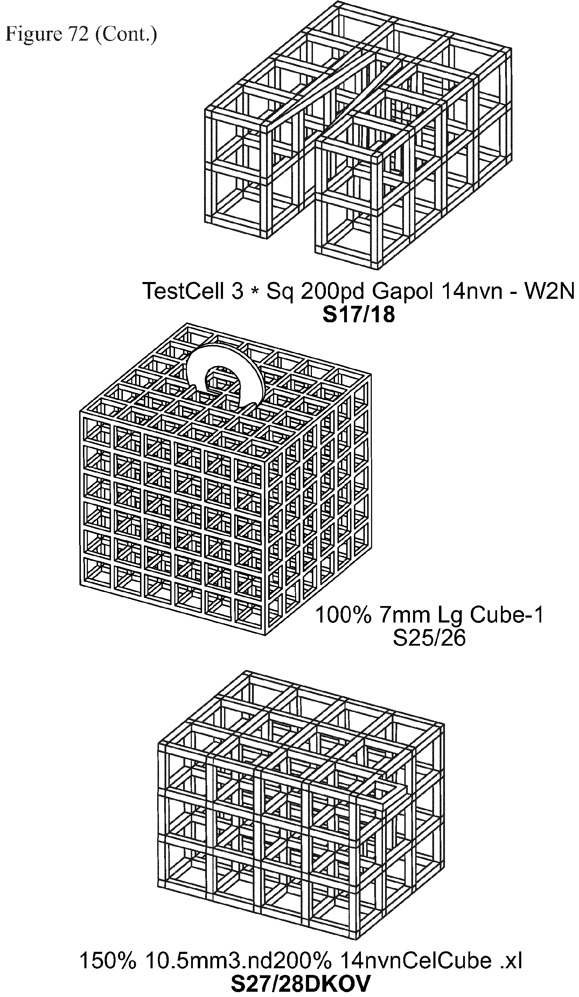

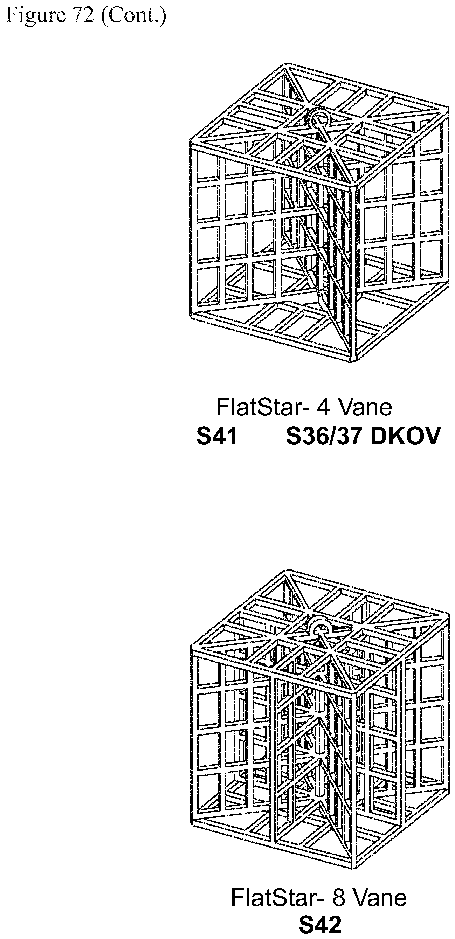

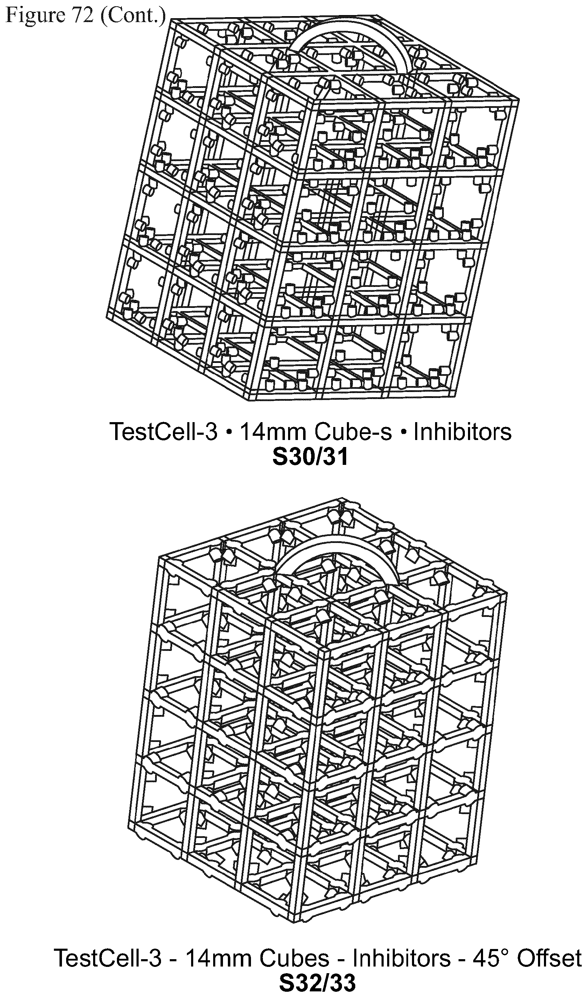

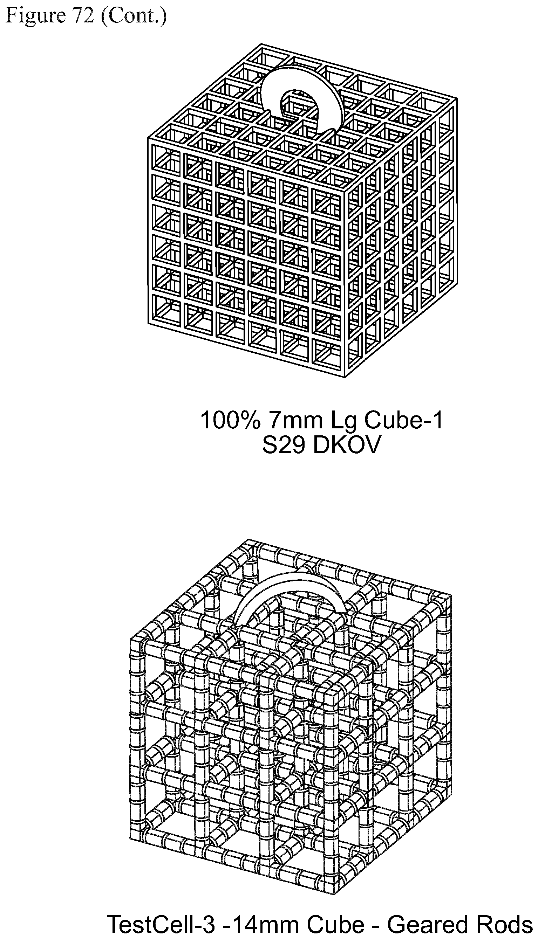

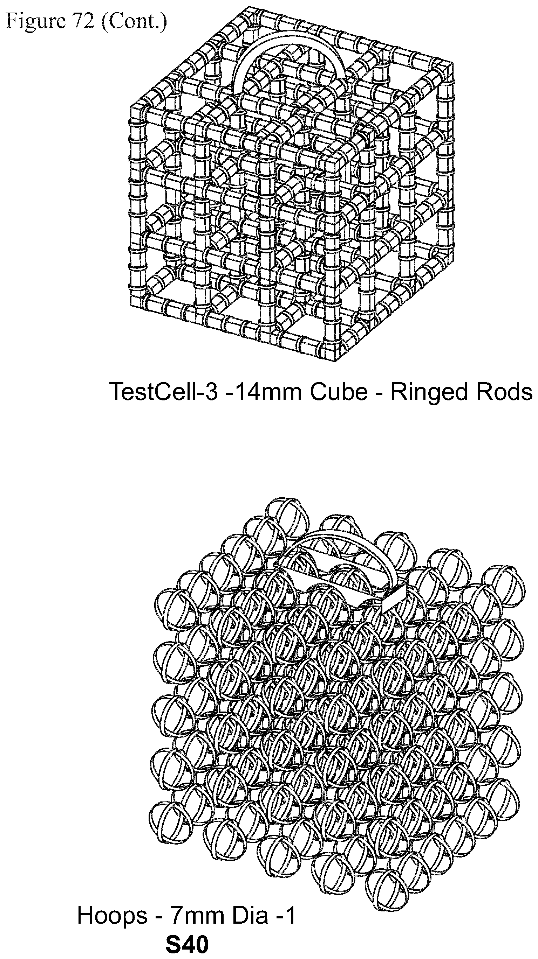

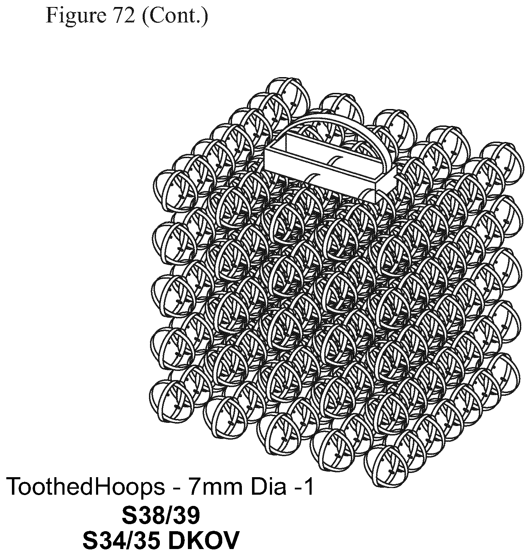

Figure 72 shows the simple scaffold designs that were studied.

## Claims

1. A fragrance delivery device comprising:

   a) a body portion,

      wherein the body portion has a volume and at least one body portion surface,
      wherein the volume comprises at least one network of a plurality of fluidly connected passages,
      wherein the at least one network of fluidly connected passages has at least one first end and at least one second end,
      wherein the at least one first end and at least one second end are separated by a distance,
      wherein at least one of the first or second ends are fluidly connected to the at least one body portion surface,
      wherein each individual passage within the plurality has a cross section,
      wherein the distance and the cross section of each passage within the plurality define a surface,

   b) at least one active composition,

      wherein the at least one active composition is disposed within the at least one network of a plurality of fluidly connected passages, and
      wherein the surface is configured to disperse the at least one active composition,

   wherein the surface is defined by a triply periodic minimal surface geometry,
   wherein the at least one active composition is a gel or a viscous liquid which is at least partially volatile, and
   wherein the at least one active composition is able to impart a fragrance to the surrounding space.

2. The device of claim 1, wherein each individual passage within the plurality has one or more branches.

3. The device of claim 1 or 2, wherein the body portion comprises a porous material.

4. The device of any one of the preceding claims, wherein the triply periodic minimal surface geometry is selected from the group consisting of: a gyroid geometry, a lidinoid geometry, a Schwarz D "diamond" geometry, or a Schwarz P "primitive" structure geometry.

5. The device of any one of the preceding claims, wherein the surface is defined according to the following Equation:

$$F(x, y, z) = sin(x) \cdot cos(y) + sin(y) \cdot cos(z) + sin(z) \cdot cos(x) = T.$$

**6.** The device of claim 5, wherein the value of T is selected from a numerical value between 0 and 1.43.

**7.** The device of claim 5, wherein the value of T is selected from a numerical value between 0 and -1.43.

**8.** The device of any one of the preceding claims, wherein the cross section of each individual passage within the plurality varies.

**9.** The device of claim 8, wherein the cross section of each individual passage within the plurality is greater in the center of the body portion than the cross section of each individual passage within the plurality at the periphery of the body portion.

**10.** The device of claim 8, wherein the cross section of each individual passage within the plurality is greater at the periphery of the body portion than the cross section of each individual passage within the plurality at the center of the body portion.

**11.** The device of any one of the preceding claims, wherein the body portion has a cross-sectional shape selected from the group consisting of: a square, a rectangle, a circle, an ellipse, a rhombus, a semi-circle, and a trapezium.

**12.** The device of any one of the preceding claims, wherein the body portion comprises two networks of a plurality of fluidly connected passages.

**13.** The device of claim 12, wherein, a first active composition is disposed within the first network, and a second active composition is disposed within the second network.

**14.** The device of any one of the preceding claims, wherein the device further comprises an airflow shield.

**15.** The device of any one of the preceding claims, wherein the at least one active composition is selected from the group consisting of an active composition comprising a wax, an active composition comprising a hydrogel, an active composition comprising an oleogel, an active composition comprising an organogel, or mixtures thereof.


**Patentansprüche**

**1.** Duftstoffspender, Folgendes umfassend:

a) einen Körperabschnitt,

wobei der Körperabschnitt einen Raum und mindestens eine Körperabschnittsfläche aufweist,
wobei der Raum mindestens ein Netzwerk aus mehreren fluidisch verbundenen Durchgängen umfasst,
wobei das mindestens eine Netzwerk aus fluidisch verbundenen Durchgängen mindestens ein erstes Ende und mindestens ein zweites Ende aufweist,
wobei das mindestens eine erste Ende und mindestens eine zweite Ende voneinander beabstandet sind,
wobei mindestens eines des ersten oder zweiten Endes mit der mindestens einen Körperabschnittfläche fluidisch verbunden ist,
wobei jeder einzelne Durchgang der mehreren Durchgänge einen Querschnitt aufweist, wobei die Entfernung und der Querschnitt jedes Durchgangs der mehreren Durchgänge eine Fläche definieren,

b) mindestens eine aktive Zusammensetzung,

wobei die mindestens eine aktive Zusammensetzung in dem mindestens einen Netzwerk mehrerer fluidisch verbundener Durchgänge angeordnet ist und
wobei die Fläche dazu ausgelegt ist, die mindestens eine aktive Zusammensetzung zu dispergieren,
wobei die Fläche durch eine dreifach-periodische minimale Flächengeometrie definiert ist,
wobei die mindestens eine aktive Zusammensetzung ein Gel oder eine viskose Flüssigkeit ist, das/die zumindest teilweise flüchtig ist, und wobei die mindestens eine aktive Zusammensetzung dazu funktionsfähig ist, einen Duftstoff an den umgebenden Raum abzugeben.

**2.** Vorrichtung nach Anspruch 1, wobei jeder einzelne Durchgang der mehreren Durchgänge eine oder mehrere Verzweigungen aufweist.

3. Vorrichtung nach Anspruch 1 oder 2, wobei der Körperabschnitt ein poröses Material umfasst.

4. Vorrichtung nach einem der vorstehenden Ansprüche, wobei die dreifach-periodische minimale Flächengeometrie aus der Gruppe ausgewählt ist, die aus Folgendem besteht: einer Gyroidgeometrie, einer Lidinoidgeometrie, einer Schwarz D Diamantgeometrie oder einer Schwarz P primitiven Strukturgeometrie.

5. Vorrichtung nach einem der vorstehenden Ansprüche, wobei die Fläche entsprechend der folgenden Gleichung definiert ist:

$$F(x,y,z) = sin(x) \cdot cos(y) + sin(y) \cdot cos(z) + sin(z) \cdot cos(x) = T.$$

6. Vorrichtung nach Anspruch 5, wobei der Wert von T aus einem numerischen Wert zwischen 0 und 1,43 ausgewählt ist.

7. Vorrichtung nach Anspruch 5, wobei der Wert von T aus einem numerischen Wert zwischen 0 und -1,43 ausgewählt ist.

8. Vorrichtung nach einem der vorstehenden Ansprüche, wobei der Querschnitt jedes einzelnen Durchgangs der mehreren Durchgänge variiert.

9. Vorrichtung nach Anspruch 8, wobei der Querschnitt jedes einzelnen Durchgangs der mehreren Durchgänge in der Mitte des Körperabschnitts größer ist als der Querschnitt jedes einzelnen Durchgangs der mehreren Durchgänge am Rand des Körperabschnitts.

10. Vorrichtung nach Anspruch 8, wobei der Querschnitt jedes einzelnen Durchgangs der mehreren Durchgänge am Rand des Körperabschnitts größer ist als der Querschnitt jedes einzelnen Durchgangs der mehreren Durchgänge in der Mitte des Körperabschnitts.

11. Vorrichtung nach einem der vorstehenden Ansprüche, wobei der Körperabschnitt eine Querschnittsform aufweist, die aus der Gruppe ausgewählt ist, die aus Folgendem besteht: einem Quadrat, einem Rechteck, einem Kreis, einer Ellipse, einem Rhombus, einem Halbkreis und einem Trapez.

12. Vorrichtung nach einem der vorstehenden Ansprüche, wobei der Körperabschnitt zwei Netzwerke aus mehreren fluidisch verbundenen Durchgängen umfasst.

13. Vorrichtung nach Anspruch 12, wobei eine erste aktive Zusammensetzung in dem ersten Netzwerk angeordnet ist und eine zweite aktive Zusammensetzung in dem zweiten Netzwerk angeordnet ist.

14. Vorrichtung nach einem der vorstehenden Ansprüche, wobei die Vorrichtung ferner eine Luftstromabschirmung umfasst.

15. Vorrichtung nach einem der vorstehenden Ansprüche umfasst, wobei die mindestens eine aktive Zusammensetzung aus der Gruppe ausgewählt ist, die aus einer aktiven Zusammensetzung mit Wachs, einer aktiven Zusammensetzung mit einem Hydrogel, einer aktiven Zusammensetzung mit einem Oleogel, einer aktiven Zusammensetzung mit einem Organogel oder Mischungen davon besteht.

**Revendications**

1. Dispositif de distribution de parfum comprenant :

   a) une partie corps,

      la partie corps présentant un volume et au moins une surface de partie corps,
      le volume comprenant au moins un réseau d'une pluralité de passages en communication fluidique,

l'au moins un réseau de passages en communication fluidique comportant au moins une première extrémité et au moins une seconde extrémité,

l'au moins une première extrémité et l'au moins une seconde extrémité étant séparées par une distance, au moins l'une des premières et seconde extrémités étant en communication fluidique avec l'au moins une surface de partie corps,

chaque passage individuel au sein de la pluralité présentant une section transversale, la distance et la section transversale de chaque passage au sein de la pluralité définissant une surface,

b) au moins une composition active,

l'au moins une composition active étant disposée à l'intérieur de l'au moins un réseau d'une pluralité de passages en communication fluidique, et

la surface étant conçue pour disperser l'au moins une composition active,

la surface étant définie par une géométrie de surface minimale triplement périodique,

l'au moins une composition active étant un gel ou un liquide visqueux qui est au moins partiellement volatil, et l'au moins une composition active étant apte à transmettre un parfum à l'espace ambiant.

2. Dispositif selon la revendication 1, dans lequel chaque passage individuel au sein de la pluralité comporte une ou plusieurs branches.

3. Dispositif selon la revendication 1 ou 2, dans lequel la partie corps comprend un matériau poreux.

4. Dispositif selon l'une quelconque des revendications précédentes, dans lequel la géométrie de surface minimale triplement périodique est choisie dans le groupe composé de : une géométrie gyroïde, une géométrie lidinoïde, une géométrie de surface minimale de Schwarz D et une géométrie de surface minimale de Schwarz P.

5. Dispositif selon l'une quelconque des revendications précédentes, dans lequel la surface est définie selon l'Équation suivante :

$$F(x,y,z) = sin(x) \cdot cos(y) + sin(y) \cdot cos(z) + sin(z) \cdot cos(x) = T.$$

6. Dispositif selon la revendication 5, dans lequel la valeur de T est choisie parmi des valeurs numériques comprises entre 0 et 1,43.

7. Dispositif selon la revendication 5, dans lequel la valeur de T est choisie parmi des valeurs numériques comprises entre 0 et -1,43.

8. Dispositif selon l'une quelconque des revendications précédentes, dans lequel la section transversale de chaque passage individuel au sein de la pluralité varie.

9. Dispositif selon la revendication 8, dans lequel la section transversale de chaque passage individuel au sein de la pluralité au centre de la partie corps est supérieure à la section transversale de chaque passage individuel au sein de la pluralité à la périphérie de la partie corps.

10. Dispositif selon la revendication 8, dans lequel la section transversale de chaque passage individuel au sein de la pluralité à la périphérie de la partie corps est supérieure à la section transversale de chaque passage individuel au sein de la pluralité au centre de la partie corps.

11. Dispositif selon l'une quelconque des revendications précédentes, dans lequel la partie corps présente une forme en section transversale choisie dans le groupe composé de : un carré, un rectangle, un cercle, une ellipse, un losange, un demi-cercle et un trapèze.

12. Dispositif selon l'une quelconque des revendications précédentes, dans lequel la partie corps comporte deux réseaux d'une pluralité de passages en communication fluidique.

13. Dispositif selon la revendication 12, dans lequel une première composition active est disposée à l'intérieur du premier réseau, et une seconde composition active est disposée à l'intérieur du second réseau.

14. Dispositif selon l'une quelconque des revendications précédentes, le dispositif comprenant, en outre, un écran de modification d'écoulement d'air.

15. Dispositif selon l'une quelconque des revendications précédentes, dans lequel l'au moins une composition active est choisie dans le groupe composé d'une composition active comprenant une cire, d'une composition active comprenant un *h*ydrogel, d'une composition active comprenant un oléogel, d'une composition active comprenant un organogel, ou de mélanges de celles-ci.

Figure 1:

Figure 2:

Figure 3:

Figure 4:

Figure 5:

Figure 6:

Figure 7:

Figure 8:

Figure 9:

Figure 10:

Figure 11:

Figure 12:

Figure 13:

Figure 14:

Figure 15:

Figure 16:

Figure 17:

Figure 18:

Figure 19:

Figure 20:

Figure 21:

Figure 22:

Figure 23:

Figure 24:

Figure 25:

Figure 26:

Figure 27:

Figure 28

Surface Area per Volume Distribution for 3D printed Scaffolds from Center to Surface

Figure 28 (Cont.):

| Description | 01: Gyroid Thickened | 02: Gyroid Thickened | 03:Gyroid Filled | 15: Gyroid Thickened | 16: Gyroid Thickened | 20: Gyroid Filled | 21: Gyroid Filled |
|---|---|---|---|---|---|---|---|
| Volumes | Volume A/B | Volume A/B | Volume A only | Volume A/B | Volume A/B | Volume A only | Volume A only |
| Used in Example | 2 | 2 | 2 | 6 | 6 | 6 | 6 |
| Gradient center to surface | None | Porosity change A/B | Porosity Change A | Frequency low to high | Frequency high to low | Frequency high to low | Frequency low to high |
| Frequency center fc | 0.3 | 0.45 | 0.4 | 0.1 | 1 | 1.8 | 0.2 |
| Frequency surface fs | 0.3 | 0.45 | 0.4 | 0.8 | 0.3 | 0.4 | 1.5 |
| Porosity Offset $P_{offset}$ | 0 | 0 | 1.2 | 0 | 0 | -1 | -1 |
| Porosity Gradient $P_{gradient}$ | 0 | 1.2 | 2.4 | 0 | 0 | 0 | 0 |

Figure 29:

Figure 30:

Figure 31:

Figure 32:

Figure 33

Figure 33 (Cont.)

Figure 34:

Sensory Evaluation of 3D printed Scaffolded Air Fresheners Against Two Controls over Time

Figure 35

**Session 1 - Day 0**

**Session 2 - Day 2**

**Session 3 - Day 5**

Figure 35(Cont.)

Session 4 - Day 7

Session 5 - Day 9

Session 6 - Day 15

Session 7 - Day 19

Figure 36:

Figure 37:

Figure 38:

Figure 39:

Figure 40:

Figure 41:

Figure 42:

Figure 43:

Figure 44:

Figure 45:

Figure 46:

Figure 47:

Figure 48:

Figure 49:

Figure 50:

Figure 51:

Figure 52:

Figure 53:

Figure 54:

Figure 55:

Figure 56:

Figure 57:

Figure 58:

Figure 59:

Figure 60:

Figure 61:

Figure 62:

basic gyroid    phase shift gradient    amplitude modification 1st term    frequency gradient    vertical shift gradient

Figure 63:

a

b

Figure 64:

Figure 65:

Figure 66:

Figure 67:

Figure 68:

Figure 69:

Figure 70:

Figure 71:

Figure 72

Testcell-2 - Round CS
**S7/8**

Testcell*3 * Square CS gap 150pet 10.Sn,m
**S9/10**

150% 10.5mm and 200% 14mm Cell Cubes.xis
**S11/12**

Figure 72 (Cont.)

150% 10.5mm and 200% 14mm Cell Cubes.Xis
**S13/14**

TestCell-3 - Sq200pd Gap of 14n,m * NZW
**S15/16**

Figure 72 (Cont.)

TestCell 3 * Sq 200pd Gapol 14nvn - W2N
**S17/18**

100% 7mm Lg Cube-1
S25/26

150% 10.5mm3.nd200% 14nvnCelCube .xl
**S27/28DKOV**

Figure 72 (Cont.)

FlatStar- 4 Vane

**S41      S36/37 DKOV**

FlatStar- 8 Vane
**S42**

Figure 72 (Cont.)

TestCell-3 • 14mm Cube-s • Inhibitors
**S30/31**

TestCell-3 - 14mm Cubes - Inhibitors - 45° Offset
**S32/33**

Figure 72 (Cont.)

100% 7mm Lg Cube-1
S29 DKOV

TestCell-3 -14mm Cube - Geared Rods

Figure 72 (Cont.)

TestCell-3 -14mm Cube - Ringed Rods

Hoops - 7mm Dia -1
**S40**

Figure 72 (Cont.)

ToothedHoops - 7mm Dia -1
**S38/39**
**S34/35 DKOV**

EP 3 773 758 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018015385 A1
- EP 3222297 A1
- WO 2013025585 A1
- WO 2016172699 A
- WO 2017017251 A1
- WO 2013030153 A1

- WO 02055116 A1
- EP 1177799 A1
- US 6039266 A
- US 20020041860 A1
- US 7708982 B

**Non-patent literature cited in the description**

- Double-Gyroid-Structured Functional Materials. **RUDOLF, M** ; **SCHERER, J.** Gyroid and Gyroid-Like Surfaces. 2013, 7-19
- **S. ANDERSSON K** ; **LARSSON M.** ; **LARSSON M. JACOB.** Biomathematics, Mathematics of Biostructures and Biodynamics. Elsevier Science, 1999
- **VENKATESH, V.** ; **REDDY, K. A. K** ; **SREEKANTH, E.** *Design of Mathematically Defined Heterogeneous Porous Scaffold Architecture for Tissue Engineering*, 2014, vol. 10 (24), 1169-1174

- Wohlers Report 2016 3D Printing and Additive Manufacturing State of the Industry.. **WOHLER, T.** Annual Worldwide Progress Report.. Wohlers Associates, Inc, 2016
- **S. ARCTANDER**. Perfume and Flavor Chemicals. Montclair, 1969
- **A. M. EL-SAYED**. *The Pherobase*, 2005, http://www.pherobase.net
- Colour Index International. The Society of Dyers and Colourist.
- **ABDERRAHMAN TAHA**. *TicknessGenerator-1.0*, https://www.facebook.com/permalink.php?story_fbid=772823949501730&id=52951025383 3102